(19) **Europäisches Patentamt**
**European Patent Office**
**Office européen des brevets**

(11) **EP 2 200 692 B1**

(12) **EUROPEAN PATENT SPECIFICATION**

(45) Date of publication and mention
of the grant of the patent:
**09.11.2016 Bulletin 2016/45**

(51) Int Cl.:
*A61N 1/00* *(2006.01)*     *A61B 5/0452* *(2006.01)*
*A61B 5/048* *(2006.01)*     *A61B 5/021* *(2006.01)*

(21) Application number: **08832864.6**

(22) Date of filing: **25.09.2008**

(86) International application number:
**PCT/US2008/011110**

(87) International publication number:
**WO 2009/042172 (02.04.2009 Gazette 2009/14)**

(54) **FREQUENCY SELECTIVE MONITORING OF PHYSIOLOGICAL SIGNALS**

FREQUENZSELEKTIVE ÜBERWACHUNG VON PHYSIOLOGISCHEN SIGNALEN

SURVEILLANCE SÉLECTIVE EN FRÉQUENCE DE SIGNAUX PHYSIOLOGIQUES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **26.09.2007 US 975372 P
01.02.2008 US 25503
24.07.2008 US 83381**

(43) Date of publication of application:
**30.06.2010 Bulletin 2010/26**

(73) Proprietor: **Medtronic, Inc.
Minneapolis, MN 55342-5604 (US)**

(72) Inventors:
 • **DENISON, Timothy, J.
Minneapolis
MN 55410 (US)**
 • **JENSEN, Randy, M.
Hampton
MN 55031 (US)**
 • **SANTA, Wesley, A.
Andover
MN 55304 (US)**

(74) Representative: **Hughes, Andrea Michelle
Dehns
St Bride's House
10 Salisbury Square
London EC4Y 8JD (GB)**

(56) References cited:
**WO-A-97/10747**     **WO-A-2006/126186**
**US-A- 3 780 725**     **US-A1- 2005 007 091**
**US-A1- 2006 133 550**

 • TIMOTHY DENISON ET AL: "A 2.2 W 94nV/Hz,
Chopper-Stabilized Instrumentation Amplifier for
EEG Detection in Chronic Implants"
SOLID-STATE CIRCUITS CONFERENCE, 2007.
ISSCC 2007. DIGEST OF TECHNICAL PAPERS.
IEEE INTERNATIONAL, IEEE, PI, 1 February 2007
(2007-02-01), pages 162-594, XP031180026 ISBN:
978-1-4244-0852-8
 • AL-THADDEUS AVESTRUZ ET AL: "A 5
W/Channel Spectral Analysis IC for Chronic
Bidirectional Brainâ Machine Interfaces" IEEE
JOURNAL OF SOLID-STATE CIRCUITS, IEEE
SERVICE CENTER, PISCATAWAY, NJ, US, vol.
43, no. 12, 1 December 2008 (2008-12-01), pages
3006-3024, XP011238677 ISSN: 0018-9200

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The invention relates to monitoring of physiological signals.

### BACKGROUND

Medical devices may be used to deliver therapy to patients to treat a variety of symptoms or conditions. Examples of therapy include electrical stimulation therapy and drug delivery therapy. Examples of symptoms or conditions include chronic pain, tremor, akinesia, Parkinson's disease, epilepsy, dystonia, neuralgia, obsessive compulsive disorder (OCD), depression, sleep dysfunction, urinary or fecal incontinence, sexual dysfunction, obesity, or gastroparesis. Information relating to symptoms or conditions may be sensed by monitoring physiological signals, such as electrocardiogram (ECG), electromyogram (EMG), electroencephalogram (EEG), electrocorticogram (ECoG), pressure, temperature, impedance, motion, and other types of signals.

Some signal monitors perform over-sampling of a wide band physiological signal and analyze selected portions of the signal via digital signal processing. This type of signal monitoring architecture is flexible in that it permits any selected frequency bands within the over-sampled wide band physiological signal to be digitally analyzed for information relating to particular symptoms or conditions. However, sampling of a wide band physiological signal may require large amounts of power consumption, computing, and memory. Therefore, medical devices with limited computing, memory and/or power capabilities, such as implantable medical devices, may not be well suited to this type of wide band signal monitoring architecture. WO 2006/126186 and WO 97/10747 teach systems for monitoring physiological signals using a heterodyne circuit.

### SUMMARY

In general, the invention is directed to a frequency selective monitor and methods for monitoring physiological signals in one or more selected frequency bands. A frequency selective monitor utilizes a heterodyning, chopper-stabilized amplifier architecture to convert a selected frequency band to a baseband for analysis. The frequency selective monitor may be useful in a variety of therapeutic and/or diagnostic applications. As examples, a frequency selective signal monitor may be provided within a medical device or within a sensor coupled to a medical device. The physiological signal may be analyzed in one or more selected frequency bands to trigger delivery of patient therapy and/or recording of diagnostic information. The subject-matter of the invention is defined by the independent claims 1 and 8.

In some cases, a frequency selective monitor as described herein may be configured to monitor a single frequency band of the wide band physiological signal. In addition, or alternatively, the techniques may be capable of efficiently hopping frequency bands in order to monitor the signal in two or more frequency bands. The frequency selective monitor may generate a triggering signal that triggers at least one of controlling therapy or recording diagnostic information based on analysis of the signal in one frequency band or multiple frequency bands. Therapy may be controlled by initiating delivery of therapy and/or adjusting therapy parameters. Recording diagnostic information may include recording the physiological signal, one or more characteristics of the signal, or other information.

In a further embodiment, the invention provides a medical device comprising a physiological signal monitoring device as in claim 1 and a therapy delivery module. The physiological signal monitoring unit comprises a physiological sensing element that receives a physiological signal, a heterodyning circuit configured to covert a selected frequency band of the physiological signal to a baseband, and a signal analysis unit that analyzes a characteristic of the signal in the selected frequency band, and generates a trigger signal triggering control of therapy to the patient based on the analyzed characteristic. The therapy delivery module controls the therapy in response to the trigger signal.

Frequency selective monitoring of physiological signals using a heterodyning architecture as described in this disclosure may provide one or more advantages. For example, a physiological signal may be monitored with reduced power, computing and memory requirements relative to techniques that rely on oversampling of the wideband signal followed by digital signal processing. Consequently, frequency selective monitoring may be readily implemented in medical devices with small sizes and limited power, computing and memory capabilities, such as implantable medical devices. In addition, a frequency selective monitor may be readily configurable, allowing a user to select different frequency bands and change frequency bands manually or automatically.

### BRIEF DESCRIPTION OF DRAWINGS

EP 2 200 692 B1

FIG. 1 is a block diagram illustrating an exemplary medical device that includes a frequency selective signal monitor capable of monitoring physiological signals associated with a patient in one or more selected frequency bands.

FIG. 2 is a block diagram illustrating an exemplary medical device that communicates with a sensor that includes a frequency selective signal monitor capable of monitoring physiological signals associated with a patient in one or more selected frequency bands.

FIG. 3 is a block diagram illustrating an exemplary frequency selective signal monitor that includes a chopper-stabilized amplifier and a signal analysis unit.

FIG. 4 is a block diagram illustrating a portion of an exemplary chopper-stabilized amplifier for use within the frequency selective signal monitor from FIG. 3.

FIGS. 5A-5D are graphs illustrating frequency components of a signal at various stages within the amplifier of FIG. 4.

FIG. 6A is a block diagram illustrating an exemplary frequency selective signal monitor that includes a chopper-stabilized superheterodyne amplifier and a signal analysis unit.

FIG. 6B is a block diagram illustrating an exemplary signal analysis unit that may receive multiple signals in different selected frequency bands from one or more superheterodyne instrumentation amplifiers.

FIG. 7 is a block diagram illustrating a portion of an exemplary chopper-stabilized superheterodyne amplifier for use within the frequency selective signal monitor from FIG. 6A.

FIGS. 8A-8D are graphs illustrating the frequency components of a signal at various stages within the superheterodyne amplifier of FIG. 7.

FIG. 9 is a block diagram illustrating a portion of an exemplary chopper-stabilized superheterodyne amplifier with in-phase and quadrature signal paths for use within a frequency selective signal monitor.

FIG. 10 is a flowchart that illustrates exemplary operation of a frequency selective signal monitor that includes a chopper-stabilized amplifier.

FIG. 11 is a flowchart that illustrates exemplary operation of a frequency selective signal monitor that includes a chopper-stabilized superheterodyne amplifier.

FIG. 12 is a circuit diagram illustrating a chopper-stabilized mixer amplifier suitable for use within the frequency selective signal monitor of FIG. 3 or FIG. 6A.

FIG. 13 is a circuit diagram illustrating a chopper-stabilized, superheterodyne instrumentation amplifier with differential inputs.

FIG. 14 is a block diagram illustrating a portion of an exemplary chopper-stabilized superheterodyne amplifier with in-phase and quadrature signal paths, as shown in FIG. 9, with the addition of optional impedance measurement circuitry.

FIG. 15 is a block diagram illustrating a portion of an exemplary chopper-stabilized superheterodyne amplifier with in-phase and quadrature signal paths, as shown in FIG. 9, with the addition of a digital signal processor.

FIG. 16 is a block diagram illustrating a sensing device integrated with a neurostimulator.

FIG. 17 is another circuit diagram illustrating a chopper-stabilized mixer amplifier suitable for use within a frequency selective signal monitor.

FIG. 18 is a circuit diagram illustrating a low pass filter suitable for use within a frequency selective signal monitor.

FIG. 19 is a circuit diagram illustrating an example output power block to extract power from the output signal of a chopper-stabilized, superheterodyne instrumentation amplifier.

FIG. 20 is a circuit diagram illustrating a clock circuit to generate a clock frequency for a chopper-stabilized, superheterodyne instrumentation amplifier.

FIG. 21 is a circuit diagram illustrating a multi-channel array of chopper-stabilized, superheterodyne instrumentation amplifiers.

FIG. 22 is a block diagram illustrating an example algorithm that can be run within the sensing device of FIG. 16.

FIG. 23 is a conceptual diagram illustrating a lead placement arrangement that exploits the reciprocity theorem.

FIG. 24 is a diagram illustrating the broad power tuning capabilities of a superheterodyne frequency selective signal monitor according to this disclosure.

FIG. 25 is a diagram illustrating noise characteristics of a superheterodyne frequency selective signal monitor according to this disclosure.

FIG. 26 is a block diagram of another example superheterodyning, chopper-stabilized instrumentation amplifier that may be useful within a frequency-selective signal monitor.

FIG. 27 is a circuit diagram illustrating a programmable differential gain amplifier suitable for use within the superheterodyne instrumentation amplifier of FIG. 26.

## DETAILED DESCRIPTION

In general, the invention is directed to a frequency selective monitor and methods for monitoring physiological signals in one or more selected frequency bands. A frequency selective monitor may utilize a heterodyning, chopper-stabilized amplifier architecture to convert a selected frequency band to a baseband for analysis. The frequency selective monitor may be useful in a variety of therapeutic and/or diagnostic applications to monitor a variety of physiological signals, such as EEG, ECoG, ECG, EMG, pressure, temperature, impedance, motion, and other types of signals. For purposes of illustration, however, frequency selective monitors will be generally described with respect to monitoring and analysis of brain signals and, particularly, one or more selected frequency bands of EEG or ECoG signals, such as alpha, beta and gamma bands. Other examples of brain signals, in addition to EEG and ECoG signals, include local field potentials (LFP's) and single cell action potentials.

A frequency selective signal monitor may be provided within a medical device or within a sensor coupled to a medical device. The physiological signal may be analyzed in one or more selected frequency bands to trigger delivery of patient therapy and/or recording of diagnostic information. For example, a frequency selective monitor may be provided within or operate in conjunction with electrical stimulation devices, drug delivery devices, loop recorders, or the like, including external or implantable stimulators, drug delivery devices, or loop recorders. Other examples of therapy devices include devices configured to provide visual, audible or tactile cueing, e.g., to break akinesia such as gait freeze or other motor freezes.

Examples of stimulation devices include electrical stimulators configured for deep brain stimulation, spinal cord stimulation, gastric stimulation, cardiac stimulation, pelvic floor stimulation, peripheral nerve stimulation or the like. Therapeutic applications include, without limitation, delivery of stimulation to treat diseases or disorders such as chronic pain, epilepsy, Parkinson's disease, dystonia, tremor, akinesia, neuralgia, sleep dysfunction, depression, obsessive compulsive disorder, obesity, gastroparesis, urinary or fecal incontinence, sexual dysfunction or the like. For purpose of illustration, however, frequency selective monitors will be generally described with respect to electrical stimulation configured to treat neurological diseases or disorders such as Parkinson's, tremor, epilepsy, depression, obsessive compulsive disorder or the like.

A frequency selective monitor may include a heterodyning circuit configured to convert a selected frequency band of the physiological signal to a baseband. The heterodyning circuit may modulate a physiological signal at a first frequency, amplify the modulated signal, and demodulate the amplified signal at a second frequency. The second frequency may be different from the first frequency. In particular, the second frequency may differ from the first frequency by an offset. The offset may correspond to a frequency within a selected frequency band, such as a center frequency of the selected frequency band. Demodulation of the amplified signal at the second frequency may substantially center the selected frequency band of the signal at baseband. For example, the center frequency of the selected frequency band may be substantially centered at DC, i.e., 0 Hz, facilitating analysis of the signal.

A frequency selective monitor as described herein may be configured to monitor a single frequency band of the wide band physiological signal. In addition, or alternatively, the techniques may be capable of efficiently hopping frequency bands in order to monitor the signal in two or more frequency bands. The frequency selective monitor may generate a triggering signal that triggers at least one of controlling therapy or recording diagnostic information based on analysis of the signal in one frequency band or multiple frequency bands. Therapy may be controlled by initiating delivery of therapy and/or adjusting therapy parameters. Recording diagnostic information may include recording the physiological signal, one or more characteristics of the signal, or other information.

As described in this disclosure, a superheterodyne-based, frequency selective signal monitor may efficiently extract signal power or other characteristics from a signal in a selected frequency band that is determined to be physiologically relevant. Local field potentials in the brain are complex, but can be analyzed with frequency domain techniques. Many key neurological biomarker potentials are encoded as variations in spectral content. Symptoms or conditions may be detected or evaluated, for example, by sensing power or power fluctuations in specific frequency bands of wide band physiological signals, such as EEG signals or ECoG signals. For EEG and ECoG signals, physical location of one or more electrodes, as sensing elements, maps functionality (e.g., motor, sensory, or other functionality) and frequency bands within the signal captured at the physical location encode the activity relating to such functionality.

Neuronal activity can be measured with a number of techniques, ranging in resolution from recordings of single cell action potentials, to local field potentials (LFPs), to ECoG signals, to the measurement of gross cortical activity with an electroencephalogram (EEG). In general, chronic sensing may present several high level requirements. For example, chronic sensing of such field potentials via an implanted sensing device may require the ability to operate with less than 25 microwatts of power, sufficiently low noise to support sensing of biomarkers in the cortex having potentials of less than 10 microvolts rms, and a power supply rejection ratio (PSRR) of greater than 80 dB to reject noise from other sources, such as an electrical stimulator integrated with or in close proximity to the sensing device. If a sensing device is integrated with an implantable electrical stimulator, for example, the combined device may have a power requirement for stimulation therapy on the order of 500 microwatts, which may leave approximately 25 microwatts for sensing.

Low frequency power fluctuations of neuronal local field potentials (LFPs) within discrete frequency bands can provide useful biomarkers for discriminating normal physiological brain activity from pathological states. LFPs may provide a measurement of the average or composite field behavior of many cells surrounding an electrode. Because LFPs represent the ensemble activity of thousands to millions of cells in an *in vivo* neural population, their recording generally avoids chronic issues like tissue encapsulation and micromotion encountered in single-unit recording. LFP biomarkers are ubiquitous and span a broad frequency spectrum, from approximately 1 Hz oscillations in deep sleep to greater than approximately 500 Hz "fast ripples" in the hippocampus, and show a wide Q variation. As an example, high gamma band power fluctuations in the motor cortex may signal motion intent.

Hence, using higher frequency bandpower tracking from signals that may have been previously filtered out of surface EEG recording may be desirable. However, high frequency bandpower tracking may exacerbate problems associated with the use of digital processing to track key biomarkers, e.g., due to the power penalty of Nyquist sampling and high-rate digital processing. As the LFP biomarkers increase in frequency, their encoding can be efficiently obtained using a circuit architecture that directly extracts energy at key neuronal bands and tracks the relatively slow power fluctuations.

A frequency selective signal monitor as described herein may analyze brain signals, such as EEG or ECoG signals, in the alpha, beta, and/or gamma bands to detect brain activity relating to a particular disorder. For example, a frequency selective signal monitor may be used to track power ratios of brain signals in the beta and gamma bands, or monitor higher gamma bands, e.g., for analysis relating to Parkinson's disease or other movement disorders. For example, the balance between 25 Hz beta waves and 50 Hz gamma waves may be hypothesized as a biomarker for a disease state relating to a movement disorder. Desynchronization of mu ($\mu$) waves (e.g., approximately 10 Hz) and an increase in power in high gamma waves (e.g., an increase of factor of four in 150 Hz waves) may also indicate a motion intent of the patient, i.e., an intent to move. As another example, a frequency selective signal monitor may be used to track desynchronization of alpha waves over the motor cortex, e.g., for analysis relating to Parkinson's disease or essential tremor. In this case, it may be possible to detect a patient intention for movement, permitting electrical stimulation or cueing to be delivered, e.g., to eliminate or reduce tremor or break akinesia. Implantable electrodes may be placed at selected locations within the brain and/or surface electrodes may be placed at selected locations on the head of the patient. In each case, the electrodes may be positioned to capture brain signals relating to particular functionality. As one example, electrodes may be positioned near the motor cortex to obtain signals indicative of movement. Analysis of one or more selected frequency bands, e.g., alpha, beta, gamma, in accordance with this disclosure then permits evaluation of different activity relating to such functionality.

As another example, a frequency selective signal monitor may track alpha wave balance between both hemispheres of the brain, e.g., as a biomarker for depression or compulsive behavior. A frequency selective signal monitor may trigger delivery of drug therapy or electrical stimulation to alleviate the depression. A frequency selective signal monitor may also identify a patient sleep state by monitoring the delta-theta-alpha-beta frequency bands in the EEG or ECoG of the patient to distinguish sleep stages of the patient and to trigger delivery of electrical stimulation to the patient during a REM sleep stage, e.g., to alleviate sleep dysfunction. For this example, the monitored frequency bands may fall in the ranges of approximately 1 Hz or lower (delta band), 4 to 8 Hz (theta band), 5 to 15 Hz (alpha band), and 15 to 35 Hz (beta band).

As a further example, a frequency selective signal monitor may identify epilepsy or onset of an epileptic seizure by monitoring a signal in the beta frequency band of the EEG or ECoG for the patient and trigger delivery of electrical stimulation to the patient to preempt, terminate, shorten or reduce severity of seizures. In addition, the frequency selective signal monitor may identify pain by monitoring a variety of frequency bands of the EEG or ECoG for the patient and trigger therapy, such as electrical stimulation or drug delivery, to the patient to alleviate the pain. Hence, a frequency selective monitor may be used for analysis relating to epilepsy, Parkinson's disease, tremor or other disorders, or to monitor other biomarker potentials in the cortex or elsewhere in selected frequency bands to detect patient pain or other patient sensations or activities.

Spectral encoding may be sensed to indicate other disease states or activities such as attention deficit hyperactivity disorder (ADHD), olfaction activity, sleep states or the like. In these and other examples above, field potential bandpower fluctuations may encode key physiological information that can be used to identify particular disease states, neurological states or patient activity. The ability to sense signals across various bands using a frequency selective signal monitor in accordance with this disclosure may be very helpful in promoting effective therapy and diagnosis.

Neurological states are generally encoded in specific frequency bands. Modulation of the spectral energy may provide information on general activity such as sleep stages, alert state, motor processing, or the like, as well as pathological states such as seizures, band power hemispheric imbalance indicative of depression, and excess beta activity indicative of Parkinson's disease. Although this modulation may range from several tens of Hertz to hundreds of Hertz, many targeted therapies and diagnostic processes may require only low bandwidth tracking of energy in specific bands. Hence, in accordance with this disclosure, a template for physiological sensing, and particularly brain sensing, is demodulation of amplitude modulated (AM) signals, i.e., where physiological information is encoded in low frequency variations within a neurological carrier frequency.

Tracking power fluctuations in physiological bands may provide information to drive therapy delivery and/or recording. A frequency selective monitor can exploit the coding properties of neural signals while eliminating the need for rapid sampling of the wide-band signal, and associated computational, memory and power costs. The frequency selective monitor may reduce the output signal to a bandpower measurement in one or more cortical or neural frequency bands. Bandpower may generally refer to a power measurement for a signal within a selected frequency band. With a superheterodyne architecture, a chopper-stabilized amplifier can down-select a specific band using the non-linear signal processing in a chopper-stabilized amplifier such that the amplifier may operate similar to a superheterodyne radio receiver. By using direct down-modulation of neural signals, powered bandpass filters that would otherwise be needed to select the frequency band can be eliminated and replaced with passive filters drawing no power. In some embodiments, two parallel channels may combine the in-phase and quadrature signals to extract the full power of the neural signal at selected frequencies, which may be programmed in nonvolatile chip memory.

The techniques described in this disclosure for monitoring a physiological signal in a selected frequency band may provide several advantages. For example, the techniques may provide a fast signal monitoring solution with low power overhead. In particular, there may be no need for over-sampling of a wide band physiological signal above the Nyquist frequency, followed by digital signal processing to analyze the sampled data. Instead, a frequency selective monitor may be configured to amplify and process an analog signal in a selected frequency band without analog to digital conversion of the sampled wide band signal. In other cases, the output of the frequency selective monitor may be digitized, but after the signal has been reduced to a band power level. Therefore, the techniques may be implemented within medical devices with small form-factors and limited power capabilities, such as implantable medical devices. Furthermore, the techniques may provide a solution that is highly configurable and allows a user, such as a physician, technician, or patient, to determine the selected frequency band in which to monitor the physiological signal for symptoms or conditions of the patient. In some embodiments, a heterodyning chopper amplifier may permit chronic sensing of brain signals, extraction of key biomarker information, and feedback to control therapy with low power electronics.

A circuit architecture that directly extracts energy at key neuronal bands and tracks the relatively slow power fluctuations is useful to monitor signals characterized by biomarker encoding. By partitioning the neural interface for analog extraction of the relevant power fluctuations before digitization, the back-end requirements for sampling, algorithms, memory, and telemetry may be reduced. A chopper stabilized, superheterodyne architecture may function to track the frequency power for a broad spectrum of neuronal biomarkers. Such a circuit may be constructed to merge chopper-stabilization with heterodyne signal processing to construct a low-noise amplifier with highly programmable, robust filtering characteristics. In some embodiments, the architecture can be tuned for center band selectivity from dc to 500 Hz using on-chip clocks, while the filter bandwidth is programmable from 5 to 25 Hz using an on-chip passive third-order lowpass filter. The filter configuration may be maintained with on-chip non-volatile memory. In addition to processing frequency biomarkers, the architecture can adapted to measure complex electrode and tissue impedance by supplying a stimulation current across the inputs and disabling the input chopper modulation.

Chopper stabilized amplifiers can be adapted to also provide wide dynamic range, high-Q filters. Chopper stabilization is an efficient architecture for amplifying low-frequency neural signals in micropower applications. Displacement of modulation and demodulation clocks within the chopper amplifier permits direct translation of the frequency of the signal. For example, an up-modulator may be set to a first frequency. The resulting up-modulated signal is then centered about the first modulation frequency, which may be selected to be well above excess aggressor noise. Demodulation is then performed with a second clock of frequency equal to the first frequency plus or minus an offset $\delta$. The net deconvolution of the signal and the demodulation frequency re-centers the signal to dc and two times the offset ($2\delta$). Since biomarkers, are encoded as low frequency fluctuations of the spectral power, it is possible to filter out the $2\delta$ component with an on-chip lowpass filter with a bandwidth defined as BW/2. Signals on either side of $\delta$ are aliased into the net pass-band at the output signal. To first order, the heterodyned chopper may extract a band equivalent to a sixth-order bandpass filter with a scale factor penalty of $4/\pi^2$.

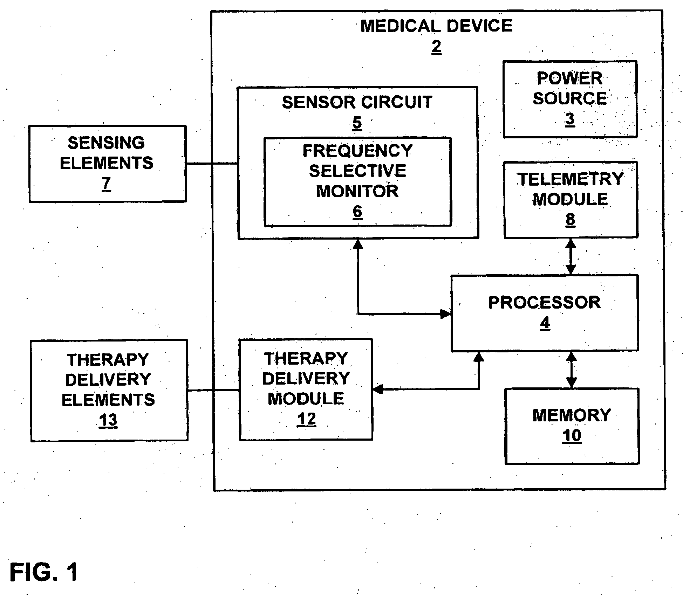

FIG. 1 is a block diagram illustrating an exemplary medical device 2 that includes a frequency selective signal monitor 6 capable of monitoring physiological signals associated with a patient in selected frequency bands. The physiological signals may be relatively low frequency signals, and may have frequency bands of interest in a range of approximately 1 to 1000 Hertz (Hz) and, more particularly, in a range of approximately 1 to 500 Hz. For example, 1 Hz oscillations may be relevant for sleep state analysis, while fast ripples in a range of approximately 200 to 500 Hz or higher may be relevant for analysis of epilepsy. In general, frequencies in the selected frequency band are less than or equal to approximately 1000 Hz, more particularly less than or equal to approximately 500 Hz, and still more particularly less than or equal to approximately 100 Hz. For EEG signals, as an example, selected frequency bands may fall in the ranges of approximately 5 to 15 Hz (alpha band), 15 to 35 Hz (beta band), and 30 to 80 Hz (gamma band). Characteristics of the signal in selected frequency bands may be useful in controlling therapy, such as electrical stimulation or drug delivery, either by initiation of delivery of therapy or adjustment of therapy parameters. Adjustment of therapy parameters may include adjustment of pulse amplitude, pulse rate, pulse width, electrode combination or the like for electrical stimulation, or adjustment of dosage, rate, frequency, lockout interval, or the like for drug delivery.

As illustrated in FIG. 1, medical device 2 may also include a power source 3, such as a rechargeable or nonrechargeable battery, a processor 4, a telemetry module 8, a memory 10, and a therapy delivery module 12. In addition, in the example of FIG. 1, frequency selective signal monitor 6 is connected to sensing elements 7 positioned at a desired location relative to the patient that detect the physiological signal. Sensing elements 7 may include a set of electrodes for sensing electrical signals. The electrodes may be, for example, implantable electrodes deployed on a lead or external surface electrodes. Sensing elements 7 may be deployed at selected tissue sites or on selected surfaces of a human patient, such as within the brain, proximate the spinal cord, on the scalp, or elsewhere. As an example, scalp electrodes may be used to measure or record EEG signals. As another example, electrodes implanted at the surface of the cortex may be used to measure or record ECoG signals. Therapy delivery module 12 may be connected to therapy delivery elements 13, such as one or more electrodes deployed on a lead or drug delivery conduits, which may be positioned at a desired location relative to the patient to deliver therapy to the patient in response to the monitored physiological signal.

In some embodiments, medical device 2 may comprise an implantable medical device capable of being implanted within the patient. In this case, sensing elements 7 may be positioned at a desired location within the patient to detect the physiological signal. Further, therapy delivery elements may be positioned at a desired location within the patient to deliver the therapy, such as electrical stimulation, drug delivery or internal audio or visual cueing. In other embodiments, medical device 2 may comprise an external medical device with sensing elements positioned at a desired location adjacent the patient to detect the physiological signal. In addition, therapy delivery elements 13 may be positioned at a desired location external to the patient to deliver the therapy, such as external audio, visual or tactile cueing via lights, displays, speakers, or the like.

Processor 4, frequency selective signal monitor 6, telemetry module 8, memory 10, and therapy delivery module 12 receive operating power from power source 3. Power source 3 may take the form of a small, rechargeable or non-rechargeable battery, or an inductive power interface that receives inductively coupled energy. In the case of a rechargeable battery, power source 3 similarly may include an inductive power interface for transfer of recharge power.

Processor 4 may include one or more microprocessors, microcontrollers, digital signal processors (DSPs), application specific integrated circuits (ASICs), field programmable gate array (FPGAs), discrete logic circuitry, or a combination of such components. Memory 10 may store therapy instructions that are available to be selected by processor 4 in response to receiving a patient therapy trigger from frequency selective signal monitor 6. In addition, processor 4 may be configured to record diagnostic information, such as sensed signals, signal characteristics, or the like in memory 10 or another memory or storage device. Memory 10 may include any combination of volatile, non-volatile, removable, magnetic, optical, or solid state media, such as read-only memory (ROM), random access memory (RAM), electronically-erasable programmable ROM (EEPROM), flash memory, or the like.

Frequency selective signal monitor 6 may form part of a sensor circuit 5 configured to monitor a variety of signals via a variety of different sensing elements 7, such as a pressure sensing element, an accelerometer, an activity monitor, an impedance monitor, an electrical signal monitor or other monitor configured to monitor heart sounds, brain signals, and/or other physiological signals. As an illustration, sensing elements 7 may comprise one or more electrodes located on a lead implanted at a target site within the patient and electrically coupled to sensor 5 via conductors. Frequency selective monitor 6 monitors the signals obtained by sensor circuit 5. Sensor circuit 5 may include suitable electrical interconnections to sensing elements and other components, as necessary. In some embodiments, frequency selective monitor 6 may directly process signals obtained from sensing elements 7 with little or no preprocessing by other components within sensor circuit 5. In other embodiments, sensor circuit 5 may include preprocessing circuitry to process or convert signals from sensing elements 7 for monitoring by frequency selective monitor 6.

A lead may carry one electrode or multiple electrodes, such as ring electrodes, segmented electrodes or electrodes arranged in a planar or non-planar array, e.g., on a paddle lead. Medical device 2 may be implantable or external. Such leads may carry sense electrodes or a combination of sense and stimulation electrodes. In some cases, different leads may be dedicated to sensing and stimulation functions. If external, medical device 2 may be coupled to one or more leads carrying sense and/or stimulation electrodes via a percutaneous extension. As a further illustration, sensing elements 7 may be surface electrodes suitable for placement on scalp, face, chest, or elsewhere on a patient, in which case such electrodes may be coupled to sensor circuit 5 via conductors within external leads. Sensing elements 7 may further comprise combinations of electrodes provided on one or more implantable leads and on or within a housing of medical device 2, or other electrode arrangements. Sensor circuitry associated with sensing elements 7 may be provided within frequency selective signal monitor 6.

In general, sensing elements 7 provide a measurement of a physiological signal associated with the patient by translating the signal to an output voltage our current. Frequency selective signal monitor 6 monitors the physiological signal in a selected frequency band without the need for rapid oversampling to digitize the signal. Instead, frequency selective signal monitor 6 may be configured to tune to a selected frequency band within the analog physiological signal. For example, frequency selective signal monitor 6 may be configured to modulate the wide band physiological signal at a first frequency, amplify the signal, demodulate the signal at a second, different frequency to baseband, extract the signal in a selected frequency band from the wide band physiological signal, and measure a characteristic of the extracted signal, such as power. In this way, the measured power may be used to determine whether frequency selective signal monitor 6 outputs a trigger signal to processor 4 to control therapy and/or record diagnostic information.

Processor 4 may receive the trigger signal and initiate delivery of therapy or adjust one or more therapy parameters specified in memory 10. Processor 4 outputs therapy instructions to therapy delivery module 12 to initiate or adjust delivery of therapy. Therapy delivery module 12 may include a stimulation generator that delivers stimulation therapy to the patient via therapy delivery elements 13 in response to receiving the therapy instructions. Therapy delivery elements 13 may be electrodes carried on one or more leads, electrodes on the housing of medical device 2, or electrodes on both a lead and the medical device housing. Alternatively, therapy delivery module 12 may include a fluid delivery device, such as a drug delivery device, including a fluid reservoir and one or more fluid delivery conduits. For cueing applications, therapy delivery module 12 may include one or more speakers, one or more lights, one or more display screens, or any combination thereof.

In some cases, as described above, therapy delivery module 12 may include a stimulation generator or other stimulation circuitry that delivers electrical signals, e.g., pulses or substantially continuous signals, such as sinusoidal signals, to the patient via at least some of the electrodes that form therapy delivery elements 13 under the control of the therapy instructions received from processor 4. Processor 4 may control therapy delivery module 12 to deliver electrical stimulation with pulse voltage or current amplitudes, pulse widths, and frequencies (i.e., pulse rates), and electrode combinations specified by the programs of the selected therapy instructions, e.g., as stored in memory 10. Processor 4 may also control therapy delivery module 12 to deliver each pulse, or a burst of pulses, according to a different program of the therapy instructions, such that multiple programs of stimulation are delivered an interleaved or alternating basis. In some embodiments, processor 4 may control therapy delivery module 12 to deliver a substantially continuous stimulation waveform rather than pulsed stimulation.

In other cases, as described above, therapy delivery module 12 may include a one or more fluid reservoirs and one or more pump units that pump fluid from the fluid reservoirs to the target site through the fluid delivery devices that form therapy delivery elements 13 under the control of the therapy instructions received from processor 4. For example, processor 4 may control which drugs are delivered and the dosage, rate and lockout interval of the drugs delivered. The fluid reservoirs may contain a drug or mixture of drugs. The fluid reservoirs may provide access for filling, e.g., by percutaneous injection of fluid via a self-sealing injection port. The fluid delivery devices may comprise, for example, fluid delivery conduits in the form of catheters that deliver, i.e., infuse or disperse, drugs from the fluid reservoirs to the same or different target sites.

In some cases, therapy delivery module 12 may include an audio signal generator, a visual signal, or a tactile stimulus (e.g., vibration) generator for cueing to disrupt akinesia or treat other conditions. Processor 4 may control therapy delivery module 12 to deliver audio, visual or tactile cueing with different parameters, such as amplitude, frequency, or the like, as specified by programs stored in memory 26.

Processor 4 also may control a telemetry module 8 to exchange information with an external programmer, such as a clinician programmer and/or patient programmer, by wireless, radio frequency (RF) telemetry. Processor 4 may control telemetry module 8 to communicate with the external programmer on a continuous basis, at periodic intervals, or upon request from the programmer. The programmer may, in turn, be connected to a computer that can program the device for algorithm and sensing adjustments, for issuing commands, for uplinking recorded loop data and for providing analysis. In addition, in some embodiments, telemetry module 8 may support wireless communication with one or more wireless sensors or sensing elements that sense physiological signals and transmit the signals to frequency selective signal monitor 6 by wireless transmission.

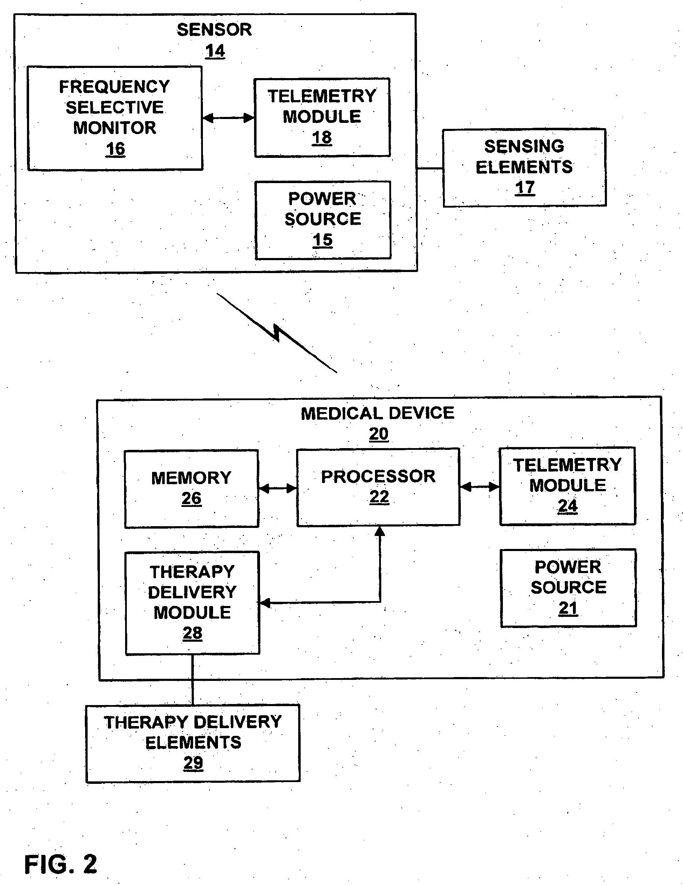

FIG. 2 is a block diagram illustrating an exemplary medical device 20 that communicates with a sensor 14 that includes a frequency selective signal monitor 16 capable of monitoring physiological signals associated with a patient in selected frequency bands. Medical device 20 may operate substantially similar to medical device 2 from FIG. 1 except that medical device 20 receives trigger signals from a frequency selective signal monitor 16 that is included in a sensor 14 separate from medical device 20.

As illustrated in FIG. 2, medical device 20 also includes a power source 21, a processor 22, a telemetry module 24, a memory 26, and a therapy delivery module 28. In addition, therapy delivery module 28 is connected to therapy delivery elements 29 positioned at a desired location relative to the patient to delivery therapy to the patient in response to the physiological signal monitored by sensor 14. Sensor 14 also includes a power source 15 and a telemetry module 18 capable of communicating with telemetry module 24 within medical device 20. In addition, frequency selective signal monitor 16 within sensor 14 may be electrically coupled to sensing elements 17 positioned at a desired location relative to the patient to monitor a physiological signal. As in the example of FIG. 1, sensor 14 may include additional components for preprocessing or conversion of physiological signals obtained from sensing elements 17. Alternatively, frequency selective monitor 16 may be directly connected to sensing elements 17 to receive the physiological signals.

Within sensor 14, frequency selective signal monitor 16 and telemetry module 18 receive operating power from power source 15. Within medical device 20, processor 22, telemetry module 24, memory 26, and therapy delivery module 28 receive operating power from power source 21. Power sources 15 and 21 may take the form of small, rechargeable or non-rechargeable batteries, or inductive power interfaces that receive inductively coupled energy. In the case of rechargeable batteries, power sources 15 and 21 similarly may include inductive power interfaces for transfer of recharge power.

In some embodiments, medical device 20 may comprise an implantable medical device capable of being implanted within the patient. Therapy delivery elements29 may be positioned at a desired location within the patient to deliver the therapy, such as electrical stimulation, drug delivery, or internal audio or visual cueing. In one case, sensor 14 may comprise an external sensor capable of communicating with medical device 20, and sensing elements 17 may be positioned at a desired location adjacent to or on a surface of the patient to detect the physiological signal. In another case, sensor 14 may comprise an implantable sensor capable of communicating with medical device 20, and sensing elements 17 may be positioned at a desired location within the patient to detect the physiological signal.

In other embodiments, medical device 20 may comprise an external medical device with therapy delivery elements 29 positioned at a desired location external to the patient to deliver the therapy, such as external audio cueing or visual cueing. An external medical device alternatively may delivery therapy via percutaneous leads or conduits. In one case, sensor 14 may comprise an external sensor capable of communicating with medical device 20, and sensing elements 17 may be positioned at a desired location adjacent the patient to detect the physiological signal. In another case, sensor 14 may comprise an implantable sensor capable of communicating with medical device 20, and sensing elements 17 may be positioned at a desired location within the patient to detect the physiological signal.

In general, sensing elements 17 may provide a wide band physiological signal associated with the patient in the form of a voltage or current signal. Frequency selective signal monitor 16 monitors the physiological signal in a selected frequency band. As in the example of FIG. 1, frequency selective signal monitor 16 may include a heterodyning circuit configured to convert a selected frequency band of the physiological signal to a baseband. The heterodyning circuit may modulate the wide band physiological signal at a first frequency, amplify the modulated signal, demodulate the amplified signal at a second frequency to baseband, extract the signal in a selected frequency band from the wide band physiological signal, and measure a characteristic of the extracted signal, such as power. Again, measured power or other measured characteristics may be used to determine whether frequency selective signal monitor 16 within sensor 14 outputs a trigger signal to medical device 20 to control delivery of therapy and/or cause medical device 20 to record diagnostic information. For example, frequency selective signal monitor 16 may output the trigger signal to processor 22 within medical device 20 via telemetry module 18 within sensor 20 and telemetry module 28 within medical device 20.

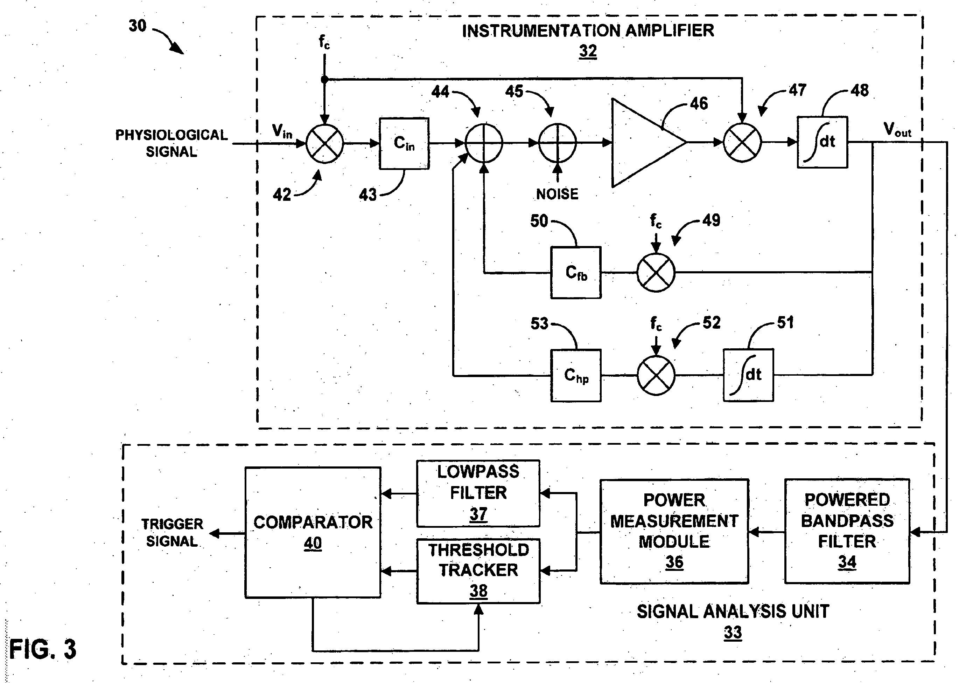

FIG. 3 is a block diagram illustrating an exemplary frequency selective signal monitor 30 that includes a chopper-stabilized instrumentation amplifier 32 and a signal analysis unit 33. In some cases, signal monitor 30 may be utilized within a medical device substantially similar to frequency selective signal monitor 6 within medical device 2 from FIG. 1. In other cases, signal monitor 30 may be utilized within a sensor that communicates with a medical device substantially similar to frequency selective signal monitor 16 within sensor 14 from FIG. 2.

As illustrated in FIG. 3, instrumentation amplifier 32 receives physiological signal ($V_{in}$) from sensing elements positioned at a desired location within a patient or external to a patient to detect the physiological signal. The physiological signal $V_{in}$ may be a voltage signal. In other cases, the physiological signal may be a current signal or impedance. The physiological signal may comprise, for example, one of an EEG, ECoG, EMG, EDG, pressure, temperature, impedance or motion signal. An EEG signal will be described for purposes of illustration. Instrumentation amplifier 32 may be configured to receive a physiological signal ($V_{in}$) as either a differential or signal-ended input. Instrumentation amplifier 32 includes a first modulator 42 for modulating the physiological signal from baseband at the a first carrier frequency ($f_c$). An input capacitance ($C_{in}$) 43 may be provided to couple the output of first modulator 42 to feedback adder 44. Feedback adder 44 will be described below in conjunction with the feedback paths.

Adder 45 represents the inclusion of a noise signal with the modulated signal. Adder 45 represents the addition of low frequency noise, but does not form an actual component of instrumentation amplifier 32. Hence, there is no addition of explicit noise. Rather, adder 45 models the noise that comes into instrumentation amplifier 32 from non-ideal transistor characteristics. At adder 45, the original baseband components of the signal are located at the carrier frequency $f_c$. The baseband components of the physiological signal may have a frequency within a range of 0 to less than or equal to approximately 1000 Hz, more particularly, 500 Hz, and still more particularly less than 100 Hz: The carrier frequency $f_c$ may be approximately 4 kHz to approximately 10 kHz. The noise signal enters the signal pathway at adder 45 to produce a noisy modulated signal. The noise signal may include 1/f noise, popcorn noise, offset, and any other external signals that may enter the signal pathway at low (baseband) frequency. At adder 45, however, the original baseband components of the signal have already been chopped to a higher frequency band by modulation of the physiological signal at the carrier frequency $f_c$ by first modulator 42. Thus, the low frequency noise signal is segregated from the original baseband components of the incoming physiological signal. The clock signal at frequency $f_c$ may be a square wave.

Amplifier 46 receives the noisy modulated input signal. Amplifier 46 amplifies the noisy modulated signal and outputs the amplified signal to a second modulator 47. In the example of FIG. 3, second modulator 47 demodulates the amplified signal at the carrier frequency $f_c$. That is, second modulator 47 modulates the noise signal up to the carrier frequency and demodulates the original baseband components of the physiological signal from the carrier frequency back to baseband. Baseband may refer to a band centered at DC, i.e., 0 Hz. Second modulator 47 is supplied with the same carrier frequency $f_c$ as first modulator 42 to demodulate the amplified signal 27. Integrator 48 operates on the demodulated signal to pass the baseband components of the signal and substantially eliminate the components of the noise signal at the carrier frequency $f_c$. In this manner, integrator 48 provides compensation and filtering to the amplified signal to produce an output signal ($V_{out}$). In other embodiments, compensation and filtering may be provided by other circuitry.

As shown in FIG. 3, instrumentation amplifier 32 may include two negative feedback paths to feedback adder 44 to reduce glitching in the output signal ($V_{out}$). In particular, the first feedback path includes a third modulator 49, which modulates the output signal at the carrier frequency $f_c$, and a feedback capacitance ($C_{fb}$) 50 that is selected to produce desired gain given the value of the input capacitance ($C_{in}$) 43. The first feedback path produces a feedback signal that is added to the original modulated signal at feedback adder 44 to produce attenuation and thereby generate gain at the output of amplifier 46.

The second feedback path is optional and includes an integrator 51, a fourth modulator 52, and high pass filter capacitance ($C_{hp}$) 53. Integrator 51 integrates the output signal and modulator 52 modulates the output of integrator 51 at the carrier frequency. High pass filter capacitance ($C_{hp}$) 53 may be selected to substantially eliminate components of the signal that have a frequency below the corner frequency of the high pass filter. For example, the second feedback path may set a corner frequency of approximately equal to 2.5 Hz, 0.5 Hz, or 0.05 Hz. The second feedback path may be provided to produce a feedback signal that is added to the original modulated signal at feedback adder 44. The second feedback path may act as a long term average or median filter to compensate for the long term behavior of the output signal ($V_{out}$). In other words, the second feedback path may subtract out or remove gradual drifts or other long-term behavior that occurs within the output signal.

A chopper-stabilized instrumentation amplifier, such as amplifier 32, may provide several advantages that make it useful for monitoring physiological signals. Three key benefits include an accurate monolithic high-pass corner, tight gain sensitivity, and low noise while operating at 1.8V. The accuracy of the high-pass filter in the second feedback path arises from the switched capacitor implementation. Since the high-pass filters are fully integrated, amplifier 46 can be scaled for large electrode arrays with minimal area penalty on the hybrid. In addition, the ability to digitally set the high-pass filter enables dynamic transient recovery post-therapy, as long as the states of the filter capacitors are preserved during transitions. The use of on-chip caps for the highpass filter also contributes to the tight sensitivity. The gain of amplifier 46 may be set by the ratio of two on-chip poly-poly caps. The low noise results from the core amplifier cell that eliminates the majority of 1/f noise and distributes currents efficiently, and the ability to provide significant gain in the front end to eliminate secondary stage contributions.

As illustrated in FIG. 3, signal analysis unit 33 receives the output signal $V_{out}$ from instrumentation amplifier. In the example of FIG. 3, signal analysis unit 33 includes a powered bandpass filter 34, a power measurement module 36, a lowpass filter 37, a threshold tracker 38 and a comparator 40. Powered bandpass filter 34 may comprise a tunable bandpass filter such that powered bandpass filter 34 may be tuned to pass the signal in a selected frequency band. In some cases, powered bandpass filter 34 may be manually tuned to the selected frequency band by a physician, technician, or the patient. In other cases, the powered bandpass filter 34 may by dynamically tuned to the selected frequency band in accordance with stored frequency band values. For example, when monitoring akinesia, the selected frequency band may be the alpha frequency band (5 Hz - 15 Hz). As another example, when monitoring tremor, the selected frequency band may be the beta frequency band (15 Hz - 35 Hz). As another example, when monitoring intent in the cortex, the selected frequency band may be the high gamma frequency band (150 Hz - 200 Hz). When monitoring pre-seizure biomarkers in epilepsy, the selected frequency band may be the fast ripple band (e.g., on the order of 200 Hz-500 Hz). As another illustration, the selected frequency band passed by filter 34 may be the gamma band (30 Hz - 80 Hz), or a portion of the gamma band.

Powered bandpass filter 34 extracts the signal in the selected frequency band. Power measurement module 36 then measures power of the extracted signal. In some cases, power measurement module 36 may extract the net power in the desired band by full wave rectification. In other cases, power measurement module 36 may extract the net power in the desired band by squaring power calculation. The measured power is then filtered by lowpass filter 37 and applied to comparator 40. A threshold tracker 38 may be provided to track fluctuations in power measurements of the selected frequency band over a period of time in order to generate a baseline power threshold of the selected frequency band for the patient. Threshold tracker 38 applies the baseline power threshold to comparator 40 in response to receiving the measured power from power measurement module 36.

Comparator 40 compares the measured power from lowpass filter 37 with the baseline power threshold from threshold tracker 38. If the measured power is greater than the baseline power threshold, comparator 40 may output a trigger signal to a processor of a medical device. The trigger signal may be a therapy trigger signal that controls therapy, e.g., by initiating therapy delivery or adjusting one or more therapy parameters. Alternatively, comparator 40 may output the trigger signal as a diagnostic recording trigger to cause a processor of the medical device to record the signal, a diagnostic event, or other information for later retrieval and evaluation. When the measured power of the signal in the selected frequency band is greater than the baseline power threshold of the selected frequency band, the increase in energy may signify a need for therapy. For example, a high-power signal in the targeted frequency may indicate the occurrence of an involuntary biomarker symptomatic of the patient's condition for which therapy is delivered. Low frequency power fluctuations of discrete frequency bands also may provide useful biomarkers for discriminating normal physiological brain activity from pathological states. As another example, a high-power signal in the targeted frequency may indicate the occurrence of a voluntary biomarker non-symptomatic of the patient's condition for which therapy is delivered. In other words, the signal may indicate one or more symptoms of a disease or disorder, or one or more activities or states of a patient, such as movement, sleep, activity, or the like.

If the measured power is equal to or less than the baseline power threshold, comparator 40 may output a power tracking measurement to threshold tracker 38, as indicated by the line from comparator 40 to threshold tracker 38. In this way, the measured power of the signal in the selected frequency band may be used by the threshold tracker 38 to update and generate the baseline power threshold of the selected frequency band for the patient. Threshold tracker 38 may include a median filter that creates the baseline threshold level after filtering the power of the signal in the selected frequency band for several minutes. Hence, the baseline power threshold may be dynamically adjusted as the sensed signal changes over time.

In some cases, frequency selective signal monitor 30 may be limited to monitoring a single frequency band of the wide band physiological signal at any specific instant or over time. Alternatively, frequency selective signal monitor 30 may be capable of efficiently hopping frequency bands in order to monitor the signal in a first frequency band, monitor the signal in a second frequency band, and then determine whether to trigger therapy and/or diagnostic recording based on some combination of the monitored signals. For example, different frequency bands may be monitored on an alternating basis to support signal analysis techniques that rely on comparison or processing of characteristics associated with multiple frequency bands.

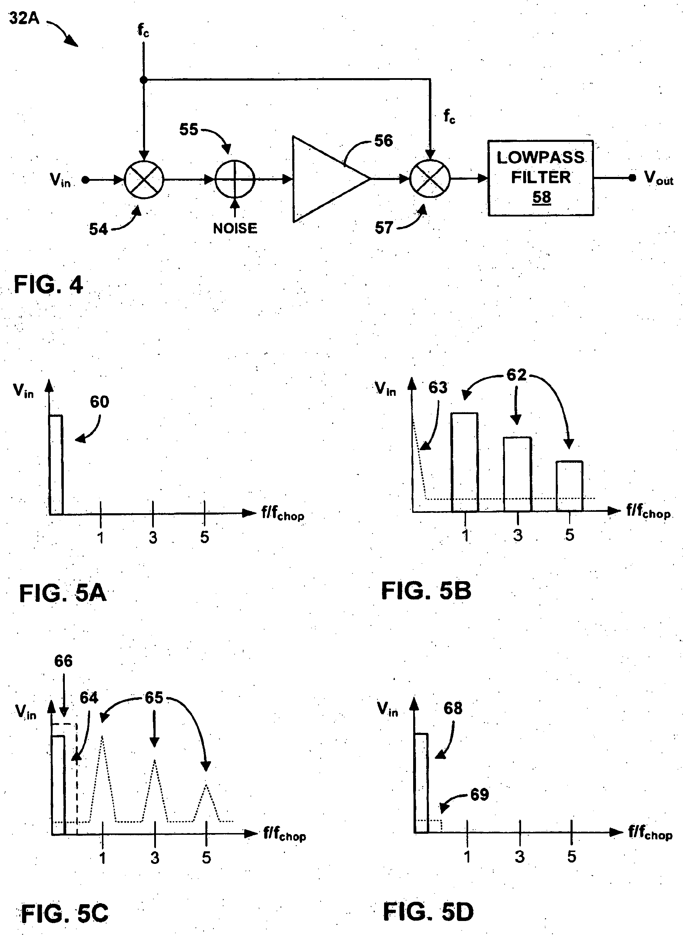

FIG. 4 is a block diagram illustrating a portion of an exemplary chopper-stabilized instrumentation amplifier 32A for use within frequency selective signal monitor 30 from FIG. 3. Instrumentation amplifier 32A illustrated in FIG. 4 operates substantially similar to instrumentation amplifier 32 from FIG. 3. Instrumentation amplifier 32A includes a first modulator 54, an adder 55 that represents addition of noise to the input signal, an amplifier 56, a second modulator 57, and a lowpass filter 58. In some embodiments, lowpass filter 58 may be an integrator, such as integrator 48 of FIG. 3.

Instrumentation amplifier 32A receives a physiological signal ($V_{in}$) associated with a patient from sensing elements, such as electrodes, positioned within or external to the patient to detect the physiological signal. First modulator 54 modulates the signal from baseband at the carrier frequency ($f_c$). Adder 55 represents the addition of a noise signal to the modulated signal and amplifier 56 amplifies the noisy modulated signal. However, adder 55 is not an actual component of instrumentation amplifier 32A. Adder 55 models the noise that comes into instrumentation amplifier 32 from non-ideal transistor characteristics. Second modulator 57 modulates the noisy amplified signal at the carrier frequency ($f_c$). In this way, the amplified signal is demodulated back to baseband and the noise signal is modulated at the carrier frequency $f_c$.

Lowpass filter 58 then filters the majority of the modulated noise signal out of the demodulated signal and outputs a low-noise physiological signal ($V_{out}$). The low-noise physiological signal may then be input to signal analysis unit 33 from FIG. 3. As described above, signal analysis unit 33 may extract the signal in a selected frequency band, measure power of the extracted signal, and compare the measured power to a baseline power threshold of the selected frequency band to determine whether to trigger patient therapy.

FIGS. 5A-5D are graphs illustrating the frequency components of a signal at various stages within instrumentation amplifier 32A of FIG. 4. In particular, FIG. 5A illustrates the frequency components of the physiological signal received by frequency selective signal monitor 30. The frequency components are represented by block 60 and located at baseband in FIG. 5A.

FIG. 5B illustrates the frequency components of the noisy modulated signal after amplification of the signal by amplifier 56. In FIG. 5B, the original baseband frequency components of the physiological signal are modulated and represented by blocks 62 at the odd harmonics. The frequency components of the noise signal added to the modulated signal are represented by dotted line 63. In FIG. 5B, the energy of the frequency components of the noise signal is located at baseband and energy of the frequency components of the desired physiological signal is located at the carrier frequency and its odd harmonics.

FIG. 5C illustrates the frequency components of the demodulated signal after demodulation by demodulator 57. In particular, the frequency components of the demodulated signal are located back at baseband and represented by block 64. The frequency components of the noise signal are up-modulated and represented by dotted line 65. The frequency components of the noise signal are located at the carrier frequency odd harmonics in FIG. 5C. FIG. 5C also illustrates the effect of lowpass filter 58 that may be applied to the demodulated signal. The passband of lowpass filter 58 is represented by dashed line 66.

FIG. 5D is a graph that illustrates the frequency components of the output signal. In FIG. 5D, the frequency components of the desired output signal are represented by block 68 and the frequency components of the noise signal are represented by dotted line 69. FIG. 5D illustrates that lowpass filter 58 removes the frequency components from the noise signal that were located outside of the passband of lowpass filter 58 shown in FIG. 5C. The energy from the noise signal is substantially eliminated from the output signal, or at least substantially reduced relative to the original noise signal that otherwise would be introduced.

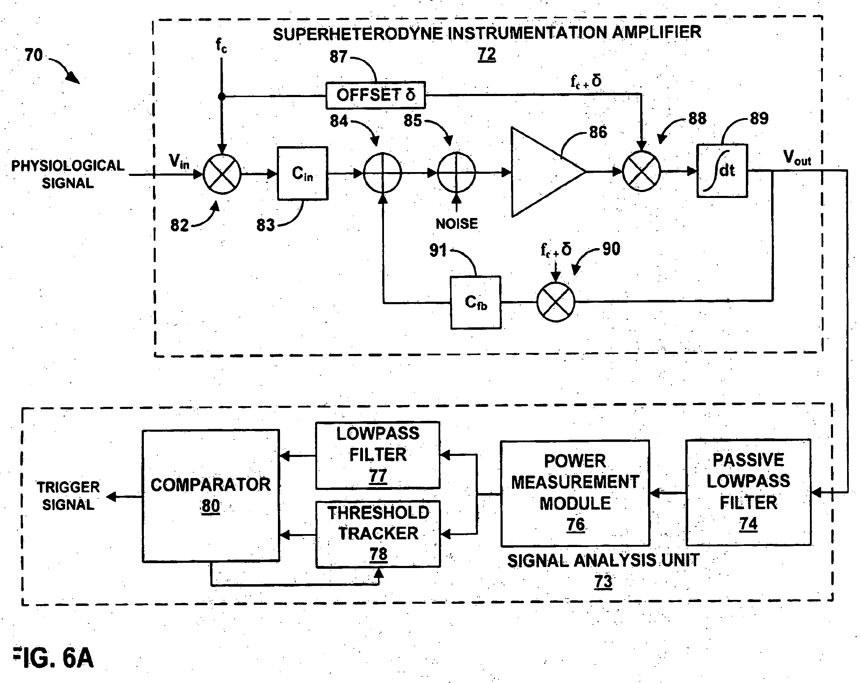

FIG. 6A is a block diagram illustrating an exemplary frequency selective signal monitor 70 that includes a chopper-stabilized superheterodyne instrumentation amplifier 72 and a signal analysis unit 73. In some cases, signal monitor 70 may be utilized within a medical device substantially similar to frequency selective signal monitor 6 within medical device 2 from FIG. 1. In other cases, monitor 70 may be utilized within a sensor that communicates with a medical device substantially similar to frequency selective signal monitor 16 within sensor 14 from FIG. 2. Monitor 70 may be configured to monitor any of the frequency bands described in this disclosure.

In general, frequency selective signal monitor 70 provides a physiological signal monitoring device comprising a physiological sensing element that receives a physiological signal, and a heterodyning circuit configured to convert a selected frequency band of the physiological signal to a baseband. The heterodyning circuit may correspond to instrumentation amplifier 72 or portions thereof. In one example, the heterodyning circuit may include a modulator 82 that modulates the signal at a first frequency, an amplifier 86 that amplifies the modulated signal, and a demodulator 88 that demodulates the amplified signal at a second frequency different from the first frequency. The device further comprises a signal analysis unit 73 that analyzes a characteristic of the signal in the selected frequency band. The second frequency is selected such that the demodulator substantially centers a selected frequency band of the signal at a baseband.

The signal analysis unit 73 may comprise a passive lowpass filter 74 that filters the demodulated signal to extract the selected frequency band of the signal at the baseband. The second frequency may differ from the first frequency by an offset that is approximately equal to a center frequency of the selected frequency band. In one embodiment, the physiological signal is an electroencephalogram (EEG) signal and the selected frequency band is one of an alpha, beta or gamma frequency band of the EEG signal. The characteristic of the demodulated signal may be a power fluctuation of the signal in the selected frequency band. The signal analysis unit 73 may generate a signal triggering at least one of control of therapy to the patient or recording of diagnostic information when the power fluctuation exceeds a threshold.

In some embodiments, the selected frequency band comprises a first selected frequency band and the characteristic comprises a first power. The demodulator 88 demodulates the amplified signal at a third frequency different from the first and second frequencies. The third frequency being selected such that the demodulator 88 substantially centers a second selected frequency band of the signal at a baseband. The signal analysis unit 73 analyzes a second power of the signal in the second selected frequency band, and calculates a power ratio between the first power and the second power. The signal analysis unit 73 generates a signal triggering at least one of control of therapy to the patient or recording of diagnostic information based on the power ratio.

In the example of FIG. 6A, chopper-stabilized, superheterodyne amplifier 72 modulates the physiological signal with a first carrier frequency $f_c$, amplifies the modulated signal, and demodulates the amplified signal to baseband with a second frequency equivalent to the first frequency $f_c$ plus (or minus) an offset $\delta$. The modulation signals at frequencies $f_c + \delta$ may be, for example, square wave signals. Signal analysis unit 73 measures a characteristic of the demodulated signal in a selected frequency band.

The second frequency is different from the first frequency $f_c$ and is selected, via the offset $\delta$, to position the demodulated signal in the selected frequency band at the baseband. In particular, the offset may be selected based on the selected frequency band. For example, the frequency band may be a frequency within the selected frequency band, such as a center frequency of the band.

If the selected frequency band is 5 to 15 Hz, for example, the offset $\delta$ may be the center frequency of this band, i.e., 10 Hz. In some embodiments, the offset $\delta$ may be a frequency elsewhere in the selected frequency band. However, the center frequency generally will be preferred. The second frequency may be generated by shifting the first frequency by the offset amount. Alternatively, the second frequency may be generated independently of the first frequency such that the difference between the first and second frequencies is the offset.

In either case, the second frequency may be equivalent to the first frequency $f_c$ plus or minus the offset $\delta$. If the first frequency $f_c$ is 4000 Hz, for example, and the selected frequency band is 5 to 15 Hz (the alpha band for EEG signals), the offset $\delta$ may be selected as the center frequency of that band, i.e., 10 Hz. In this case, the second frequency is the first frequency of 4000 Hz plus or minus 10 Hz. Using the superheterodyne structure, the signal is modulated at 4000 Hz by modulator 82, amplified by amplifier 86 and then demodulated by demodulator 88 at 3990 or 4010 Hz (the first frequency $f_c$ of 4000 Hz plus or minus the offset $\delta$ of 10 Hz) to position the 5 to 15 Hz band centered at 10 Hz at baseband, e.g., DC. In this manner the 5 to 15 Hz band can be directly downconverted such that it is substantially centered at DC.

As illustrated in FIG. 6A, superheterodyne instrumentation amplifier 72 receives a physiological signal (e.g., $V_{in}$) from sensing elements positioned at a desired location within a patient or external to a patient to detect the physiological signal. For example, the physiological signal may comprise one of an EEG, ECoG, EMG, EDG, pressure, temperature, impedance or motion signal. Again, an EEG signal will be described for purposes of illustration. However, ECoG or other types of brain signals including any of a variety of LFP's may be sensed, particularly for implantable applications. Superheterodyne instrumentation amplifier 72 may be configured to receive the physiological signal ($V_{in}$) as either a differential or signal-ended input. Superheterodyne instrumentation amplifier 72 includes first modulator 82 for modulating the physiological signal from baseband at the carrier frequency ($f_c$). In the example of FIG. 6A, an input capacitance ($C_{in}$) 83 couples the output of first modulator 82 to feedback adder 84. Feedback adder 84 will be described below in conjunction with the feedback paths.

Adder 85 represents the inclusion of a noise signal with the modulated signal. Adder 85 represents the addition of low frequency noise, but does not form an actual component of superheterodyne instrumentation amplifier 72. Adder 85 models the noise that comes into superheterodyne instrumentation amplifier 72 from non-ideal transistor characteristics. At adder 85, the original baseband components of the signal are located at the carrier frequency $f_c$. As an example, the baseband components of the signal may have a frequency within a range of 0 to approximately 1000 Hz and the carrier frequency $f_c$ may be approximately 4 kHz to approximately 10 kHz. The noise signal enters the signal pathway, as represented by adder 85, to produce a noisy modulated signal. The noise signal may include 1/f noise, popcorn noise, offset, and any other external signals that may enter the signal pathway at low (baseband) frequency. At adder 85, however, the original baseband components of the signal have already been chopped to a higher frequency band, e.g., 4000 Hz, by first modulator 82. Thus, the slow-frequency noise signal is segregated from the original baseband components of the signal.

Amplifier 86 receives the noisy modulated input signal from adder 85. Amplifier 86 amplifies the noisy modulated signal and outputs the amplified signal to a second modulator 88. Offset ($\delta$) 87 may be tuned such that it is approximately equal to a frequency within the selected frequency band, and preferably the center frequency of the selected frequency band. The resulting modulation frequency ($f_c \pm \delta$) used by demodulator 88 is then different from the first carrier frequency $f_c$ by the offset amount $\delta$. In some cases, offset $\delta$ 87 may be manually tuned according to the selected frequency band by a physician, technician, or the patient. In other cases, the offset $\delta$ 87 may by dynamically tuned to the selected frequency band in accordance with stored frequency band values. For example, different frequency bands may be scanned by automatically or manually tuning the offset $\delta$ according to center frequencies of the desired bands.

As an example, when monitoring akinesia, the selected frequency band may be the alpha frequency band (5 Hz to 15 Hz). In this case, the offset $\delta$ may be approximately the center frequency of the alpha band, i.e., 10 Hz. As another example, when monitoring tremor, the selected frequency band may be the beta frequency band (15 Hz-35 Hz). In this case, the offset $\delta$ may be approximately the center frequency of the beta band, i.e., 25 Hz. As another example, when monitoring intent in the cortex, the selected frequency band may be the high gamma frequency band (150 Hz - 200 Hz). In this case, the offset $\delta$ may be approximately the center frequency of the high gamma band, i.e., 175 Hz. When monitoring pre-seizure biomarkers in epilepsy, the selected frequency may be fast ripples (200 Hz - 500 Hz), in which case the offset $\delta$ may be approximately 500 Hz. As another illustration, the selected frequency band passed by filter 34 may be the gamma band (30 Hz - 80 Hz), in which case the offset $\delta$ may be tuned to approximately the center frequency of the gamma band, i.e., 55 Hz.

Hence, the signal in the selected frequency band may be produced by selecting the offset ($\delta$) 87 such that the carrier frequency plus or minus the offset frequency ($f_c \pm \delta$) is equal to a frequency within the selected frequency band, such as the center frequency of the selected frequency band. In each case, as explained above, the offset may be selected to correspond to the desired band. For example, an offset of 5 Hz would place the alpha band at the baseband frequency, e.g., DC, upon downconversion by the demodulator. Similarly, an offset of 15 Hz would place the beta band at DC upon downconversion, and an offset of 30 Hz would place the gamma band at DC upon downconversion. In this manner, the pertinent frequency band is centered at the baseband. Then, passive low pass filtering may be applied to select the frequency band. In this manner, the superheterodyne architecture serves to position the desired frequency band at baseband as a function of the selected offset frequency used to produce the second frequency for demodulation. In general, in the example of FIG. 6A, powered bandpass filtering is not required. Likewise, the selected frequency band can be obtained without the need for oversampling and digitization of the wideband signal.

With further reference to FIG. 6A, second modulator 88 demodulates the amplified signal at the second frequency $f_c \pm \delta$, which is separated from the carrier frequency $f_c$ by the offset $\delta$. That is, second modulator 88 modulates the noise signal up to the $f_c \pm \delta$ frequency and demodulates the components of the signal in the selected frequency band directly to baseband. Integrator 89 operates on the demodulated signal to pass the components of the signal in the selected frequency band positioned at baseband and substantially eliminate the components of the noise signal at higher frequencies. In this manner, integrator 89 provides compensation and filtering to the amplified signal to produce an output signal ($V_{out}$). In other embodiments, compensation and filtering may be provided by other circuitry.

As shown in FIG. 6A, superheterodyne instrumentation amplifier 72 may include a negative feedback path to feedback adder 84 to reduce glitching in the output signal ($V_{out}$). In particular, the feedback path includes a third modulator 90, which modulates the output signal at the carrier frequency plus or minus the offset $\delta$, and a feedback capacitance ($C_{fb}$) 91 that is selected to produce desired gain given the value of the input capacitance ($C_{in}$) 83. The feedback path produces a feedback signal that is added to the original modulated signal at feedback adder 84 to produce attenuation and thereby generate gain at the output of amplifier 86.

Compensation of the feedback path in the mixer amplifier may be achieved in several ways. The output stage of the amplifier may serve as an integrator for stabilizing the feedback path. Since the modulation in the chopper amplifier is correlated with that in the feedback path, the overall feedback path can be compensated by using a compensation capacitor, such as a 16 pF compensation capacitor, for example. In some embodiments, the compensation capacitor may stabilize the amplifier as an equivalent first-order system and the amplifier gain may eliminate the need for a compensation to zero. In one embodiment, a target bandwidth of 1 kHz may be selected and the feedback path may be scaled to achieve an equivalent gain ratio of 100. In such a case, a 0.4 heterodyning scale factor results from the clock differential between the input and feedback paths, but is not part of the synchronous closed-loop path and does not need to be included in that loop. In some embodiments, an additional feedback path similar to the second feedback path denoted by components 51, 52 and 53 illustrated in FIG. 3 may be used in conjunction with superheterodyne instrumentation amplifier 72.

As described above, chopper-stabilized, superheterodyne instrumentation amplifier 72 can be used to achieve direct downconversion of a selected frequency band centered at a frequency that is offset from baseband by an amount $\delta$. Again, if the alpha band is centered at 10 Hz, then the offset amount $\delta$ used to produce the demodulation frequency $f_c \pm \delta$ may be 10 Hz. As illustrated in FIG. 6A, first modulator 82 is run at the carrier frequency ($f_c$), which is specified by the 1/f corner and other constraints, while second modulator 88 is run at the selected frequency band ($f_c \pm \delta$). Multiplication of the physiological signal by the carrier frequency convolves the signal in the frequency domain. The net effect of upmodulation is to place the signal at the carrier frequency ($f_c$). By then running second modulator 88 at a different frequency ($f_c \pm \delta$), the convolution of the signal sends the signal in the selected frequency band to baseband and $2\delta$. Integrator 89 may be provided to filter out the $2\delta$ component and passes the baseband component of the signal in the selected frequency band. Thus, superheterodyne instrumentation amplifier 72 may have a dual role of both amplifying a physiological signal and of selecting the biomarker band. In one embodiment, amplifier 72 may amplify the physiological signal by 32dB, with a noise floor of under 150nV/rtHz while drawing 750nA, and translate the band-center of interest to DC.

Superheterodyne instrumentation amplifier 72 may operate under the concept of balancing an up-modulated charge from the differential input voltage with an upmodulated feedback charge, such that the net gain for the amplifier is set by the relative scaling of the input and feedback capacitors. As described above, the front end modulation clock may run on a clock signal independent from the demodulation amplifier and the feedback network. Thus, with amplification set by on-chip capacitor ratios, the relative clock difference, $\delta$, between the two clocks translates the relative frequency of the input signal by an equivalent amount to achieve the desired heterodyning transfer function:

$$V_{out}(f) = \frac{4}{\pi^2} \cdot \frac{C_{in}}{C_{fb}} \cdot \sum_{n,odd} \frac{1}{n^2} \cdot V_{in}(f + \delta \cdot n) \cdot \cos(\phi) \qquad (1)$$

where $C_{in}$ represents the input capacitance value, $C_{fb}$ represents the feedback capacitance value, n represents the harmonic order, f represents the carrier frequency, $\delta$ represents the frequency offset value, $\phi$ represents the phase between the demodulator clock and the physiological signal input, $V_{in}$ represents the physiological signal input, and $V_{out}$ represents the output of the instrumentation amplifier. In some embodiments, the ratio of input and feedback capacitances mary be set to 20pF/200fF in order to provide 32dB of gain. As illustrated in FIG. 6A, signal analysis unit 73 receives the output signal from instrumentation amplifier. In the example of FIG. 6A, signal analysis unit 73 includes a passive lowpass filter 74, a power measurement module 76, a lowpass filter 77, a threshold tracker 78 and a comparator 80. Passive lowpass filter 74 extracts the signal in the selected frequency band positioned at baseband. For example, lowpass filter 74 may be configured to reject frequencies above a desired frequency, thereby preserving the signal in the selected frequency band. Power measurement module 76 then measures power of the extracted signal. In some cases, power measurement module 76 may extract the net power in the desired band by full wave rectification. In other cases, power measurement module 76 may extract the net power in the desired band by a squaring power calculation, which may be provided by a squaring power circuit. By summing the squared power calculations, phase sensitivity can be reduced. The measured power is then filtered by lowpass filter 77 and applied to comparator 80. Threshold tracker 78 tracks fluctuations in power measurements of the selected frequency band over a period of time in order to generate a baseline power threshold of the selected frequency band for the patient. Threshold tracker 78 applies the baseline power threshold to comparator 80 in response to receiving the measured power from power measurement module 76.

Comparator 80 compares the measured power from lowpass filter 77 with the baseline power threshold from threshold tracker 78. If the measured power is greater than the baseline power threshold, comparator 80 may output a trigger signal to a processor of a medical device to control therapy and/or recording of diagnostic information, e.g., as described with reference to FIG. 3. If the measured power is equal to or less than the baseline power threshold, comparator 80 outputs a power tracking measurement to threshold tracker 78, as indicated by the line from comparator 80 to threshold tracker 78. Threshold tracker 78 may include a median filter that creates the baseline threshold level after filtering the power of the signal in the selected frequency band for several minutes. In this way, the measured power of the signal in the selected frequency band may be used by the threshold tracker 78 to update and generate the baseline power threshold of the selected frequency band for the patient. Hence, the baseline power threshold may be dynamically adjusted as the sensed signal changes over time. A signal above or below the baseline power threshold may signify an event that may support generation of a trigger signal.

As described with reference to FIG. 3, in some cases, frequency selective signal monitor 70 may be limited to monitoring a single frequency band of the wide band physiological signal at any specific instant. Alternatively, frequency selective signal monitor 70 may be capable of efficiently hopping frequency bands in order to monitor the signal in a first frequency band, monitor the signal in a second frequency band, and then determine whether to trigger therapy and/or diagnostic recording based on some combination of the monitored signals. For example, different frequency bands may be monitored on an alternating basis to support signal analysis techniques that rely on comparison or processing of characteristics associated with multiple frequency bands.

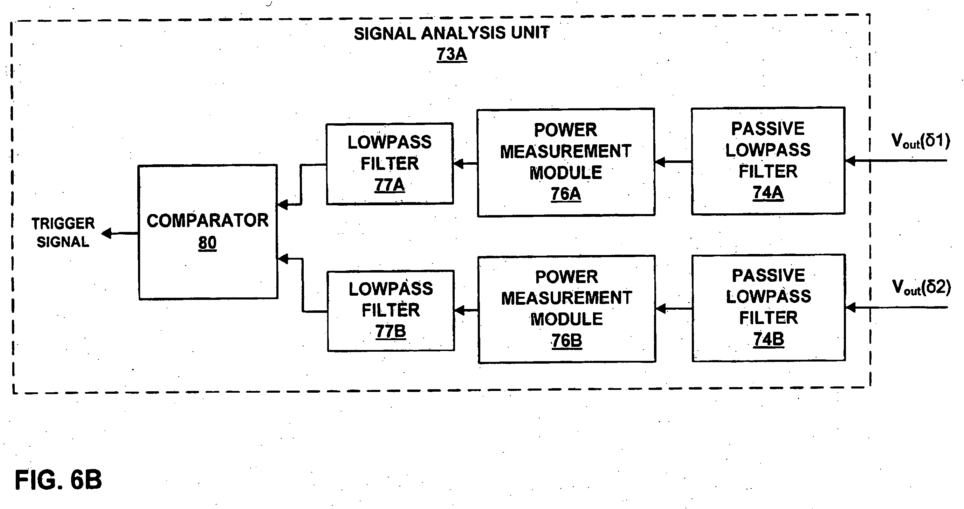

FIG. 6B is a block diagram illustrating an exemplary signal analysis unit 73A that may receive multiple signals in different selected frequency bands from one or more superheterodyne instrumentation amplifiers, such as instrumentation amplifier 72 from FIG. 6A. For example, signal analysis unit 73A may be a multichannel signal analysis unit providing simultaneous measurements in different bands. In the embodiment illustrated in FIG. 6B, signal analysis unit 73A may analyze a characteristic of each of the received signals in the different selected frequency bands in relation to the other received signals. In this way, signal analysis unit 73A may simultaneously measure power fluctuations in multiple frequency bands of the wide band physiological signal at any specific instant. In some cases, signal analysis unit 73A may analyze a power ratio between the power in different frequency bands to determine whether to generate a trigger signal.

In some cases, multiple bandpower ratios could be analyzed, e.g., a first bandpower ratio between first and second bands plus a second bandpower ratio between first and third, second and third, or third and fourth bands, where the bands are overlapping or non-overlapping. Alternatively, or additionally, signal analysis unit 73A may be configured to select different bands for measurement For example, signal analysis unit 73A may analyze a signal in a first selected frequency band to determine whether an event is indicated, e.g., by a deviation of bandpower from a threshold level. Then, if the signal in the first selected frequency band indicates an event, signal analysis unit 73A may analyze a signal in a second selected frequency band to confirm or validate the event before generating a trigger signal.

Alternatively, or additionally, signal analysis unit 73A may generate the trigger signal based on the measurement in the first selected frequency band and then proceed to analyze the signal in the second selected frequency band to determine whether to generate another trigger signal relating to another phase of therapy or data recording. Likewise, signal analysis unit 73A may selectively tune to different combinations of multiple bands to measure bandpower ratios to identify an event to generate a trigger signal, validate an event before generating a trigger signal, and/or generate a trigger signal followed by analysis of different bandpower ratios to determine whether to generate a trigger signal for the next phase of therapy or data recording. When tracking sleep and sleep states, as one example, it may be helpful to analyze bandpower fluctuations along a well-defined trajectory.

As another feature, signal analysis unit 73A may be configured to shift between a bandpower measurement mode in which power measurements are made based on offset delta and BW/2 that are focused on a band, and a raw signal analysis mode in which the raw signal is amplified for analysis. For example, signal analysis unit 73A may switch modes to analyze a raw EEG signal, e.g., to identify biomarkers during research or at the beginning of operation of an implantable stimulator. As an illustration, signal analysis unit 73A may be used to define seizure characteristics for a patient using raw EEG recording.

The raw EEG recording may be digitized and analyzed using a digital signal processor (DSP) or other digital processing device to analyze the signal for biomarkers. Once a pertinent frequency biomarker is identified, the bandpower measurement mode may be activated to add the offset delta shift and lowpass filter to implement the superheterodyne process for efficient low power operation. In this case, signal analysis unit 73A may operate in an initial mode to digitally analyze raw EEG signals and identify one or more biomarkers, and then transition to a second mode using the superheterodyne architecture to track events associated with the biomarkers, such as bandpower. The first mode may be a higher power mode, while the second, superheterodyne mode may be a lower power mode.

As another example, for epilepsy, signal analysis unit 73A may initially operate in a first mode that uses the superheterodyne architecture. If an event is detected in the first mode, then signal analysis unit 73A may transition to a second mode in which the raw EEG signal is digitally analyzed or recorded. In this case, the first mode using the superheterodyne architecture may be a lower power mode and the second mode involving digital analysis and/or loop recording may be a higher power mode. Features for switching between different bands or modes, as described above, may be generally applicable to signal analysis units 33, 73 and 73A or other signal analysis units similar to those described in this disclosure.

As illustrated in FIG. 6B, signal analysis unit 73A may comprise a first passive lowpass filter 74A that filters a first demodulated signal to extract a first selected frequency band of the signal at the baseband. Signal analysis unit 73A may also comprise a second passive lowpass filter 74B that filters a second demodulated signal to extract a second selected frequency band of the signal at the baseband, The characteristics of the first and second demodulated signals may be power fluctuations of the signals in the selected frequency bands. Signal analysis unit 73A may generate a signal triggering at least one of control of therapy to the patient or recording of diagnostic information based on the power ratio of the first and second signals.

Signal analysis unit 73A receives a first output signal from an instrumentation amplifier, such as instrumentation amplifier 72 from FIG. 6A. Signal analysis unit 73A also receives a second output signal from an instrumentation amplifier, which may be the same instrumentation amplifier or a different instrumentation amplifier as the first output signal. As shown in FIG. 6B, the first output signal was demodulated at a carrier frequency plus or minus a first offset ($\delta$1) tuned such that it is approximately equal to the center frequency of the first selected frequency band. Furthermore, the second output signal was demodulated at the carrier frequency plus or minus a second offset ($\delta$2) tuned such that it is approximately equal to the center frequency of the second selected frequency band.

In the example of FIG. 6B, signal analysis unit 73 includes first and second passive lowpass filters 74A, 74B, power measurement modules 76A, 76B, lowpass filters 77A, 77B, and a comparator 80A. First passive lowpass filter 74A extracts the first signal in the first selected frequency band positioned at baseband. Power measurement module 76A then measures power of the extracted first signal. The measured power is then filtered by lowpass filter 77A and applied to comparator 80A. Second passive lowpass filter 74B extracts the second signal in the second selected frequency band positioned at baseband. Power measurement module 76B then measures power of the extracted second signal. The measured power is then filtered by lowpass filter 77B and applied to comparator 80A.

Comparator 80A compares the measured power of the first signal from lowpass filter 77A with the measured power from the second signal from lowpass filter 77B. If the power ratio of the first and second signals is greater than a baseline power ratio threshold, comparator 80A may output a trigger signal to a processor of a medical device to control therapy and/or recording of diagnostic information, e.g., as described with reference to FIG. 3. In some embodiments, signal analysis unit 73A may be capable of receiving more than two signals in different selected frequency bands.

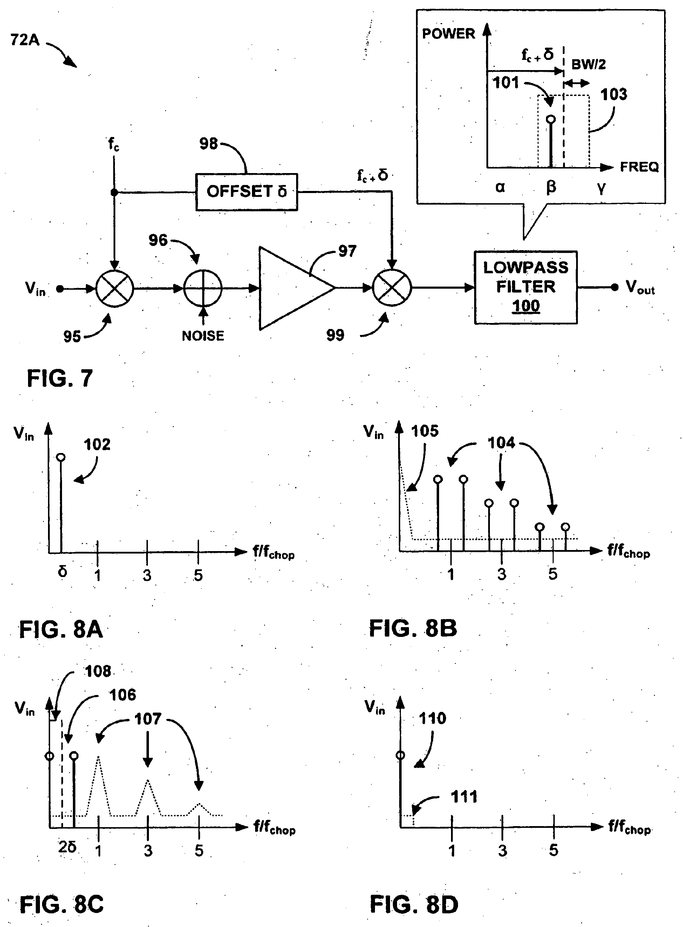

FIG. 7 is a block diagram illustrating a portion of an exemplary chopper-stabilized superheterodyne instrumentation amplifier 72A for use within frequency selective signal monitor 70 from FIG. 6A. Superheterodyne instrumentation amplifier 72A illustrated in FIG. 7 may operate substantially similar to superheterodyne instrumentation amplifier 72 from FIG. 6A. Superheterodyne instrumentation amplifier 72A includes a first modulator 95, an amplifier 97, a frequency offset 98, a second modulator 99, and a lowpass filter 100. In some embodiments, lowpass filter 100 may be an integrator, such as integrator 89 of FIG. 6A. Adder 96 represents addition of noise to the chopped signal. However, adder 96 does not form an actual component of superheterodyne instrumentation amplifier 72A. Adder 96 models the noise that comes into superheterodyne instrumentation amplifier 72A from non-ideal transistor characteristics.

Superheterodyne instrumentation amplifier 72A receives a physiological signal ($V_{in}$) associated with a patient from sensing elements, such as electrodes, positioned within or external to the patient to detect the physiological signal. First modulator 95 modulates the signal from baseband at the carrier frequency ($f_c$). A noise signal is added to the modulated signal, as represented by adder 96. Amplifier 97 amplifies the noisy modulated signal. Frequency offset 98 is tuned such that the carrier frequency plus or minus frequency offset 98 ($f_c \pm \delta$) is equal to the selected frequency band. Hence, the offset $\delta$ may be selected to target a desired frequency band. Second modulator 99 modulates the noisy amplified signal at offset frequency 98 from the carrier frequency $f_c$. In this way, the amplified signal in the selected frequency band is demodulated directly to baseband and the noise signal is modulated to the selected frequency band.

Lowpass filter 100 may filter the majority of the modulated noise signal out of the demodulated signal and set the effective bandwidth of its passband around the center frequency of the selected frequency band. As illustrated in the detail associated with lowpass filter 100 in FIG. 7, a passband 103 of lowpass filter 100 may be positioned at a center frequency of the selected frequency band. In some cases, the offset $\delta$ may be equal to this center frequency. Lowpass filter 100 may then set the effective bandwidth (BW/2) of the passband around the center frequency such that the passband encompasses the entire selected frequency band. In this way, lowpass filter 100 passes a signal 101 positioned anywhere within the selected frequency band.

For example, if the selected frequency band is 5 to 15 Hz, for example, the offset $\delta$ may be the center frequency of this band, i.e., 10 Hz, and the effective bandwidth may be half the full bandwidth of the selected frequency band, i.e., 5 Hz. In this case, lowpass filter 100 rejects or at least attenuates signals above 5 Hz, thereby limiting the passband signal to the alpha band, which is centered at 0 Hz as a result of the superheterodyne process. Hence, the center frequency of the selected frequency band can be specified with the offset $\delta$, and the bandwidth BW of the passband can be obtained independently with the lowpass filter 100, with BW/2 about each side of the center frequency.

Lowpass filter 100 then outputs a low-noise physiological signal ($V_{out}$). The low-noise physiological signal may then be input to signal analysis unit 73 from FIG. 6A. As described above, signal analysis unit 73 may extract the signal in the selected frequency band positioned at baseband, measure power of the extracted signal, and compare the measured power to a baseline power threshold of the selected frequency band to determine whether to trigger patient therapy.

A superheterodyning, chopper-stabilized amplifier, as described in this disclosure, may be used to extract bandpower measurements at key physiological frequencies, with an architecture that is flexible, robust and low-noise. The amplifier merges heterodyning and chopper stabilization for flexible bandpass selection. The addition of a relative clock shift $\delta$ selects the center of the band, while a Lowpass filter sets the bandpass width. A chopper stabilized amplifiers may provide wide dynamic range, high-Q filter. Chopper stabilization is a noise/power efficient architecture for amplifying low-frequency neural signals in micropower applications with excellent process immunity.

By displacing the clocks within the chopper amplifier to translate the frequency of the signal, the amplifier can readily tune to particular frequency bands. For example,, the up-modulator can set to one frequency, $F_{clk}$. At the input to the mixer amplifier, the signal is then centered about the $F_{clk}$ modulation frequency, well above excess aggressor noise. Demodulation may be performed with a second clock of frequency $F_{clk2} = F_{clk} + \delta$. The net deconvolution of the signal and the demodulation clock re-centers the signal to dc and $2\delta$ at the output of the demodulator.

Since biomarkers may be encoded as low frequency fluctuations of the spectral power, a low pass filter can be used to filter out the $2\delta$ component. For example, the filter may be realized by an on-chip, two-pole, lowpass filter with a bandwidth defined as BW/2, where BW represents the bandwidth of the target frequency band. Signals on either side of $\delta$ are aliased into the net pass-band at $V_{OUT}$. The heterodyning chopper-stabilized amplifier may suppress harmonics as the square of the harmonic order, to yield a net output at signal $V_{out}$ that may be represented by the following equation:

$$Vout(f) = \frac{4}{\pi^2} \sum_{n,odd} \frac{1}{n^2} \cdot V_{in}(f \pm \delta \cdot n)\cos(\phi) \qquad (2)$$

where $n$ denotes the harmonic order, $f$ represents frequency, $\delta$ represents the delta offset applied to the modulation clock frequency, and $\phi$ is the phase between the $\delta$ clock and the physiological signal input. The heterodyned chopper-stabilized amplifier extracts a band equivalent to a second-order bandpass filter with a scale factor of $4/\pi^2$. The center frequency can be set by a programmable clock difference, which is simple to synthesize on-chip, while the bandwidth (and Q) can be set independently by a programmable lowpass filter. In some embodiments, the programmable lowpass filter may have a quasi-Gaussian profile.

FIGS. 8A-8D are graphs illustrating the frequency components of a signal at various stages within superheterodyne instrumentation amplifier 72A of FIG. 7. In particular, FIG. 8A illustrates the frequency components in a selected frequency band within the physiological signal received by frequency selective signal monitor 70. The frequency components of the physiological signal are represented by line 102 and located at offset $\delta$ from baseband in FIG. 8A.

FIG. 8B illustrates the frequency components of the noisy modulated signal produced by modulator 95 and amplifier 97. In FIG. 8B, the original offset frequency components of the physiological signal have been up-modulated at carrier frequency $f_c$ and are represented by lines 104 at the odd harmonics. The frequency components of the noise signal added to the modulated signal are represented by dotted line 105. In FIG. 8B, the energy of the frequency components of the noise signal is located substantially at baseband and energy of the frequency components of the desired signal is located at the carrier frequency ($f_c$) plus and minus frequency offset ($\delta$) 98 and its odd harmonics.

FIG. 8C illustrates the frequency components of the demodulated signal produced by demodulator 99. In particular, the frequency components of the demodulated signal are located at baseband and at twice the frequency offset ($2\delta$), represented by lines 106. The frequency components of the noise signal are modulated and represented by dotted line 107. The frequency components of the noise signal are located at the carrier frequency plus or minus the offset frequency ($\delta$) 98 and its odd harmonics in FIG. 8C. FIG. 8C also illustrates the effect of lowpass filter 100 that may be applied to the demodulated signal. The passband of lowpass filter 100 is represented by dashed line 108.

FIG. 8D is a graph that illustrates the frequency components of the output signal. In FIG. 8D, the frequency components of the output signal are represented by line 110 and the frequency components of the noise signal are represented by dotted line 111. FIG. 8D illustrates that lowpass filter 100 removes the frequency components of the demodulated signal located at twice the offset frequency ($2\delta$). In this way, lowpass filter 100 positions the frequency components of the signal at the desired frequency band within the physiological signal at baseband. In addition, lowpass filter 100 removes the frequency components from the noise signal that were located outside of the passband of lowpass filter 100 shown in FIG. 8C. The energy from the noise signal is substantially eliminated from the output signal, or at least substantially reduced relative to the original noise signal that otherwise would be introduced.

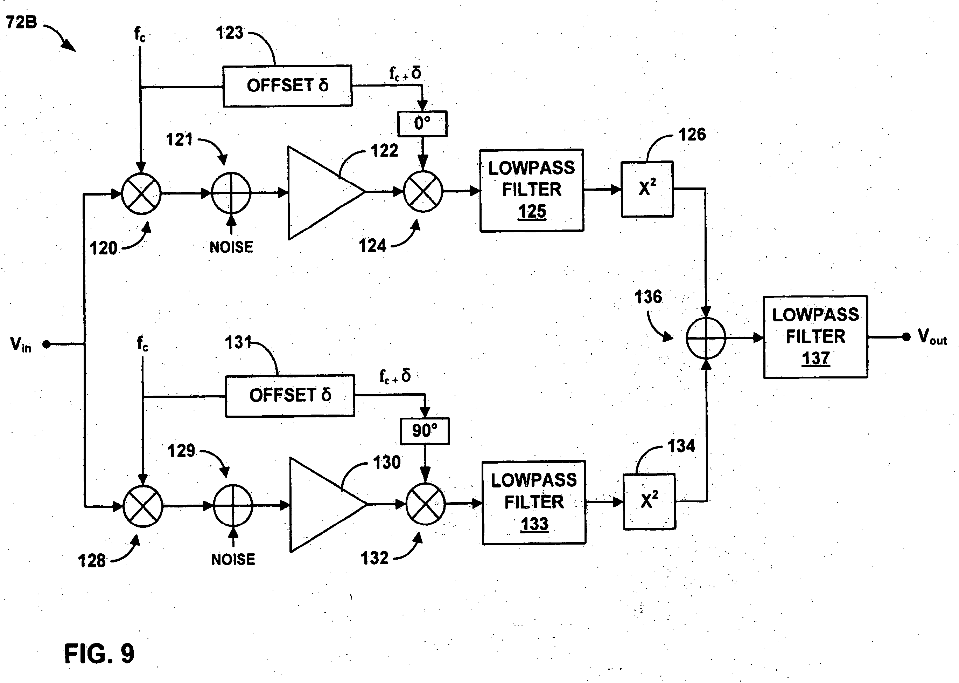

FIG. 9 is a block diagram illustrating a portion of an exemplary chopper-stabilized superheterodyne instrumentation amplifier 72B with in-phase (I) and quadrature (Q) signal paths for use within frequency selective signal monitor 70 from FIG. 6A. The in-phase and quadrature signal paths substantially reduce phase sensitivity within superheterodyne instrumentation amplifier 72B. Because the signals obtained from the patient and the clocks used to produce the modulation frequencies are uncorrelated, the phases of these signals may not be synchronized. To address the phasing issue, two parallel heterodyning amplifiers may be driven with in-phase (I) and quadrature (Q) clocks created with on-chip distribution circuits. Net power extraction then can be achieved with superposition of the in-phase and quadrature signals. Superposition can be achieved using on-chip self-cascoded Gilbert mixers to calculate the sum of the squares and superimposing currents. In some embodiments, the use of heterodyning techniques and chopper stabilization may provide low noise signal extraction with robust filtering that may be relatively immune from process, temperature and/or mismatch variations.

An analog implementation may use an on-chip self-cascoded Gilbert mixer to calculate the sum of squares, as mentioned above. Alternatively, a digital approach may take advantage of the low bandwidth of the I and Q channels after lowpass filtering, and digitize at that point in the signal chain for digital power computation. Digital computation at the I/Q stage has advantages. For example, power extraction is more linear than a tanh function. In addition, digital computation simplifies offset calibration to suppress distortion, and preserves the phase information for cross-channel coherence analysis. With either technique, a sum of squares in the two channels can eliminate the phase sensitivity between the physiological signal and the modulation clock frequency. The power output signal can lowpass filtered to the order of 1 Hz to track the essential dynamics of a desired biomarker.

Superheterodyne instrumentation amplifier 72B illustrated in FIG. 9 may operate substantially similar to super-heterodyne instrumentation amplifier 72 from FIG. 6A. Superheterodyne instrumentation amplifier 72B includes an in-phase (I) signal path with a first modulator 120, an amplifier 122, an in-phase frequency offset $(\delta)$ 123, a second modulator 124, a Lowpass filter 125, and a squaring unit 126. Adder 121 represents addition of noise. Adder 121 models the noise from non-ideal transistor characteristics. Superheterodyne instrumentation amplifier 72B includes a quadrature phase (Q) signal path with a third modulator 128, an adder 129, an amplifier 130, a quadrature frequency offset $(\delta)$ 131, a fourth modulator 132, a lowpass filter 133, and a squaring unit 134. Adder 129 represents addition of noise. Adder 129 models the noise from non-ideal transistor characteristics. Up-modulators 120 and 128, down-modulators 124 and 132, and amplifiers 122 and 130 may form a heterodyning circuit configured to convert a selected frequency band of the physiological signal to a baseband according to this disclosure.

Superheterodyne instrumentation amplifier 72B receives a physiological signal $(V_{in})$ associated with a patient from one or more sensing elements. The in-phase (I) signal path modulates the signal from baseband at the carrier frequency $(f_c)$, permits addition of a noise signal to the modulated signal, and amplifies the noisy modulated signal. In-phase frequency offset 123 may be tuned such that it is substantially equivalent to a center frequency of a selected frequency band. For the alpha band (5 to 15Hz), for example, the offset 123 may be approximately 10 Hz. In this example, if the modulation carrier frequency $f_c$ applied by modulator 120 is 4000 Hz, then the demodulation frequency $f_c \pm \delta$ may be 3990 Hz or 4010 Hz.

Second modulator 124 modulates the noisy amplified signal at a frequency $(f_c \pm \delta)$ offset from the carrier frequency $f_c$ by the offset amount $\delta$. In this way, then amplified signal in the selected frequency band may be demodulated directly to baseband and the noise signal may be modulated up to the second frequency $f_c \pm \delta$. The selected frequency band of the physiological signal is then substantially centered at baseband, e.g., DC. For the alpha band (5 to 15 Hz), for example, the center frequency of 10 Hz is centered at 0 Hz at baseband. Lowpass filter 125 filters the majority of the modulated noise signal out of the demodulated signal and outputs a low-noise physiological signal. The low-noise physiological signal may then be squared with squaring unit 126 and input to adder 136. In some cases, squaring unit 126 may comprise a self-cascoded Gilbert mixer. The output of squaring unit 126 represents the spectral power of the in-phase signal.

In a similar fashion, the quadrature (Q) signal path modulates the signal from baseband at the carrier frequency $(f_c)$. The Q signal path permits addition of a noise signal to the modulated signal, as represented by adder 129, and amplifies the noisy modulated signal via amplifier 130. Again, quadrature offset frequency $(\delta)$ 131 may be tuned such that it is approximately equal to the center frequency of the selected frequency band. As a result, the demodulation frequency applied to demodulator 132 is $(f_c \pm \delta)$. In the quadrature signal path, however, an additional phase shift of 90 degrees is added to the demodulation frequency for demodulator 132. Hence, the demodulation frequency for demodulator 132, like demodulator 124, is $f_c \pm \delta$. However, the demodulation frequency for demodulator 132 is phase shifted by 90 degrees relative to the demodulation frequency for demodulator 124 of the in-phase signal path.

Fourth modulator 132 modulates the noisy amplified signal at the quadrature frequency 131 from the carrier frequency. In this way, the amplified signal in the selected frequency band is demodulated directly to baseband and the noise signal is modulated at the demodulation frequency $f_c \pm \delta$. Lowpass filter 133 filters the majority of the modulated noise signal out of the demodulated signal and outputs a low-noise physiological signal. The low-noise physiological signal may then be squared and input to adder 136. Like squaring unit 126, squaring unit 134 may comprise a self-cascoded Gilbert mixer. The output of squaring unit 134 represents the spectral power of the quadrature signal.

Adder 136 combines the signals output from squaring unit 126 in the in-phase signal path and squaring unit 134 in the quadrature signal path. The output of adder 136 may be input to a lowpass filter 137 that generates a low-noise, phase-insensitive output signal $(V_{out})$. In one example embodiment, lowpass filter 127 may be programmable and configured to achieve a net power output between approximately 1 and 10 Hz, and to achieve a net gain on the order of $1V/V^2$ with a nominal input signal of 10mV into the block, with a 120nA total bias.

As described above, the signal may be input to signal analysis unit 73 from FIG. 6A, and signal analysis unit 73 may extract the signal in the selected frequency band positioned at baseband, measure power of the extracted signal, and compare the measured power to a baseline power threshold of the selected frequency band to determine whether to trigger patient therapy. Alternatively, signal analysis unit 73 may analyze other characteristics of the signal. The signal Vout may be applied to the signal analysis unit 73 as an analog signal. Alternatively, an analog-to-digital converter (ADC) may be provided to convert the signal Vout to a digital signal for application to signal analysis unit 73. Hence, signal analysis unit 73 may include one or more analog components, one or more digital components, or a combination of analog and digital components.

The spectral density of a signal may derived from the conjugate product of the Fourier transform which includes a windowing function 'w(t)' that reflects the bandwidth BW of interest according to the following equation:

$$\phi(f) = \frac{X(f)^* X(f)}{2\pi},$$

$$\text{where } X(f) = \int_{-\infty}^{\infty} x(t)w(t)e^{-j2\pi ft}dt. \tag{3}$$

Expanding out the spectral power φ(f) using Euler's identity demonstrates that the net energy can be measured by the superposition of two orthogonal signal sources representing an 'in-phase' and 'quadrature' signal. The expanded spectral power φ(f) is given according to the following equation:

$$\phi(f) = \left| \int_{-\infty}^{\infty} x(t)w(t)[\cos(2\pi ft)]\,dt \right|^2$$

$$+ \left| \int_{-\infty}^{\infty} x(t)w(t)[\sin(2\pi ft)]\,dt \right|^2 \tag{4}$$

Both terms are considered since the phase relationship between the neural circuit and the interface IC are not correlated.

An analog signal chain for flexible spectral analysis can be designed according to Equation (4). In addition to achieving significant amplification of the signals, the input neural signal may be multiplied by a sine and cosine term at the bandcenter, δ, and then windowed or otherwise set the effective BW. The resulting signals may be squared and then added together with a final lowpass filter prior to digitization. A modified chopper amplifier may assist in performing the linear multiplication of the neural signal and the tone at δ in order to achieve both robust amplification and spectral extraction that is both highly flexible and robust to process variations.

The nonlinear properties of a chopper amplifier can be exploited for spectral analysis. Chopper stabilization can provide a noise- and power-efficient architecture for amplifying low-frequency neural signals in micropower biomedical applications. Moreover, chopper stabilized amplifiers can be adapted to provide wide dynamic range, high-Q filters.

As demonstrated by Equation (4), the net spectral power is extracted by superimposing an in-phase and quadrature channel. Since the physiological signal and the integrated circuit (IC) clocks are uncorrelated, a phase offset may occur between the signals. The superposition of the in-phase and quadrature channels in superheterodyne chopper amplifier 72B of FIG. 9 may assist in accounting for this phase offset. The net power extraction at $V_{out}$ achieved by the superposition of the squared in-phase and quadrature signals may be represented by the following equation:

$$V_{EEG\_Power}(f) \propto [\frac{4}{\pi^2} \cdot \sum_{n,odd} \frac{1}{n^2} \cdot V_{in}(f + \delta \cdot n)]^2 \tag{5}$$

where n represents the harmonic order, f represents the carrier frequency, δ represents the frequency offset value, and $\phi$ represents the phase between the demodulator clock and the physiological signal input. Since the signal power falls off with a 1/f law, the net power of the physiological signals at the third harmonic are effectively attenuated so that acceptable selectivity can be maintained with respect to the key band of interest. In some embodiments, a notched clock strategy may be used to drive the heterodyning choppers in order to suppress higher-order harmonic content. This can allow for even greater harmonic suppression.

To achieve low power, an analog implementation may use an on-chip self-cascoded Gilbert mixer to calculate the sum of squares by superimposing currents. To prevent residual offsets in the tanh circuits from creating modulation products in the I and Q channels, the inputs to the Gilbert multipliers may be chopped with a 64Hz square wave. The power output signal can be lowpass filtered to the order of 1 Hz to track the essential dynamics of the biomarker, easing resource requirements in the digital processing blocks.

In addition to bandpower extraction, a heterodyning chopper-stabilized amplifier may have several uses when the clock difference, ($\delta$), is set to zero. One application is to measure a standard time-domain neural signal without preprocessing, which can be useful for prescreening waveforms to identify spectral biomarkers of interest and to confirm algorithm functionality. Another application is to measure impedance with the addition of current stimulation injected across electrodes at the chopper clock frequency, and fixing the state of the front-end modulators, as will be described. Tapping the signal output of the in-phase channel then provides the real component of the impedance, while the output of the quadrature port is the complex impedance. This measurement can be useful for characterizing electrodes and tissue properties as well as properties of the electrode/tissue interface.

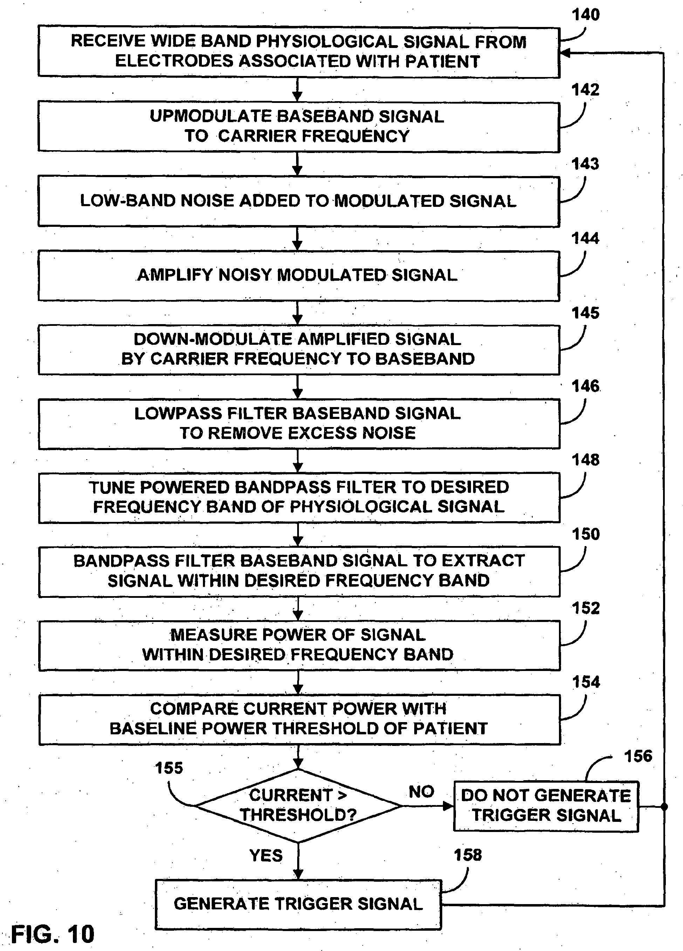

FIG. 10 is a flowchart that illustrates an exemplary operation of a frequency selective signal monitor that includes a chopper-stabilized instrumentation amplifier. The operation of FIG. 10 will be described herein in reference to frequency selective signal monitor 30 that includes chopper-stabilized instrumentation amplifier 32 from FIG. 3.

Frequency selective signal monitor 30 receives a physiological signal associated with a patient (140). First modulator 42 modulates the physiological signal from baseband at the carrier frequency (142). Adder 45 represents the addition of a low-band noise signal with the modulated signal (143). Amplifier 46 amplifies the noisy modulated signal (144). Second modulator 47 then demodulates the signal at the carrier frequency to position the input physiological signal at baseband (145). Integrator 48 applies a lowpass filter to the demodulated signal to remove excess noise from the demodulated signal (146). Instrumentation amplifier 32 then outputs a low-noise physiological signal to signal analysis unit 33.

Powered bandpass filter 34 within signal analysis unit 33 may be tuned to a selected frequency band (148). In some cases, powered bandpass filter 34 may be manually-tuned to the selected frequency band by a physician, technician, or the patient. In other cases, the powered bandpass filter 34 may by dynamically tuned to the selected frequency band in accordance with stored frequency band values. Powered bandpass filter 34 is applied to the low-power physiological signal output from instrumentation amplifier 32 to extract the signal in the selected frequency band from the wide band physiological signal (150). Power measurement module 36 measures the power of the extracted signal (152).

The measured power is then filtered by lowpass filter 37 and applied to comparator 40. Threshold tracker 38 tracks fluctuations in power measurements of the selected frequency band for the patient over a period of time. In this way, threshold tracker 38 generates a baseline power threshold of the selected frequency band for the patient based on the fluctuations. Comparator 40 compares the measured power to the baseline power threshold of the selected frequency band for the patient (154). If the measured power is greater than the baseline power threshold (YES branch of 155), comparator 40 outputs a trigger signal (158) to a processor of a medical device. If the measured power is less than the baseline power threshold (NO branch of 155), the comparator 40 outputs a power tracking measurement to threshold tracker 38 to generate the baseline power threshold and does not generate the trigger signal (156). In either case, after comparator 40 determines whether to generate the trigger signal, frequency selective signal monitor 30 continues to monitor the wide band physiological signal associated with the patient (140).

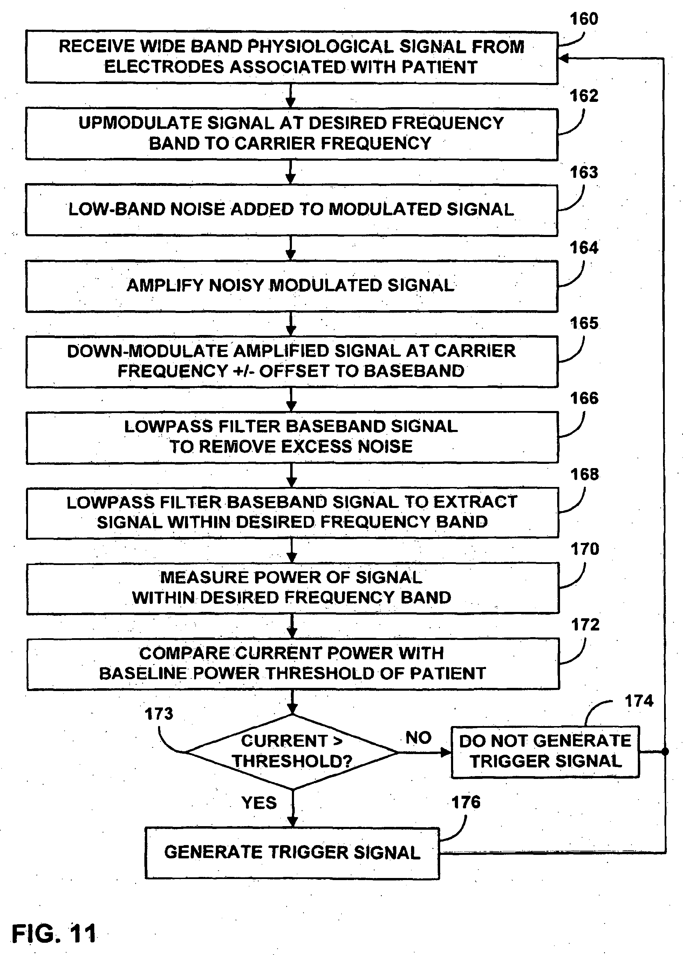

FIG. 11 is a flowchart that illustrates an exemplary operation of a frequency selective signal monitor that includes a chopper-stabilized superheterodyne instrumentation amplifier. The operation of FIG. 11 will be described herein in reference to frequency selective signal monitor 70 that includes chopper-stabilized superheterodyne instrumentation amplifier 72 from FIG. 6A.

Frequency selective signal monitor 70 receives a physiological signal associated with a patient (160). Modulator 82 modulates the physiological signal from baseband at the carrier frequency (162). Adder 85 represents addition of a low-band noise signal with the modulated signal (163). Amplifier 86 amplifies the noisy modulated signal (164). Frequency offset 87 is tuned such that it substantially corresponds to a center frequency of the selected frequency band. Demodulator 88 then demodulates the signal in directly to baseband at the carrier frequency plus or minus the frequency offset (166). Integrator 89 applies a lowpass filter to the demodulated signal to remove excess noise from the demodulated signal (167). Superheterodyne instrumentation amplifier 72 then outputs a low-noise physiological signal to signal analysis unit 73.

Passive lowpass filter 74 within signal analysis unit 73 is applied to the low-noise physiological signal from superheterodyne instrumentation amplifier 72 to extract the signal in the selected frequency band positioned at baseband from the wide band physiological signal (168). Power measurement module 76 measures power of the extracted signal (170). The measured power is then filtered by lowpass filter 77 and applied to comparator 80. Threshold tracker 78 tracks fluctuations in power measurements of the selected frequency band for the patient over a period of time. Again, in this way, threshold tracker 78 may generate a baseline power threshold of the selected frequency band for the patient based on the fluctuations.

Comparator 80 compares the current power to the baseline power threshold in order to identify a need for patient therapy (172). If the current power is greater than the baseline power threshold (YES branch of 173), comparator 80 generates a trigger signal (176). If the current power is less than the baseline power threshold (NO branch of 173), the comparator 80 does not generate a trigger signal (174). In either case, after comparator 80 determines whether patient therapy has been triggered, frequency selective signal monitor 70 continues to monitor the wide band physiological signal associated with the patient (160).

The techniques described herein for monitoring a physiological signal in a selected frequency band without rapid signal sampling may provide several advantages. For example, the techniques may provide a fast signal monitoring solution with low power, computing and memory overhead. Therefore, the techniques may be implemented within medical devices with small form-factors and limited power, computing and memory capabilities, such as implantable medical devices. Furthermore, the techniques may provide a solution that is highly configurable and allows a user, such as a physician, technician, or patient, to select the frequency band in which to monitor the physiological signal for symptoms or conditions of the patient.

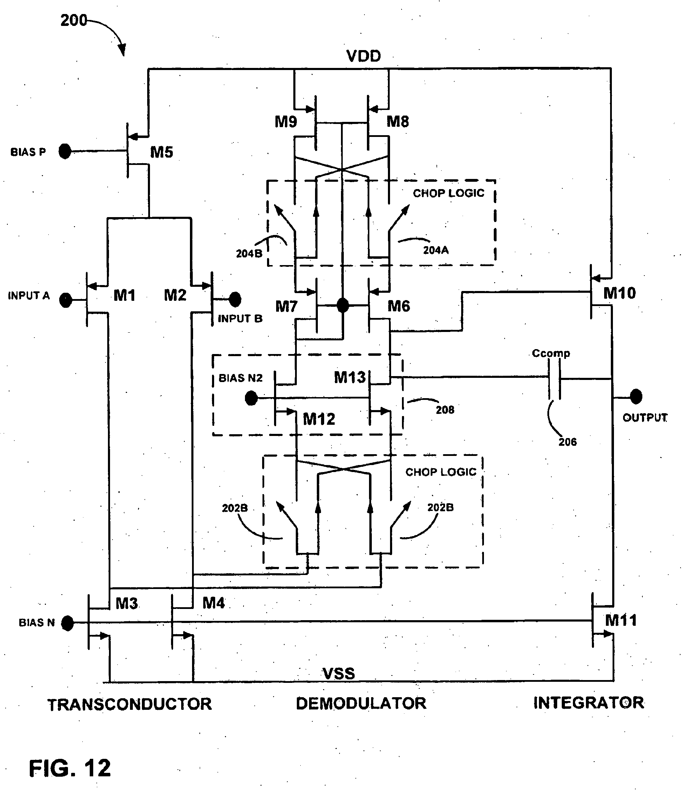

FIG. 12 is a circuit diagram illustrating an example mixer amplifier circuit 200 for use in instrumentation amplifier 32 of FIG. 3 or superheterodyne instrumentation amplifier 72 of FIG. 6A. For example, circuit 200 represents an example of amplifier 46, demodulator 47 and integrator 48 in FIG. 3 or amplifier 86, demodulator 88 and integrator 89 in FIG. 6A. Although the example of FIG. 12 illustrates a differential input, circuit 200 may be constructed with a single-ended input. Accordingly, circuit 200 of FIG. 12 is provided for purposes of illustration, without limitation as to other embodiments. In FIG. 12, VDD and VSS indicate power and ground potentials, respectively.

Mixer amplifier circuit 200 amplifies a noisy modulated input signal to produce an amplified signal and demodulates the amplified signal. Mixer amplifier circuit 200 also substantially eliminates noise from the demodulated signal to generate the output signal. In the example of FIG. 12, mixer amplifier circuit 200 is a modified folded-cascode amplifier with switching at low impedance nodes. The modified folded-cascode architecture allows currents to be partitioned to maximize noise efficiency. In general, the folded cascode architecture is modified in FIG. 12 by adding two sets of switches. One set of switches is illustrated in FIG. 12 as switches 202A and 202B (collectively referred to as "switches 202") and the other set of switches includes switches 204A and 204B (collectively referred to as "switches 204").

Switches 202 are driven by chop logic to support the chopping of the amplified signal for demodulation at the chop frequency. In particular, switches 202 demodulate the amplified signal and modulate front-end offsets and 1/f noise. Switches 204 are embedded within a self-biased cascode mirror formed by transistors M6, M7, M8 and M9, and are driven by chop logic to up-modulate the low frequency errors from transistors M8 and M9. Low frequency errors in transistors M6 and M7 are attenuated by source degeneration from transistors M8 and M9. The output of mixer amplifier circuit 200 is at baseband, allowing an integrator formed by transistor M10 and capacitor 206 (Ccomp) to stabilize a feedback path (not shown in FIG. 12) between the output and input and filter modulated offsets.

In the example of FIG. 12, mixer amplifier circuit 200 has three main blocks: a transconductor, a demodulator, and an integrator. The core is similar to a folded cascode. In the transconductor section, transistor M5 is a current source for the differential pair of input transistors M1 and M2. In some embodiments, transistor M5 may pass approximately 800 nA, which is split between transistors M1 and M2, e.g., 400 nA each. Transistors M1 and M2 are the inputs to amplifier 14. Small voltage differences steer differential current into the drains of transistors M1 and M2 in a typical differential pair way. Transistors M3 and M4 serve as low side current sinks, and may each sink roughly 500nA, which is a fixed, generally nonvarying current. Transistors M1, M2, M3, M4 and M5 together form a differential transconductor.

In this example, approximately 100 nA of current is pulled through each leg of the demodulator section. The AC current at the chop frequency from transistors M1 and M2 also flows through the legs of the demodulator. Switches 202 alternate the current back and forth between the legs of the demodulator to demodulate the measurement signal back to baseband, while the offsets from the transconductor are up-modulated to the chopper frequency. As discussed previously, transistors M6, M7, M8 and M9 form a self-biased cascode mirror, and make the signal single-ended before passing into the output integrator formed by transistor M10 and capacitor 206 (Ccomp). Switches 204 placed within the cascode (M6-M9) upmodulate the low frequency errors from transistors M8 and M9, while the low frequency errors of transistor M6 and transistor M7 are suppressed by the source degeneration they see from transistors M8 and M9. Source degeneration also keeps errors from Bias N2 transistors 208 suppressed. Bias N2 transistors M12 and M13 form a common gate amplifier that presents a low impedance to the chopper switching and passes the signal current to transistors M6 and M7 with immunity to the voltage on the drains.

The output DC signal current and the upmodulated error current pass to the integrator, which is formed by transistor M10, capacitor 206, and the bottom NFET current source transistor M11. Again, this integrator serves to both stabilize the feedback path and filter out the upmodulated error sources. The bias for transistor M10 may be approximately 100nA, and is scaled compared to transistor M8. The bias for lowside NFET M11 may also be approximately 100nA (sink). As a result, the integrator is balanced with no signal. If more current drive is desired, current in the integration tail can be increased appropriately using standard integrate circuit design techniques. The transistors in the example of FIG. 12 may be field effect transistors (FETs), and more particularly complementary metal-oxide semiconductor (CMOS) transistors.

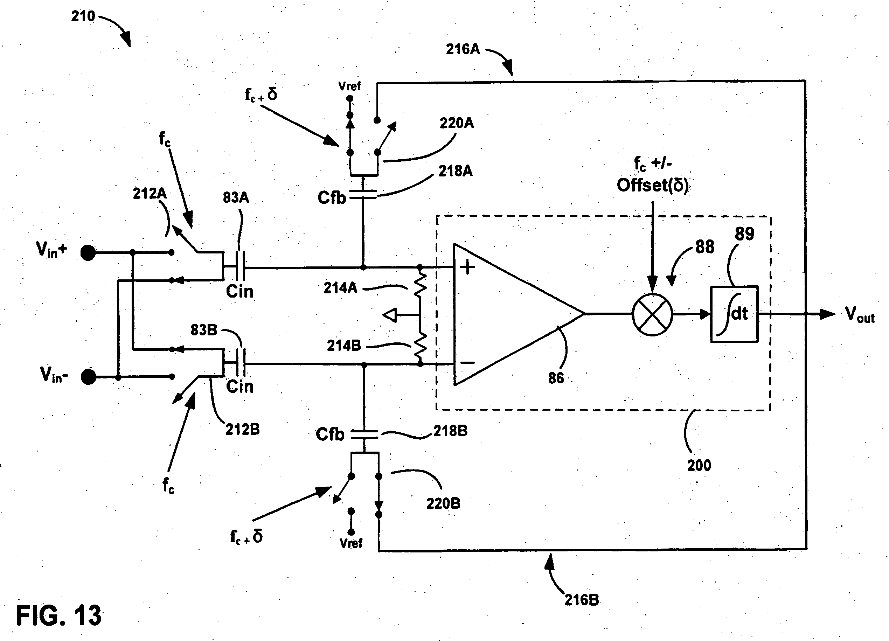

FIG. 13 is a circuit diagram illustrating an instrumentation amplifier 210 with differential inputs $V_{in}+$ and $V_{in}-$. Instrumentation amplifier 210 is an example embodiment of superheterodyne instrumentation amplifier 72 previously described in this disclosure with reference to FIG. 6A. FIG. 13 uses several reference numerals from FIG. 6A to refer to like components. In general, instrumentation amplifier 210 may be constructed as a single-ended or differential amplifier. The example of FIG. 13 illustrates example circuitry for implementing a differential amplifier. Circuitry similar to the circuitry of FIG. 13 also could be used to implement a differential version of instrumentation amplifier 32 of FIG. 3. The circuitry of FIG. 13 may be configured for use in each of the I and Q signal paths of FIG. 9.

In the example of FIG. 13, instrumentation amplifier 210 includes an interface to one or more sensing elements that produce a differential input signal providing voltage signals $V_{in}+$, $V_{in}-$. The differential input signal may be provided by a sensor comprising any of a variety of sensing elements, such as a set of one or more electrodes, an accelerometer, a pressure sensor, a force sensor, a gyroscope, a humidity sensor, a chemical sensor, or the like. For brain sensing, the differential signal $V_{in}+$, $V_{in}-$ may be, for example, an EEG or ECoG signal.

The differential input voltage signals are connected to respective capacitors 83A and 83B (collectively referred to as "capacitors 83") through switches 212A and 212B, respectively. Switches 212A and 212B may collectively form modulator 82 of FIG. 6A. Switches 212A, 212B are driven by a clock signal provided by a system clock (not shown) at the carrier frequency $f_c$. Switches 212A, 212B may be cross-coupled to each other, as shown in FIG. 13, to reject common-mode signals. Capacitors 83 are coupled at one end to a corresponding one of switches 212A, 212B and to a corresponding input of amplifier 86 at the other end. In particular, capacitor 83A is coupled to the positive input of amplifier 86, and capacitor 83B is coupled to the negative input of amplifier 86, providing a differential input. Amplifier 86, modulator 88 and integrator 89 together may form a mixer amplifier, which may be constructed similar to mixer amplifier 200 of FIG. 12.

In FIG. 13, switches 212A, 212B and capacitors 83A, 83B form a front end of instrumentation amplifier 210. In particular, the front end may operate as a continuous time switched capacitor network. Switches 212A, 212B toggle between an open state and a closed state in which inputs signals $V_{in}+$, $V_{in}-$ are coupled to capacitors 83A, 83B at a clock frequency $f_c$ to modulate (chop) the input signal to the carrier (clock) frequency. As mentioned previously, the input signal may be a low frequency signal within a range of approximately 0 Hz to approximately 1000 Hz and, more particularly, approximately 0 Hz to 500 Hz, and still more particularly less than or equal to approximately 100 Hz. The carrier frequency may be within a range of approximately 4 kHz to approximately 10 kHz. Hence, the low frequency signal is chopped to the higher chop frequency band.

Switches 212A, 212B toggle in-phase with one another to provide a differential input signal to amplifier 86. During one phase of the clock signal $f_c$, switch 212A connects Vin+ to capacitor 83A and switch 212B connects Vin- to capacitor 83B. During another phase, switches 212A, 212B change state such that switch 212A decouples Vin+ from capacitor 83A and switch 212B decouples Vin-from capacitor 83B. Switches 212A, 212B synchronously alternate between the first and second phases to modulate the differential voltage at the carrier frequency. The resulting chopped differential signal is applied across capacitors 83A, 83B, which couple the differential signal across the positive and negative inputs of amplifier 86.

Resistors 214A and 214B (collectively referred to as "resistors 214") may be included to provide a DC conduction path that controls the voltage bias at the input of amplifier 86. In other words, resistors 214 may be selected to provide an equivalent resistance that is used to keep the bias impedance high. Resistors 214 may, for example, be selected to provide a 5 GΩ equivalent resistor, but the absolute size of the equivalent resistor is not critical to the performance of instrumentation amplifier 210. In general, increasing the impedance improves the noise performance and rejection of harmonics, but extends the recovery time from an overload. To provide a frame of reference, a 5 GΩ equivalent resistor results in a referred-to-input (RTI) noise of approximately 20 nV/rt Hz with an input capacitance (Cin) of approximately 25 pF. In light of this, a stronger motivation for keeping the impedance high is the rejection of high frequency harmonics which can alias into the signal chain due to settling at the input nodes of amplifier 86 during each half of a clock cycle.

Resistors 214 are merely exemplary and serve to illustrate one of many different biasing schemes for controlling the signal input to amplifier 86. In fact, the biasing scheme is flexible because the absolute value of the resulting equivalent resistance is not critical. In general, the time constant of resistor 214 and input capacitor 83 may be selected to be approximately 100 times longer than the reciprocal of the chopping frequency.

Amplifier 86 may produce noise and offset in the differential signal applied to its inputs. For this reason, the differential input signal is chopped via switches 212A, 212B and capacitors 83A, 83B to place the signal of interest in a different frequency band from the noise and offset. Then, instrumentation amplifier 210 chops the amplified signal at modulator 88 a second time to demodulate the signal of interest down to baseband while modulating the noise and offset up to the chop frequency band. In this manner, instrumentation amplifier 210 maintains substantial separation between the noise and offset and the signal of interest.

Modulator 88 may support direct downconversion of the selected frequency band using a superheterodyne process. In particular, modulator 88 may demodulate the output of amplifier 86 at a frequency equal to the carrier frequency $f_c$ used by switches 212A, 212B plus or minus an offset $\delta$ that is substantially equal to the center frequency of the selected frequency band. In other words, modulator 88 demodulates the amplified signal at a frequency of $f_c \pm \delta$. Integrator 89 may be provided to integrate the output of modulator 88 to produce output signal Vout. Amplifier 86 and differential feedback path branches 216A, 216B process the noisy modulated input signal to achieve a stable measurement of the low frequency input signal output while operating at low power.

Operating at low power tends to limit the bandwidth of amplifier 86 and creates distortion (ripple) in the output signal. Amplifier 86, modulator 88, integrator 89 and feedback paths 216A, 216B may substantially eliminate dynamic limitations of chopper stabilization through a combination of chopping at low-impedance nodes and AC feedback, respectively.

In FIG. 13, amplifier 86, modulator 88 and integrator 89 are represented with appropriate circuit symbols in the interest of simplicity. However, it should be understood that such components may be implemented in accordance with the circuit diagram of mixer amplifier circuit 200 provided in FIG. 12. Instrumentation amplifier 210 may provide synchronous demodulation with respect to the input signal and substantially eliminate 1/f noise, popcorn noise, and offset from the signal to output a signal that is an amplified representation of the differential voltage Vin+, Vin-.

Without the negative feedback provided by feedback path 216A, 216B, the output of amplifier 86, modulator 88 and integrator 89 could include spikes superimposed on the desired signal because of the limited bandwidth of the amplifier at low power. However, the negative feedback provided by feedback path 216A, 216B suppresses these spikes so that the output of instrumentation amplifier 210 in steady state is an amplified representation of the differential voltage produced across the inputs of amplifier 86 with very little noise.

Feedback paths 216A, 216B, as shown in FIG. 13, include two feedback path branches that provide a differential-to-single ended interface. Amplifier 86, modulator 88 and integrator 89 may be referred to collectively as a mixer amplifier. The top feedback path branch 216A modulates the output of this mixer amplifier to provide negative feedback to the positive input terminal of amplifier 86. The top feedback path branch 216A includes capacitor 218A and switch 220A. Similarly, the bottom feedback path branch 216B includes capacitor 218B and switch 220B that modulate the output of the mixer amplifier to provide negative feedback to the negative input terminal of the mixer amplifier. Capacitors 218A, 218B are connected at one end to switches 220A, 220B, respectively, and at the other end to the positive and negative input terminals of the mixer amplifier, respectively. Capacitors 218A, 218B may correspond to capacitor 91 in FIG. 6A. Likewise, switches 220A, 220B may correspond to modulator 90 of FIG. 6A.

Switches 220A and 220B toggle between a reference voltage (Vref) and the output of the mixer amplifier 200 to place a charge on capacitors 218A and 218B, respectively. The reference voltage may be, for example, a mid-rail voltage between a maximum rail voltage of amplifier 86 and ground. For example, if the amplifier circuit is powered with a source of 0 to 2 volts, then the mid-rail Vref voltage may be on the order of 1 volt. Switches 220A and 220B should be 180 degrees out of phase with each other to ensure that a negative feedback path exists during each half of the clock cycle. One of switches 220A, 220B should also be synchronized with the mixer amplifier 200 so that the negative feedback suppresses the amplitude of the input signal to the mixer amplifier to keep the signal change small in steady state. Hence, a first one of the switches 220A, 220B may modulate at a frequency of $f_c \pm \delta$, while a second switch 220A, 220B modulates at a frequency of $f_c \pm \delta$, but 180 degrees out of phase with the first switch. By keeping the signal change small and switching at low impedance nodes of the mixer amplifier, e.g., as shown in the circuit diagram of FIG. 12, the only significant voltage transitions occur at switching nodes. Consequently, glitching (ripples) is substantially eliminated or reduced at the output of the mixer amplifier.

Switches 212 and 220, as well as the switches at low impedance nodes of the mixer amplifier, may be CMOS SPDT switches. CMOS switches provide fast switching dynamics that enables switching to be viewed as a continuous process. The transfer function of instrumentation amplifier 210 may be defined by the transfer function provided in equation (6) below, where Vout is the voltage of the output of mixer amplifier 200, Cin is the capacitance of input capacitors 83, $\Delta V$ in is the differential voltage at the inputs to amplifier 86, Cfb is the capacitance of feedback capacitors 218A, 218B, and Vref is the reference voltage that switches 220A, 220B mix with the output of mixer amplifier 200.

$$Vout = Cin(\Delta Vin)/Cfb + Vref \qquad (6)$$

From equation (6), it is clear that the gain of instrumentation amplifier 210 is set by the ratio of input capacitors Cin and feedback capacitors Cfb, i.e., capacitors 83 and capacitors 218. The ratio of Cin/Cfb may be selected to be on the order of 100. Capacitors 218 may be poly-poly, on-chip capacitors or other types of MOS capacitors and should be well matched, i.e., symmetrical.

Although not shown in FIG. 13, instrumentation amplifier 210 may include shunt feedback paths for auto-zeroing amplifier 210. The shunt feedback paths may be used to quickly reset amplifier 210. An emergency recharge switch also may be provided to shunt the biasing node to help reset the amplifier quickly. The function of input capacitors 83 is to up-modulate the low-frequency differential voltage and reject common-mode signals. As discussed above, to achieve up-modulation, the differential inputs are connected to sensing capacitors 83A, 83B through SPDT switches 212A, 212B, respectively. The phasing of the switches provides for a differential input to the ac transconductance mixing amplifier 116. These switches 212A, 212B operate at the clock frequency, e.g., 4 kHz. Because capacitors 83A, 83B toggle between the two inputs, the differential voltage is up-modulated to the carrier frequency while the low-frequency common-mode signals are suppressed by a zero in the charge transfer function. The rejection of higher-bandwidth common signals relies on this differential architecture and good matching of the capacitors.

Blanking circuitry may be provided in some embodiments for applications in which measurements are taken in conjunction with stimulation pulses delivered by a cardiac pacemaker, cardiac defibrillator, or neurostimulator. Such blanking circuitry may be added between the inputs of amplifier 86 and coupling capacitors 83A, 83B to ensure that the input signal settles before reconnecting amplifier 86 to the input signal. For example, the blanking circuitry may be a blanking multiplexer (MUX) that selectively couples and de-couples amplifier 86 from the input signal. This blanking circuitry may selectively decouple the amplifier 86 from the differential input signal and selectively disable the first and second modulators, i.e., switches 212, 220, e.g., during delivery of a stimulation pulse.

A blanking MUX is optional but may be desirable. The clocks driving switches 212, 220 to function as modulators cannot be simply shut off because the residual offset voltage on the mixer amplifier would saturate the amplifier in a few milliseconds. For this reason, a blanking MUX may be provided to decouple amplifier 86 from the input signal for a specified period of time during and following application of a stimulation by a cardiac pacemaker or defibrillator, or by a neurostimulator.

To achieve suitable blanking, the input and feedback switches 212, 220 should be disabled while the mixer amplifier continues to demodulate the input signal. This holds the state of integrator 89 within the mixer amplifier because the modulated signal is not present at the inputs of the integrator, while the demodulator continues to chop the DC offsets. Accordingly, a blanking MUX may further include circuitry or be associated with circuitry configured to selectively disable switches 212, 220 during a blanking interval. Post blanking, the mixer amplifier may require additional time to resettle because some perturbations may remain. Thus, the total blanking time includes time for demodulating the input signal while the input switches 212, 220 are disabled and time for settling of any remaining perturbations. An example blanking time following application of a stimulation pulse may be approximately 8 ms with 5 ms for the mixer amplifier and 3 ms for the AC coupling components.

Examples of various additional chopper amplifier circuits that may be adapted for use with techniques, circuits and devices of this disclosure are described in U.S. Patent No. 7,385,443, issued June 10, 2008, to Timothy J. Denison, entitled "Chopper Stabilized Instrumentation Amplifier."

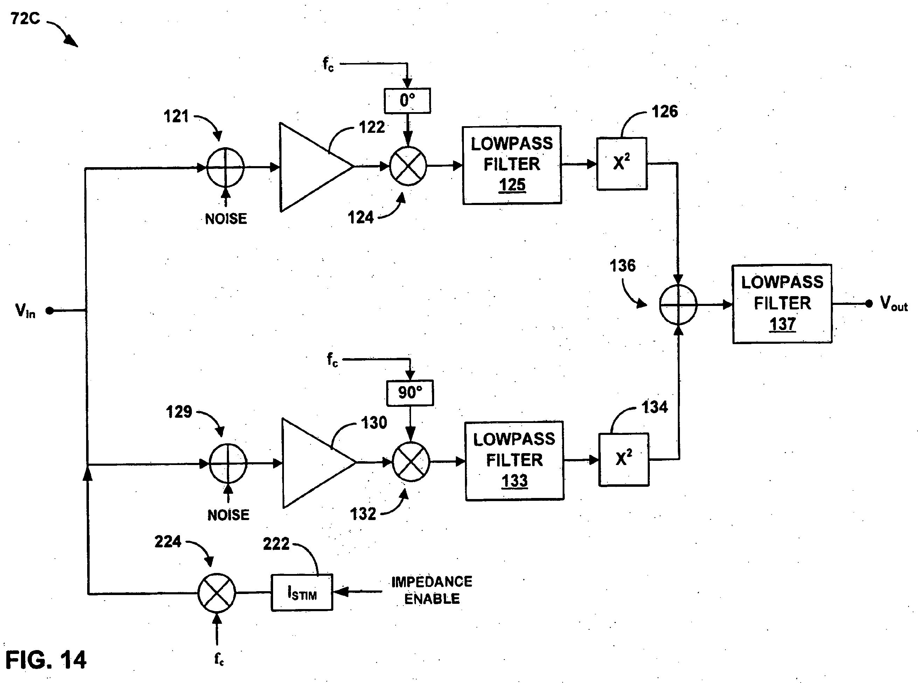

FIG. 14 is a block diagram illustrating a portion of an exemplary chopper-stabilized superheterodyne amplifier 72C with in-phase and quadrature signal paths, as shown in FIG. 9, with the addition of optional impedance measurement circuitry. Amplifier 72C of FIG. 14 is substantially identical to amplifier 72B of FIG. 9. As shown in the example of FIG. 14, however, the superheterodyne architecture may be adapted to measure complex electrode and tissue impedance by supplying a stimulation current across the inputs of the instrumentation amplifier and disabling the input chopper modulation. To that end, a current source 222 delivers a current $I_{stim}$ modulated at the carrier frequency $f_c$ in response to an impedance measurement enable signal. Thus, up-modulator 224, down-modulators 124 and 132, and amplifiers 122 and 130 may form a heterodyning circuit configured to convert a selected frequency band of the physiological signal to a baseband according to this disclosure.

Current source 222 applies the current $I_{stim}$ to modulator 224, which modulates the current $I_{stim}$ at the carrier frequency $f_c$ The current $I_{stim}$ is then applied across the inputs to the I and Q signal paths of amplifier 72C. To support impedance measurement, the operation of the front-end modulators (not shown in FIG. 14; 120, 128 in FIG. 9) is temporarily stopped by fixing the states of the modulators in response to the impedance measurement enable signal. The stimulation current may be on the order of 10 microamps (uA), and may be injected across a set of input electrodes at the carrier frequency $f_c$. The output of the in-phase signal path provides the real component of the impendance, while the output of the quadrature port is the complex impedance of the impedance. The real and complex components can be squared, then summed, and lowpass filtered to produce an output impedance signal.

A chopper-stabilized superheterodyne amplifier circuit, as described in this disclosure, may be analyzed in terms of a performance figure of merit. For a chopper-stabilized amplifier with a powered bandpass filter, rather than a superheterodyne structure, if W = center frequency, BW = bandwidth, and A = gain, then a gain-bandwidth product of A*(W+BW/2) is needed to realize such a system. With a chopper-stabilized superheterodyne amplifier circuit, only A*(BW/2) is needed because the band of interest is selected. However, to compensate for the scaling of the signal by $4/\pi^2$, to maintain signal to noise ratio (SNR), it may be necessary to scale current by that square, and also add a factor of two for in-phase and quadrature channels. Consequently, a metric of $[(\pi^2/4)^2]*A*BW/2$ is needed. This metric indicates when the chopper-stabilized superheterodyne amplifier circuit provides desirable efficiency. In general, the chopper-stabilized superheterodyne amplifier circuit may be particularly useful when $(W + BW/2)/BW \sim Q > (\pi)^4/16 \sim 6$. If the

applicable Q is greater than 6, then the chopper-stabilized superheterodyne amplifier is both easy to implement and the most efficient approach. In some embodiments, a heterodyning chopper amplifier in accordance with this disclosure may operate at power levels on the order of 8 microwatts while permitting direction extraction of 2 microvolt-RMS brain biomarker signals.

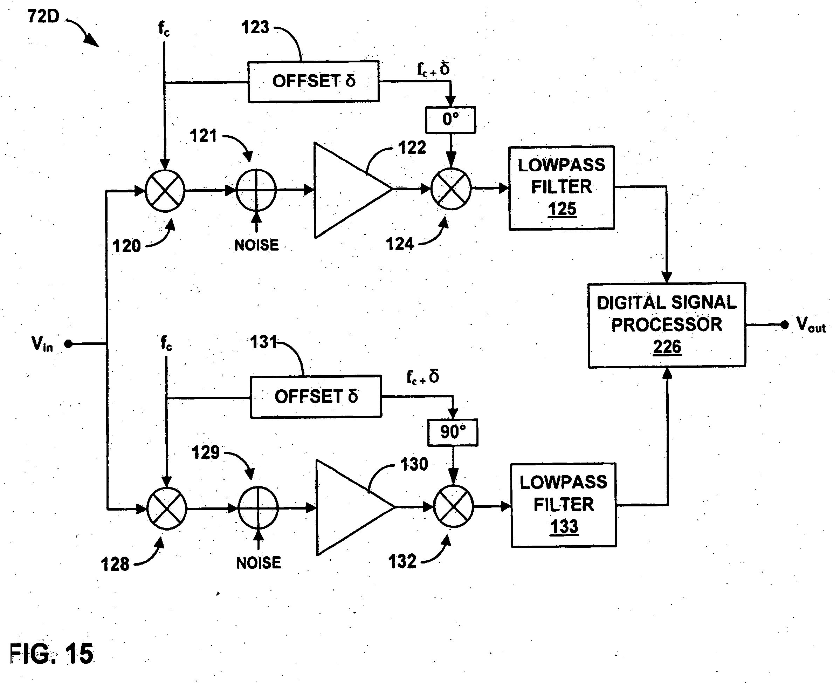

FIG. 15 is a block diagram illustrating a portion of an exemplary chopper-stabilized superheterodyne amplifier 72D with in-phase and quadrature signal. paths, as shown in FIG. 9, with the addition of a digital signal processor 226. Amplifier 72D of FIG. 15 is substantially identical to amplifier 72B of FIG. 9. As shown in the example of FIG. 15, however, the superheterodyne architecture may be adapted to digitally perform the squaring, summing, and filtering functions of the in-phase and quadrature signal paths.

In-phase lowpass filter 125 delivers the in-phase signal to digital signal processor 226 and quadrature lowpass filter 133 delivers the quadrature signal to digital signal processor 226. Digital signal processor 226 includes or is coupled to an analog-to-digital converter (ADC) to convert the in-phase signal and the quadrature signal to digital signals for processing. Digital signal processor 226 squares the digital in-phase signal and the digital quadrature signal. Digital signal processor 226 then sums the squared digital signals together and filters the summed digital signal to generate a low-noise, phase-insensitive digital output signal ($V_{out}$).

As described above, the signal may be input to signal analysis unit 73 from FIG. 6A. As described above, signal analysis unit 73 may extract the signal in the selected frequency band positioned at baseband, measure power of the extracted signal, and compare the measured power to a baseline power threshold of the selected frequency band to determine whether to trigger patient therapy. Alternatively, signal analysis unit 73 may analyze other characteristics of the signal. In the embodiment illustrated in FIG. 9, the signal $V_{out}$ may be applied to the signal analysis unit 73 as a digital signal. Hence, signal analysis unit 73 may include one or more digital components.

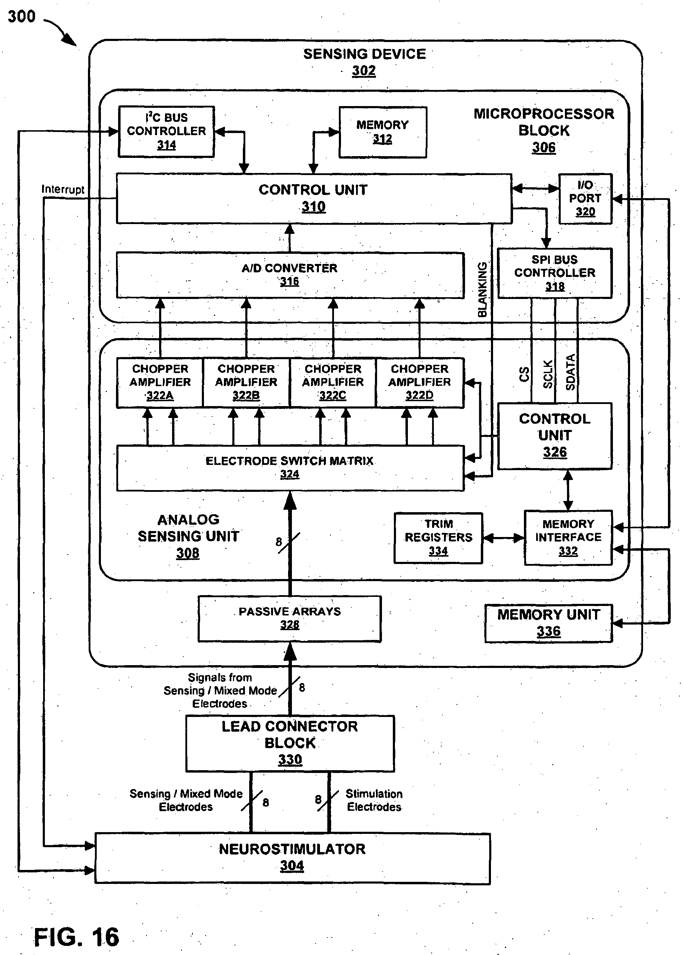

FIG. 16 is a block diagram illustrating an example sensing device 302 integrated with a neurostimulator 304 to form a combined stimulation and sensing system 300. The sensing device 302 may generally include a frequency selective monitoring device with one or more heterodyning, chopper-stabilized amplifiers, in accordance with any of various embodiments described in this disclosure. The stimulation and sensing system 300 may be implantable and may be constructed to reside within a common housing. The architecture of the system 300 may be partitioned to provide a balance between low-power operation, accuracy and flexibility. Sensing device 302 may include an analog sensing unit 308 that connects to the electrodes for conditioning and amplifying field potentials, a microprocessor 306 or equivalent processing unit for performing algorithms on the signal based on feature extraction, and a memory unit 336 for recording events or general data-logging.

Connections between the sensing device 302 and a controller of the stimulator 304 can be made through an interrupt vector and inter-integrated circuit (I2C) bus port. The partitioning of the signal chain between analog and digital blocks may focus on designing a robust analog front-end to extract the core information of interest and thereby maximize information content prior to digitization. This partitioning may allow the digitizer and signal processing algorithms to be run in the microprocessor block at low clock rates and with a reduced power requirement. In one embodiment, microprocessor block 306 may be configured to utilize less than one percent of the available processor resources and to keep system power below approximately 25 $\mu$W.

In some embodiments, sensing device 302 may provide added diagnostic and closed-loop titration capabilities to an existing neurostimulator or other therapy device. In such cases, sensing device 302 may send commands to neurostimulator 304 for titration of therapy parameter values associated with the therapy device based on an algorithm. Connections between the sensing extension and the electrodes may be made through a protection network that isolates the sensing device from stimulation and blocks DC currents.

In the example of FIG. 16, sensing device 302 includes a microprocessor block 306 having a microprocessor control unit 310, an analog-to-digital (A/D) converter 316, a serial peripheral interface (SPI) bus controller 318, an input/output port 320, internal memory 312, and an I2C bus controller 314. Sensing device 302 may be formed as one or more integrated circuits (ICs). Sensing device 302 also includes an analog sensing unit 308, which may include an electrode switch matrix 324, a control unit 326, one or more heterodyning, chopper-stabilized chopper amplifiers 322A-D arranged as separate sensing channels, a memory interface 332, and trim registers 334.

Sensing device 302 also may include external memory 336 and one or more passive arrays 328. The passive arrays 328 may form a passive protection network between the sense electrodes in lead connector block 330 and the analog sensing unit IC 308. Each chopper amplifier channel 322A-D may be configured to receive a signal from a respective electrode via connector block 330 and extract signal power in a defined frequency band. The analog sensing unit IC 308 may increase the information content and lower the bandwidth prior to digitization by A/D converter 316 within microprocessor block 306. By operating at low rates, the microprocessor block 306 may be able to digitize, track events, and write to memory while maintaining microwatt power operation.

As an illustration, FIG. 16 shows eight different sensing/mixed mode electrodes and eight different stimulation electrodes coupled to a lead connector block 330 of the combined stimulation and sensing system 300. The electrodes may be carried at a distal end of one or more implantable leads. For example, electrical contacts corresponding to the distal electrodes may be formed at a proximal end of the lead or leads, and coupled to the electrodes via conductors that extend along the length of the leads. The electrical contacts may be coupled to the output of a stimulation generator of the neurostimulator 304 and to the passive arrays 328 of sensing device 302. In some embodiments, some of the electrodes may deliver electrical stimulation energy from the neurostimulator 304, while other electrodes may deliver electrical stimulation energy and serve as sensing electrodes for the analog sensing unit 308.

As further shown in the example of FIG. 16, electrode switch matrix 324 may be configured to switch eight electrode inputs onto eight inputs associated with four chopper amplifier channels 322A-D. For example, a first pair of sense electrodes may form a first input for chopper amplifier 322A, and a second pair of sense electrodes may form a second input for chopper amplifier 322B. The first and second pairs of electrodes may share one or more electrodes, or alternatively, may include mutually exclusive pairs of electrodes. The electrode switch matrix 324 may selectively switch different pairs of electrodes across the input of different chopper amplifier channels 322. Control unit 326 may control the operation of electrode switch matrix 324 and the chopper amplifiers 322A-D. Control unit 326 may be coupled to SPI bus controller 318 of microprocessor block 306 via conversion start (CS), serial clock (SCLK), and serial data (SDATA) lines.

Microprocessor block 306 may exchange information with analog sensing unit 308 via memory interface 332 and I/O port 320. Trim registers 334 may be provided for calibration or adjustment of various aspects of analog sensing unit 308. External memory 336 may store sensed data, and exchange data with memory interface 332 of analog sensing unit 308. A/D converter 316 of microprocessor block 306 receives the outputs of the chopper amplifier channels 322A-D and converts the analog output signals to digital values for processing and analysis by the microprocessor control unit 310. Chopper amplifiers 322A-D allow analog sensing unit 308 to extract energy from a specified band defined by the band-center, $\delta$, with a bandwidth about $\delta$ defined by BW. The chopper amplifiers 322A-D may represent any of the chopper amplifiers discussed in this disclosure, including amplifiers 32, 32A, 72, 72A, 72B, 72C, 72D, and 210 shown in FIGS. 3, 4, 6A, 7, 9 and 13-15. In addition, chopper amplifiers 322A-D may include a signal analysis unit as discussed in this disclosure. Example signal analysis units include units are illustrated in FIGS. 3, 6A, 6B, 9, 14, and 15. Although four chopper amplifier channels 322A-D are shown in FIG. 16, fewer or greater numbers of chopper amplifier channels may be provided.

Microprocessor block 306 may contain a digital control interface that enables microprocessor control of amplifier channels 322A-D through memory mapped registers. Parameters such as gain and trim states can be adjusted through the control interface. In addition, an interface may be provided to a 1 MB loop recording SRAM. The digital control interface may reduce the number of control lines needed by the microprocessor. In addition, the digital control interface may also provide a sample clock for A/D converter 316, which may allow control unit 310 to enter a low power sleep mode between samples and thereby cause the duty cycle to be reduced for digitization and algorithm processing. In some embodiments, the duty cycle may be reduced to as low as 1%.

The various chopper amplifier channels 322A-D may be provided to sense signals via different electrode pairs or to sense signals in different frequency bands. Control unit 326 of analog sensing unit 308 may adjust the clock offsets of the chopper amplifiers 322A-D to cause the chopper amplifiers to extract band power from different frequency bands on a selective basis. In some embodiments, microprocessor block 306, analog sensing unit 308 or both may be programmable so that the selected frequency bands monitored by the chopper amplifier channels 322A-D can be adjusted.

The spectral processors 322A-D and the electrode coupling circuit may be interfaced through an input switch matrix 324 that allows for flexible selection of the electrode vector for measurement after electrode placement. The configuration of each of the spectral processors and the switch montage may be held in an on-chip register bank and EEPROM memory, which is accessed through microprocessor block 306. The output of the analog spectral processors 322A-D may be fed into the analog to digital converter 316 of microprocessor block 306 for digitization. Power supplies may be provided from the existing neurostimulator 304.

Microprocessor control unit 310 may generate a blanking signal to decouple the sense electrodes from the chopper amplifier channels 322A-D via the electrode switch matrix 324 when a stimulation pulse or waveform is applied by the neurostimulator 304. Microprocessor control unit 310 may communicate with the neurostimulator 304 via an interrupt and the I$^2$C bus to coordinate operation of the sensing device 302 and the neurostimulator 304. Although a neurostimulator304 is shown in FIG. 16, a general purpose electrical stimulator for any of a variety of applications such as nerve, tissue or muscle stimulation may be provided.

A differential clock generator may generate the system clocks necessary to drive the heterodyning chopper amplifiers. The clock generator may comprise a clock tree such that the four channels share a common 4kHz "$F_{clk}$" driver for the front-end modulators. The common clock may help prevent beating at the tissue interface. Each sensing channel may also have a dedicated local clock to create the $F_{clk} + \delta$ reference for the back-end of the amplifiers 322A-D. The clocks may be trimmed to 2 times their nominal value and then downsampled to provide the quadrature drivers necessary for the parallel branches of the spectral processor. The clock itself may be constructed from a relaxation oscillator. In one embodiment, a current budget of 200nA/channel may be allocated for the clock in order to minimize the impact on system power.

In one example, the clock frequency may be adjustable with capacitive trims to achieve 4 Hz step sizes from DC to 500 Hz. The trims may be accessible through a register port, and the microprocessor block may routinely calibrate the clocks comparing periods with the crystal oscillator embedded with the existing neurostimulator to minimize drift.

As shown in FIG. 16, analog sensing unit 308 may extract band power measurements at key physiological frequencies by using chopper stabilization to achieve a noise & power efficient architecture for amplifying low-frequency physiological signals in micropower applications. The extracted band power measurements from analog sensing unit 308 may then be processed using microprocessor block 306, so that algorithms can be customized by making firmware changes. By the time the signals from the analog sensing unit 308 arrive at microprocessor block 306, the biomarkers of interest have already had their bandpower measurement extracted by analog sensing unit 308. Since these signals change very slowly compared to the frequencies that encode the biomarkers, microprocessor block 308 may sample and process the signals at low rates, such as rates of 5 Hz or lower in some embodiments. This technique of using analog sensing unit 308 as an analog preprocessor and of running additional algorithms at slower rates on a low power microprocessor, such as microprocessor block 306, can result in a sensing device that runs on a power budget that is on the order of a magnitude lower than that of the stimulation therapy.

In some embodiments, analog to digital converter (ADC) 316 may sample and store raw EEG (time-domain) data at a higher rate (such as 200 Hz) along with the 5 Hz band power data. Such an embodiment may allow for additional post processing and analysis of the data and may be useful for algorithm validation or to identify new biomarkers.

When measuring neuronal activity, the band power fluctuations in the local field potentials (LFPs) are generally orders of magnitude slower than the frequency at which they are encoded, so the use of efficient analog preprocessing before performing analog to digital conversion can greatly reduce the overall energy requirements for implementing a complete mixed-signal system. A preprocessing device, that directly extracts energy in key neuronal bands and tracks the relatively slow power fluctuations, such as analog sensing unit 308 in FIG. 16, may be considered an example of such an architecture that reduces overall energy requirements.

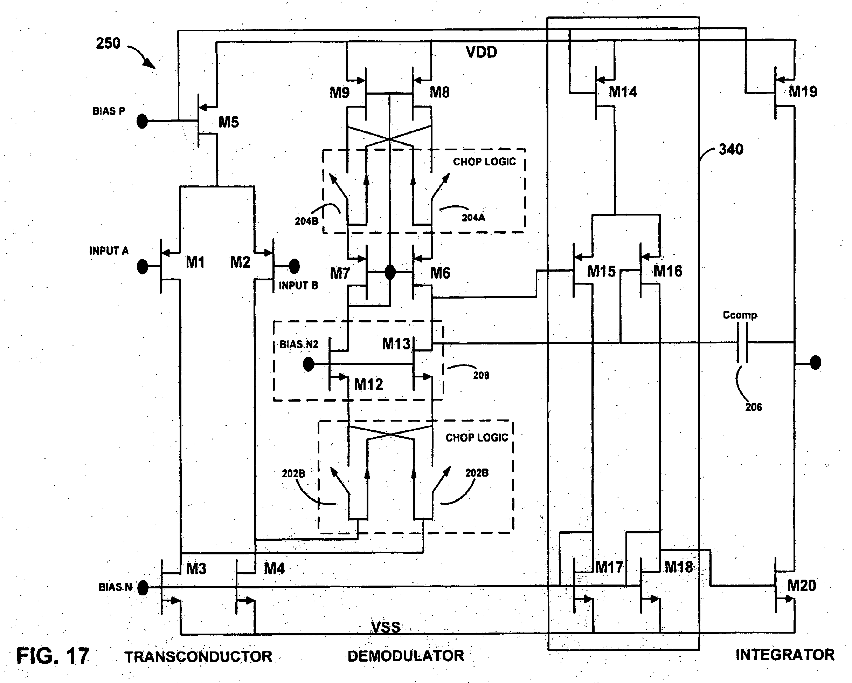

FIG. 17 is another circuit diagram illustrating a chopper-stabilized mixer amplifier 250 suitable for use within the frequency selective signal monitor of FIG. 3 or FIG. 6A. Chopper-stabilized mixer amplifier 250 is similar to the mixer amplifier shown in FIG. 12, but with the addition of transistors 340 and a modified output stage. Components that are substantially similar to components shown in FIG. 12 are numbered alike. Transistors 340 are arranged to form a second stage differential amplifier that improves the power supply rejection ratio (PSRR) of the circuit by tracking both nodes of a top-side, self-cascoded PFET, current mirror. The second stage differential amplifier 340 may be used to help reject extraneous signals on the power supply that can arise from external sources such as delivery of electrical stimulation.

The various transistors in the example of FIG. 17 may be sized for acceptable matching at the second stage differential amplifier 340 and appropriate biasing per general design techniques. As an example, transistors M1 and M2 may have sizing ratios of 100/4; transistors M3 and M4 may have sizing ratios of 200/10; transistors M6 and M7 may have sizing ratios of 40/2; transistors M8 and M9 may have sizing ratios of 5/80; transistors M12 and M13 may have sizing ratios of 20/4; transistors M 14 may have a sizing ratio of 20/10; transistors M15, M16, M 17 and M18 may have sizing ratios of 10/10; and transistors M19 and M20 may have sizing ratios of 120/10. The ratios described above are width/length ratios. The compensation capacitor may have a value of 16 picofarads in one example. In addition, different amounts of current may be allocated to different legs of the circuit. In one example, the leg of the circuit containing transistor M5 may be allocated 640 nanoamps of current; the legs of the circuit containing transistors M3 and M4 may each be allocated 400 nanoamps of current; and the leg containing transistor M20 may be allocated 120 nanoamps of current. The transistors in the mixer amplifier 250 may be field effect transistors (FETs), and more particularly complementary metal-oxide semiconductor (CMOS) transistors.

As shown in FIG. 17, mixer amplifier 250 may include a differential input to the second stage to robustly bias the output stage integrator. One benefit of the current-mode switch architecture in FIG 17 is that the transients from chopper modulation are orders of magnitude faster than the chopper clock period. This separation of dynamics helps to suppress the second harmonic distortion and amplification errors. Since the output of the transconductance stage is at baseband, the integrator can both compensate the feedback loop and filter upmodulated offsets and noise. As an additional advantage arising from heterodyning, the band of interest is also shifted to DC, so the remaining signal chain circuitry can run at reduced bandwidth to minimize power.

The folded-cascode design allows currents to be partitioned in order to improve noise performance. In one example, 300 nA of current may be allocated to flow through each input pair, 50 nA of current may be allocated to flow through each leg of the folded cascade, 50 nA of current may be allocated for the output stage, and 50 nA of current may be allocated for bias generation and distribution. Such a partitioning directs the majority of current into the input pair to maximize transconductance compared to other field-effect transistors (FETs) in the amplifier, and biases the transistors at sub-threshold levels. In one embodiment, the biasing N-channel FETs (NFETs), may be scaled relatively large to suppress the noise contribution from the NFETs and thereby further suppress effective 1/f noise. In addition, an additional 500 k$\Omega$ of source degeneration may be used to lower the effective transconductance of the biasing NFETs relative to the input pair.

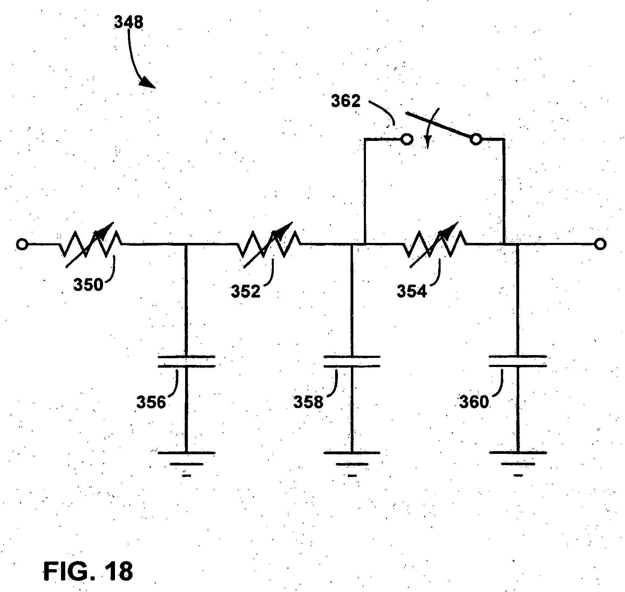

FIG. 18 is a circuit diagram illustrating a low pass filter 348 suitable for use within a frequency selective signal monitor including a heterodyning, chopper amplifier as described in this disclosure. For example, the low pass filter 348 may be used as an output low pass filter from a heterodyning chopper amplifier, e.g., such as low pass filter 58, 74 or 100 in FIGS. 4, 6A, 6B or 7. As shown in FIG. 18, the low pass filter 348 may include an input, coupled to a first variable resistor 350, that receives an output signal, e.g., from the demodulator of the mixer amplifier. The first resistor 350 is coupled in series to a second variable resistor 352 and a third variable resistor 354. The values of the variable resistors may be set by trim registers. The node between the first and second resistors 350, 352 is coupled to ground via a capacitor 356. The node between the second and third resistors 352, 354 is also coupled to ground via a capacitor 358. The node at the output of the filter 348 at one end of the third resistor 354 is coupled to ground via a capacitor 360. A switch 362 coupled in parallel across the third resistor 354 can be closed to remove the third resistor and selectively provide either a two pole or three pole mode for the low pass filter 348. In one example, the variable resistors 350, 352, 354 may be 10-35 mega ohm variable resistors, and the capacitors 356, 358, 360 may be 100 Pico farad capacitors. In general, the low pass filter 348 of FIG. 18 may provide a programmable on-chip filter that selectively provides either two or three poles. In one example, low pass filter 348 of FIG. 18 may be programmed to have low pass -3 dB corner (BW/2) of 4.5 Hz to 15 Hz (3 pole) or 10 Hz to 25 Hz (2 pole), and the trim step size may be set to 4 Hz increments (2 pole) or 2 Hz increments (3 pole).

The low-pass filter 348 may be constructed as a passive circuit with high resistance CrSi materials and poly-poly capacitors. The low-pass filter 348 may mimic a quasi-Gaussian response. As shown in FIG. 18, the signal chain may be a staggered chain of RC filters. Such a signal chain may increase linearity and headroom for the filter while reducing the power dissipation. The trimming may be achieved with field-effect transistor (FET) switches to shunt elements of the CrSi resistor string. In some embodiments, the time constant scaling may be trimmed for a quasi-Gaussian profile to attempt to reduce the time-frequency duality limits of the Fourier transform.

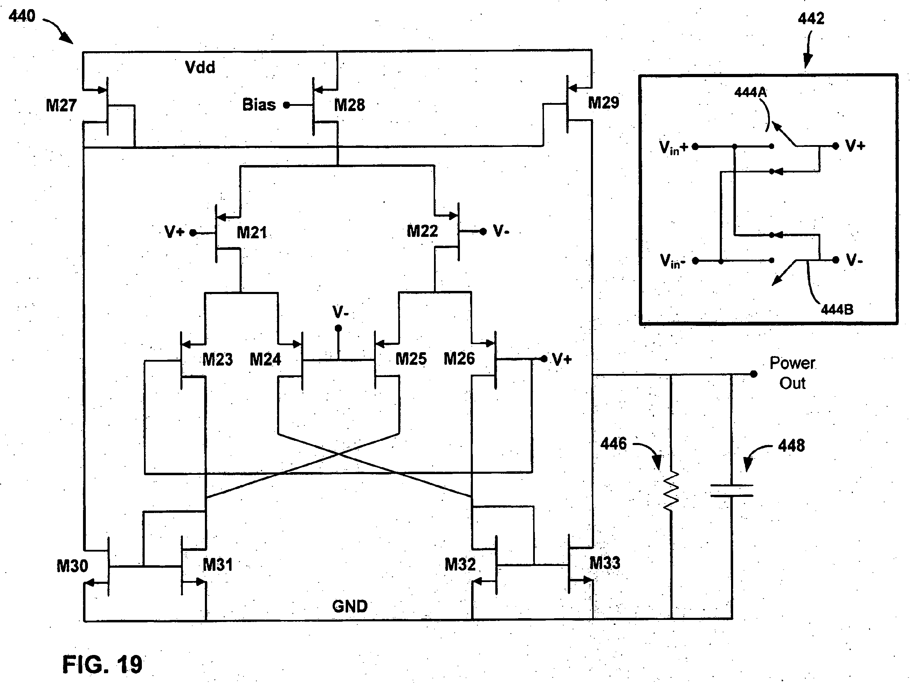

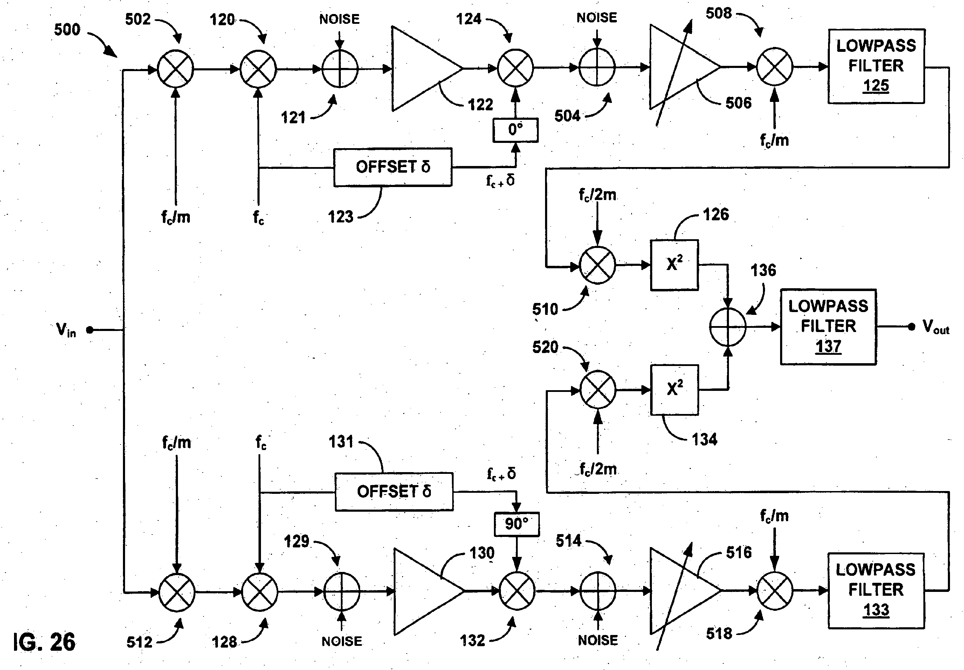

FIG. 19 is a circuit diagram illustrating an example output power block 440 to extract power from the output signal of a chopper-stabilized, superheterodyne instrumentation amplifier. For example, the output power block 440 may be used within a power extraction module of a signal analysis unit, e.g., such as power extraction modules 36, 76, 76A or 76B in FIGS. 3, 6A or 6B. As another example, the output power block 440 may be used within a squaring unit of a superheterodyning instrumentation amplifier, e.g., such as squaring units 126 or 134, in FIGS. 9, 14, or 26. The circuit of FIG. 19 may be constructed as a selfbiased cascode/Gilbert multiplier to extract net power from the signal chain. As shown in FIG. 19, transistors M21, M22, M23, M24, M25 and M26 may form the Gilbert multiplier. Transistors M27, M29, M30, M31, M32, M33 form current mirrors that reflect the currents to the output summing node. Transistor M28 is a biasing transistor. The transistors in output power block 440 may be field effect transistors (FETs), and more particularly complementary metal-oxide semiconductor (CMOS) transistors. Resistor 446 is configured to set the gain of output power block 440 and capacitor 448 is configured to set the low pass corner frequency of the low pass filter. For example, capacitor 448 may set the low pass corner frequency of low pass filter 137 illustrated in FIGS. 9 and 26.

The various transistors in the example of FIG. 19 may be sized for appropriate operation and biasing per general design techniques. As an example, transistors M21 and M22 may have sizing ratios of 15/30; transistors M23, M24, M25, and M26 may have sizing ratios of 80/2; transistors M27, M30, M31, and M32 may have sizing ratios of 15/30; transistor M28 may have a sizing ratio of 20/20; and transistors M29 and M33 may have sizing ratios of 150/30. The ratios described above are width/length ratios. In one example, resistor 446 may have a value of 60 mega-ohms, and capacitor 448 may have a value of 250 picofarads. The transistors in the mixer amplifier 250 may be field effect transistors (FETs), and more particularly complementary metal-oxide semiconductor (CMOS) transistors.

Two phases are necessary to reconstruct a hypotenuse of the signal. For ease of illustration, however, FIG. 19 shows the circuit associated with only one of the phases. The extracted power may be represented by the following equation:

$$V_{out}(t) = [\cos^2(\phi) + \sin^2(\phi)] \bullet [I_b R \tanh^2(\frac{V_{in}(t)}{2\eta V_{th}})] \quad (7)$$

where $\phi$ is the phase of the input signal, t is time, $I_b$ is the bias current of the circuit, Vin is the input signal applied to the power block, $V_{th}$ is the thermal voltage (kT/q), which is 27 millivolts at body temperature, R is the value of the load resistor for the circuit, which sets the gain, and $\eta$ is the sub-threshold slope factor, which is a function of the fabrication process and usually falls between approximately 1.5 and 1.7. The above equation represents the output voltage that is produced by combining two of the power extraction blocks shown in FIG. 19. The $\sin^2$ and $\cos^2$ terms represent the sum of two phases of the signal (in-phase and quadrature). By squaring and adding the two phases together, phase drops out between the physiological signal and the on-chip clock, producing a robust power measurement. The $\tanh^2$ term represents the scaling of the translinear multiplier circuit that does the power extraction.

As shown in FIG. 19, the multiplier may be constructed as a self-biased cascade architecture to provide the necessary level shifting to drive the inputs to the tangent-squaring circuit. The in-phase and quadrature channels may each use the same multiplier architecture, and the outputs of each of the channels may be superimposed at the output node to provide a rail-to-rail drive onto a series resistor. The net transfer function of the power extraction module may be represented by the following equation:

$$V_{out}(t) = [I_b R \tanh^2(\frac{V_{in}(t)}{2\eta V_{th}})] \qquad (8)$$

Equation (8) demonstrates that phase sensitivity of the signal chain is eliminated during the power estimation step. The transfer function achieves 1 V/V$^2$ scaling assuming a differential pair bias of 60nA and load resistor of 60M$\Omega$, 10mV at the input and a subthreshold factor of 1.5 for the process. To provide additional accuracy for biomarker detection, chopper stabilization of the multipliers may also be employed. The multipliers may have intrinsic offsets (Voff) on the order of mV, which are not trivial compared to the microvolt biomarkers. The net transfer function with these offsets taken into account may be represented by the following equation:

$$V_{out}(t) \propto V_{in}^2(t) + V_{off}^2 + 2[V_{in}V_{off}] \qquad (9)$$

where $V_{off}$ is the offset due to mismatches among the transistors from finite tolerance. When these offsets are added to the input signal, they form a product that adds a relative amplitude scaling that is dependent on the offset of the multiplier and can be different between the channels. As a signal beats between the in-phase and quadrature channels, the scaling mismatch may create distortion. In order to suppress the effect of these offsets, the inputs may be modulated at 64 Hz with an input chopper. The net transfer function through the multiplier may thus be represented as:

$$V_{out}(t) \propto V_{in}^2(t) + V_{off}^2 + 2[(\Delta)V_{in}V_{off} - (1-\Delta)V_{in}V_{off}] \qquad (10)$$

where $\Delta$ is the duty cycle of the chopper. If the duty cycle approaches 0.5 and the output of the power block lowpass filters the 64 Hz modulation product, then the cross-product is eliminated and the offset is limited to a static offset term that the algorithm can trim out during a calibration process.

An input chopper, such as front-end chopper 442, is an example of a circuit that may suppress intermodulation. The input of the front-end chopper 442 may be the output of a low pass filter that is coupled to the output of the mixer amplifier in the heterodyning chopper-stabilized amplifier. The low pass filter may produce differential Vin+ and Vin-signals. For example, a lowpass filter such as lowpass filters 58, 74, 100, 125 or 133 may produce Vin+ and Vin- signals that can be applied to the front-end chopper 442 to produce the V+ and V- signals that are applied to the differential input of the power block 440. The switches 444A, 444B in the front-end chopper may be switched at a desired chop frequency, such as 64 Hz. For example, to prevent residual offsets in tanh circuits from creating intermodulation products in the I and Q channels, the inputs to the Gilbert multipliers can be chopped with a square wave, e.g., at 64 Hz, via the front-end chopper 442. Providing chopping via the front-end chopper 442 eliminates or reduces the intermodulation products. Without the front-end chopper 442, significant 'beating' of the offsets could occur in the stage and the input signal, which could corrupt the signal significantly. Chopping via front-end 442 can reduce or eliminate this issue. A front-end chopper in the power block could also be desirable in applications in which a heterodyning chopper-stabilized amplifier is used for wireless telemetry applications, e.g., in an RF receiver. As one example, front-end chopper 442 may be used to implement modulators 510 and 520 of the superheterodyning, chopper-stabilized instrumentation amplifier shown in FIG. 26.

The output of power block 440 may have an on-chip capacitor to limit the power bandwidth, when the pad and interconnect parasitics are added to power output node. In some embodiments, the power bandwidth is limited to 10 Hz. In additional embodiments, filtering may also be added to the power block by switching in an off-chip capacitor.

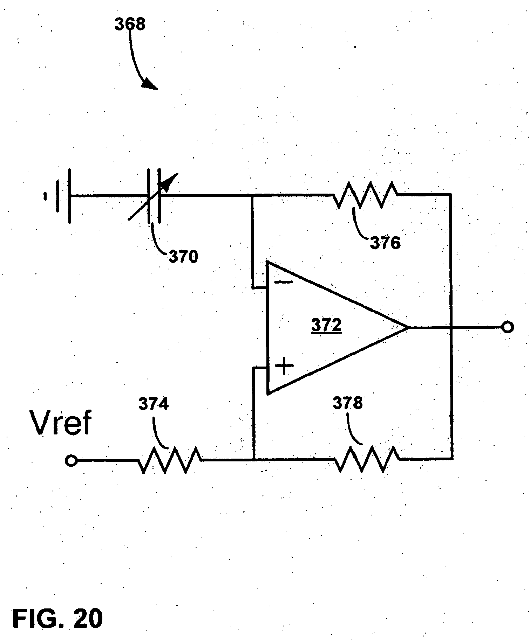

FIG. 20 is a circuit diagram illustrating a clock circuit 368 to generate a clock frequency for a chopper-stabilized, superheterodyne instrumentation amplifier. The delta clock frequency $\delta$ may be important to operation of the amplifier circuit. As shown in FIG. 20, the clock circuit 368 may include an inverting amplifier 372 having a negative input coupled to ground via a variable capacitor 370, and a positive input coupled to a reference voltage Vref via resistor 374. Amplifier 372 forms a comparator. The value of Vref may be selected to be any value between the lower and upper power rails that is convenient for biasing the comparator. The output of the amplifier 372 may be coupled to the negative input via resistor 376, and to the positive input via feedback resistor 378. A microprocessor routine may be configured to recalibrate the clock using a crystalbased system clock as a reference. As one example, variable capacitor 370 may have a capacitance value of 125 Femto farads scalable up to 32 times that value, resistors 374, 378 may have resistance values of 10 mega ohms, and resistor 376 may have a resistance value of 675 kohms.

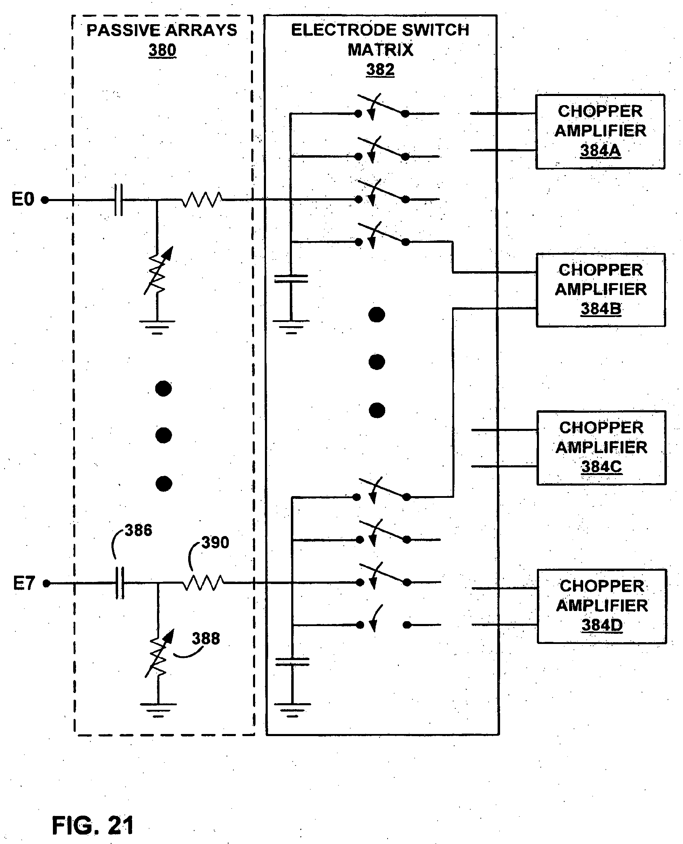

FIG. 21 is a circuit diagram illustrating a multi-channel array of chopperstabilized, superheterodyne instrumentation amplifiers. In the example of FIG. 21, various chopper-stabilized amplifiers 384 are coupled to different pairs of electrodes (E0, E1, etc.) via a switch matrix 382 and passive arrays 380. Each passive array 380 may include external capacitor components. For example, each passive array 380 may comprise a coupling capacitor 386 (e.g., 100 Nano farads) having one end coupled to an electrode (E0, E1, etc.) as an input, and another end coupled to ground via a variable resistor 388 and to a switch in the switch matrix via resistor 390 (e.g., 15 kohm). Again, particular resistor or capacitor values are provided for illustration and should be considered non-limiting. The variable resistor 388 may be programmable on-chip to form a high pass filter in conjunction with the capacitor 386 and series resistor 390. The switches may be formed by +/-10 volts ESD cells with series clamps limited to +/- 3 volts. Each chopper amplifier 384 has a negative input coupled to one electrode and a positive input coupled to another electrode via the switch matrix 382 and the passive arrays 380. The electrodes (E0, E1, etc.) may comprise platinum-iridium electrodes. Although each electrode input to electrode switch matrix 382 is depicted as having a fanout of four, it should be recognized that other combinations are possible. For example, each electrode input may have a fanout of eight resulting in the capability of routing any electrode input to any chopper amplifier input.

Passive arrays 380 may be configured to block DC current flowing through the electrode-sensing device interface in order to avoid corrosion and pH imbalance. The high common-mode input impedance of the chopper amplifier may minimize any common-mode rejection ratio (CMRR) reduction that can occur due to loading imbalances of the input matrix because the matching of the 100nF passive array is limited to 80dB. In addition, ESD cells and on-chip blocking clamps may maintain high impedance over a +/-10 V differential drive across an electrode pair. The combination of coupling capacitors and high input impedance reduces the parallel load of the sensing interface compared to tissue.

The blocking capacitors may provide low-frequency highpass filtering of the signal chain. The capacitors may be used in combination with a programmable resistor on the sensing device to set the high-pass corner for the signal chain. The high-pass corner can be selected at various frequencies through appropriate register selection. Example frequencies include 0.5, 2.5 and 8Hz, in addition to a DC test mode. Such functionality may help reduce the area of sensing device 302.

Each of the heterodyning chopper amplifier channels may be configurable with its own dedicated differential clock to select a band of interest. To avoid beating of the clocks at the non-linear electrode-tissue interface, a common front-end clock may be shared for all of the channels. The differential clock may be embedded in the back-half of the signal chain on-chip, and isolated from the front-end. In some embodiments, the signal may be pre-filtered at the front-end prior to the silicon junctions. Lowpass filtering helps minimize rectification of high-bandwidth signals from sources like telemetry links. To implement this, a series on-chip resistor may be shunted by an off-chip capacitor, one per channel, in front of all low-voltage rectifying junctions such as the limiting-clamp or switch matrix. In one embodiment, the series on-chip resistor may be a 15k$\Omega$ resistor and the off-chip capacitor may be a 3.3nF capacitor.

A frequency-selective signal monitor incorporating a heterodyning, chopper-stabilized amplifier circuit may be desirable in a variety of applications, including the monitoring of neuronal activity in the brain. For examples, a micropower architecture for extraction and processing of neuronal biomarkers may be helpful in promoting the expansion of the diagnostic and therapeutic capabilities of implantable medical devices such as electrical stimulators. The design of a sensing circuit for monitoring of neuronal activity can be challenging. First, in many applications, the signal input should be robust for chronic recording. Second, the circuit architecture should be capable of achieving signal processing, algorithm control, and telemetry with a limited power budget.

For the first requirement, a robust signal input may be obtained by measuring field potentials, which generally represent ensemble behavior in a neural network and can be measured chronically. For the second requirement, architecting an effective solution may require identification of the key information of interest and partitioning the signal chain to play to the strengths of analog versus digital processing. In various embodiments, a frequency selective signal monitor incorporating a heterodyning, chopper-stabilized amplifier circuit, as described in this disclosure, may satisfy the above requirements for neuronal activity monitoring.

As described in this disclosure, for many neurological states of interest, information is encoded as low frequency power fluctuations within well-defined frequency bands of field potentials, similar to the coding found in an amplitude modulation (AM) radio. Recognizing this similarity, incoming field potential signals can be processed with low-power analog circuits to amplify and extract power fluctuations at physiologically-relevant frequencies prior to digital processing. In essence, a frequency-selective signal monitor circuit may adapt a chopper-stabilized instrumentation amplifier to act as a superheterodyning AM receiver for brain signals.

Because power fluctuations in neuronal signals are often orders of magnitude slower than the frequency at which they are encoded, analog preprocessing can greatly reduce the power requirements for implementing a complete mixed-signal system. As the science of neuronal field potentials is rapidly evolving, a superheterodyning chopper circuit as described in this disclosure may be advantageous since it can be made highly flexible while being robust to process, temperature, and mismatch variations. In some embodiments, a circuit as described in this disclosure may exhibit a noise floor of under 2 microvolts rms, and a total system current of 25 microwatts/channel (with a 1.8V power supply) including bandpower extraction, digitization, and algorithmic processing.

A heterodyning chopper amplifier channel generally corresponding to the amplifier circuits described in this disclosure was prototyped in an 0.8 micron CMOS process with high-resistance CrSi to verify the theory of operation. Table 1 below shows some of the heterodyning chopper amplifier results.

### TABLE 1

| Specification | Value | Units/Comments |
|---|---|---|
| Supply Voltage | 1.7 to 3.3 | Volts |
| Supply Current | 5 | $\mu$W/channel (1.8V) |
| Gain | 54 (min) to 80 | dB, programmable |
| Noise | < 2 | $\mu$V rms, 10Hz noise bandwidth |
| CMRR, PSRR | > 80 | dB (DC to 60Hz) |
| Bandpower Center ($\delta$) Trim Step Size | DC to 500<br>5 | Hz<br>Hz |
| Bandpower Bandwidth (BW/2) Trim Step Size | 5 to 25<br>4 | Hz (2-pole)<br>Hz |
| High-Pass Corners | 0.4, 2.5, 8 | Hz |
| Clock Jitter | < +/- 1 | Hz, 4$\sigma$ |
| Clock Drift | < 0.1 | Hz/C |

The total IC current draw of 7 $\mu$W from a 1.8V supply; 5 $\mu$W was allocated for the heterodyning chopper chain, and 2 $\mu$W for the support circuitry. The IC exhibited broad power tuning capabilities for biomarkers between 10 Hz to 500 Hz (with trim steps of 5 Hz). This range of programmability covers both known biomarkers detectable in surface EEG, as well as significantly higher frequency biomarkers. Trim states may be written from a microprocessor via an I2C port, and can be either adjusted as part of an algorithm (e.g. a swept-sine spectrogram) or a state can be locked in with a non-volatile memory array on-chip.

The noise floor of the signal chain was measured to be approximately (2 $\mu$Volts rms)$^2$ with channel conditions programmed to BW=10Hz, and $BW_{power}$ = 1 Hz, in excellent agreement to theoretical expectations and suitable for detesting relevant biomarkers for a neuroprosthesis. The power supply rejection ratio (PSRR) was measured to be greater than 80dB for frequencies that fold into the power output. Since the maximum supply perturbation is bounded to 10mV during stimulation, supply noise is negligible in practice.

The differential clock performance may be important to proper operation of the signal chain. The maximum differential clock jitter was bounded (4$\sigma$) to +/- 1 Hz using 150nA total bias current, and the mean clock drift was approximately 0.1 Hz/C. The tight differential clock tolerance ensures robust programmability using on-chip oscillators.

In some embodiments, a frequency selection monitor based on a heterodyning chopper amplifier circuit may be implemented in a swept spectrum analyzer. In a swept spectrum analyzer, a microprocessor or other controller may be configured to shift the heterodyning frequency in discrete 5 Hz steps, and the power is then digitized and stored in the memory module. A swept spectrum mode may be useful for identifying bands of field potential energy, with a power efficient search algorithm. The swept spectrum feature may be utilized full time or as a selectable mode for operation when desired. This example emphasizes the power of analog preprocessing coupled with a flexible microprocessor.

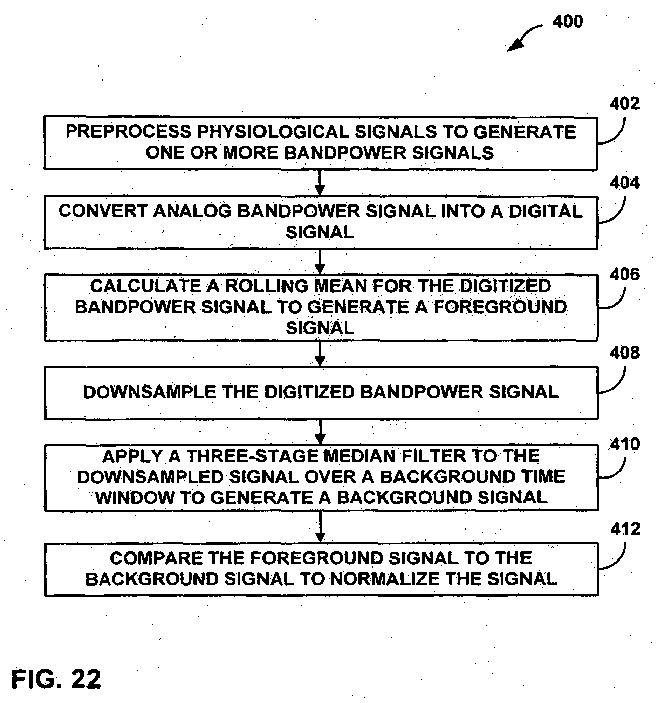

FIG. 22 is a flow diagram illustrating an example process 400 that can be run within sensing system 300. In process 400, analog sensing unit 308 may monitor physiological signals received from one or more sensing electrodes and provide an analog signal processor that performs preprocessing on the signals to generate one or more bandpower signals (402). The rest of process 400 will be described with respect to a single bandpower signal although it should be recognized that the process is also capable of being implemented in parallel with multiple bandpower signals. According to process 400, analog-to-digital (A/D) converter 316 may covert the analog bandpower signal into a digital signal (404). Microprocessor block 308 may generate a foreground signal for the digitized bandpower signal by calculating a rolling mean of the signal over a short foreground time window (e.g., 2 seconds) (406).

Microprocessor block 306 may downsample the digitized bandpower signal to a lower sampling rate (408). Microprocessor block 306 may generate a background signal for the digitized bandpower signal by applying to the digitized bandpower signal a three-stage median filter over a background time window (e.g., 30 minutes) followed by a lowpass smoothing filter (410). In some embodiments, the background time window may be longer than the foreground time window. Microprocessor block 306 may normalize the bandpower signals by comparing the short foreground time window (e.g., 2 seconds) to the longer background time window (e.g., 30 minutes) (412). This normalized signal is then fed into detection/tracking logic within microprocessor block 306, which enables the system to monitor changes in the power for the selected frequency band. The detection/tracking logic may produce detection output and tracking output that can then be used to trigger loop recording and/or to titrate stimulation therapy.

Microprocessor block 306 may control settings on the analog sensing unit 308 through one or more control registers. This enables configuration of the gain and switch matrix as well as parameters like bias trims. Since microprocessor block 306 is also running the algorithms, it is possible to perform feedback control back to the analog sensing unit 308. For example, the background signal in process 400 of FIG. 22 may be used to adjust the gain in analog sensing unit 308 on the fly, thereby keeping the operating point in the optimal range for detection headroom. This particular scheme may be referred to as "background feedback gain control."

A neurostimulation therapy and sensing system may inject and measure signals that have magnitudes that are several orders of magnitude apart. For example, the signals being sensed by the system (i.e. the physiological signals) may be on the order of microvolts, while the signals injected by the system (i.e. the stimulation signals) may be on the order of volts resulting in the extraction of a biomarker that is six orders of magnitude lower than the stimulation signal. In addition, some neurostimulation therapies involve delivering stimulation continuously, or at least a significant portion of the time, so shutting down sensing, or 'blanking', during this time may not be a desirable option.

One way to manage the large differential in the magnitude of the injection and measurement signals is to have separate leads for stimulation and for sensing. In addition to the physical separation of the leads, careful placement of the leads and sense/stim configuration can take advantage of the reciprocity theorem of electromagnetism. Stated mathematically:

$$\phi_B - \phi_A = \frac{\vec{E}_{AB} \bullet \vec{Id}}{I_{AB}} \rightarrow 0 \qquad\qquad (11)$$

The dot product relationship in Equation (11) indicates minimum effect when the measurement vector is orthogonal to the stimulation current flow. Thus, the differential amplitude of the stimulation as seen by the sense electrodes can be greatly reduced by careful lead placement.

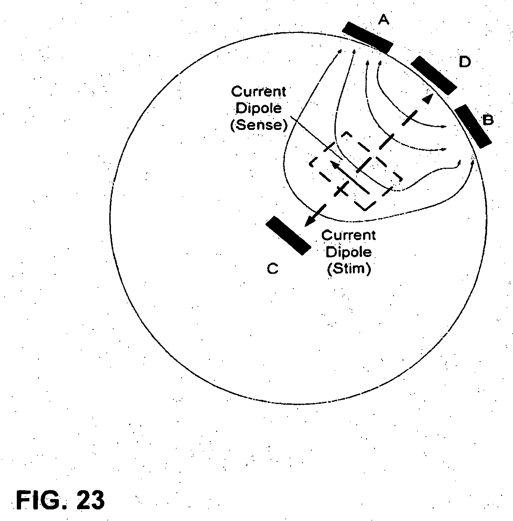

FIG. 23 is a conceptual diagram illustrating a lead placement arrangement that exploits the relationship expressed by the reciprocity theorem. Intuitively, the mathematical relationship can be thought of as imposing a symmetry constraint on the sense-stimulation electrode system. FIG. 23 shows an example where the sensing dipole (A↔B) is placed symmetrically about a unipolar stimulation electrode (C↔D) with far-field return. Note that when the dipole from therapy stimulation is orthogonal to the biomarker sensing vector, the chances of extracting a signal may be greatly increased.

Additional embodiments described in this disclosure may provide a system based upon a neural sensing and algorithm extension applied to a neurostimulator. The design of the sensing device may support efficiently extracting neuronal biomarkers using analog preprocessing prior to digitization and analysis by various algorithms. The architecture provides broad 'tunability' and robustness. Such a fully implantable system may be used to answer questions with the goal of improving neurostimulation therapies, such as DBS.

Moreover, such a system that includes both sensing and stimulation capabilities may provide one or more advantages. For example, such systems may help identify chronic biomarkers within the brain without the spatiotemporal filtering limitations commonly associated with surface EEG recording. As another example, such systems may be able to determine what algorithms provide closed-loop control that is both safe and effective. As yet another example, such algorithms may evaluate whether improvements in therapy outcomes outweigh the complexities of closed-loop control.

A sensing device designed in accordance with this disclosure may provide a mixed-signal sense and control architecture enabling a closed-loop neuromodulation device. Such a device may be used as a research tool for exploring real-time titration of neuromodulation based on bioelectrical markers in the brain. In some embodiments, the device architecture may be partitioned with respect to the neural coding of the biomarkers. Such partitioning may allow the device to accurately and chronically monitor neuronal activity, process algorithms, and titrate stimulation with an architecture that is robust, ultra-low power, and flexible. Many biomarkers of interest are encoded as low frequency power fluctuations of discrete frequency bands. A sensing system utilizing a custom integrated circuit (IC) that configures a micro-power chopper-stabilized amplifier to also act as a super-heterodyne filter may allow for accurate tracking of power fluctuations. Heterodyning provides the flexibility to accurately select biomarker parameters over a broad physiological spectrum. In addition, extracting core neural information in the analog domain reduces the power requirements for the digital processing of the control algorithm. The IC may use 5µW of power and achieve a detection floor of 1µVrms biomarkers, and may use less than 25µW/channel to perform biomarker extraction, algorithmic processing, and control of the neurostimulator.

A mixed signal sensing device generally corresponding to the sensing device described in this disclosure was prototyped in a 0.8um CMOS process with high-resistance CrSi to verify the theory of operation of the heterodyning chopper amplifier. The total current draw of the prototype was 2.5µA per channel from a 1.8V supply, where 2.2µA was allocated for the heterodyning chopper chain, and 0.3µA for the shared support circuitry. Table 2 below shows the results.

TABLE 2

| Specification | Value | Units/Comments |
|---|---|---|
| Supply Voltage | 1.4 to 3.3 | Volts |
| Supply Current | 4.5 | µW/channel (1.8V) |
| Total Channel Gain | 54 to 80 | dB, programmable |
| Noise Floor (detection) | 1 | µV rms, 10 Hz noise bandwidth, 1 Hz power band |
| CMRR, PSRR | >80 | dB (DC to 500Hz) |
| Bandpower Center ($\delta$) Trim Step Size | DC to 500<br>5 | Hz<br>Hz |
| Bandpower<br>Bandwidth (BW/2)<br>Trim Step Size | 5 to 25<br>3 to 15<br>4,2 +/- 15% | Hz (2-pole)<br>Hz (3-pole)<br>Hz (2,3 pole) |
| High-Pass Corners | 0.4, 2.5, 8 | Hz |
| Clock Jitter | < +/- 1 | Hz, 4$\sigma$ |
| Clock Drift | < 0.1, 0.5 | Hz / C (mean, 4$\sigma$) |
| Linearity (Pre-power) | < -65dB | THD (0.001-1mV input) |

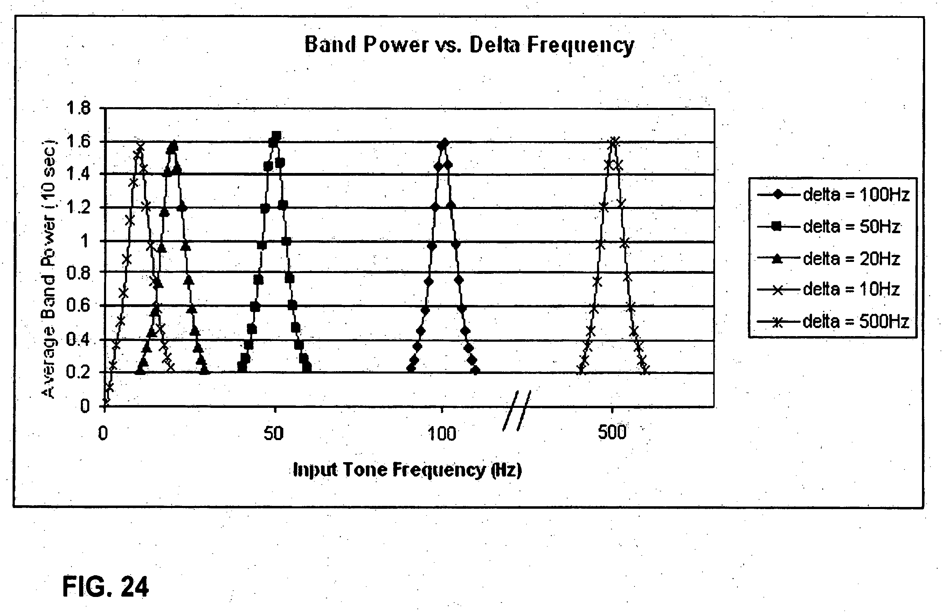

FIG. 24 is a diagram illustrating the broad power tuning capabilities of the chopper for biomarkers between 10 Hz to 500 Hz. This range of programmability, in 5Hz steps, covers both known biomarkers detectable in surface EEG, as well as significantly higher frequency biomarkers. Trim states may be written from the microprocessor, and can be either adjusted as part of an algorithm, e.g. a swept-sine spectrogram, or a state can be set with an on-chip EEPROM.

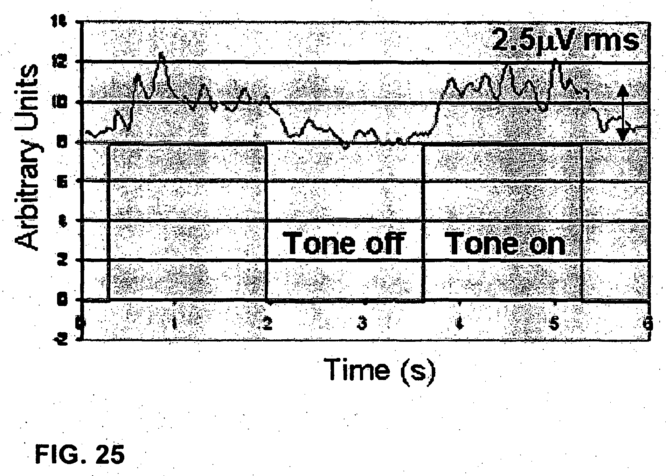

The signal chain's noise floor was measured to be approximately (1 µVrms)$^2$ with channel conditions programmed to BW=10Hz, and BWpower =1 Hz, in agreement with theoretical expectations and suitable for detecting relevant biomarkers for a neuroprosthesis. FIG 25 is a diagram illustrating bandpower response from a 2.5uVrms (top) to 50Hz gate (bottom) tone step. The power supply ripple rejection ratio (PSRR) was measured to be greater than 80dB for frequencies at risk of folding into the power output. The maximum supply perturbation during stimulation was measured to be under 10mV.

The maximum differential clock jitter was measured and bounded (4$\sigma$) to +/- 1 Hz using 200nA channel bias current, and the clock drift (4$\sigma$) was 0.5 Hz/C, with a mean of 0.1 Hz/C. Based on practical algorithm studies using data from twenty patients, the measured clock tolerance provides acceptable tuning within the normal physiological temperature range (37C +/- 2C) and ensures band tuning is maintained with adequate precision.

The following section covers the results for a prototype system having a sensing device working within a full prototype closed-loop neurostimulator. The system may generally correspond to sensing system 300 depicted in FIG. 16. The discussion of system-level results requires a brief overview of both algorithm implementations that are enabled with the processing partitioning techniques described herein, and the constraints on electrode sense-stimulation interactions.

The algorithm used in the prototype generally corresponds to the algorithm illustrated in FIG. 22. This algorithm may be useful for various applications, such as seizure detection for example. In the prototype, the bandpower signal was normalized by comparing a short foreground time window (such as 2 seconds) to a longer background time window (such as 30 minutes). Normalization allows the system to adapt not only to different signals, but to variability over time. The normalized signal was then fed into detection logic, which enables the system to monitor for transient changes in the power for the selected frequency band. This detection logic can then be used to trigger loop recording and/or to titrate stimulation therapy.

In the prototype, the microprocessor controlled the settings on the sensing chip and loop recorder through control registers. This enabled configuration of the gain and switch matrix as well as parameters like bias trims. Since the processor is also running the algorithms, it was possible to perform feedback control back to the analog sensing unit. For example, upper and lower thresholds could be put on the background power measurement in the algorithm shown in FIG. 22 and this information could be used to adjust the gain of the programmable gain amplifier. Such a technique can adjust to the slowly-varying background power in the patient's brain, which may help to minimize the dynamic range requirements of the microprocessor ADC and keep the operating point in the optimal range for biomarker detection. The microprocessor block could also transfer the data to the loop recorder SRAM with the aid of a digital interface block within the sensing device. All together, the digitization, algorithm and loop recorder were run with a 1% duty cycle, keeping microprocessor current to 12.5$\mu$A per channel.

The signal processing was partitioned such that the sensing device signals were processed using a microprocessor, so that algorithms could be customized by making firmware changes downloadable through telemetry. The biomarker of interest had already had their power-in-a-band measurement extracted by the sensing device. Since this signal may change very slowly compared to the frequencies that encode the biomarkers, sampling and processing were done at rate of 5 Hz or lower. Using this method of analog preprocessing and running algorithms at slow rates, we could limit the total power of the sensing extension to an order of a magnitude lower than that of the stimulation therapy.

Analog headroom may be managed by minimizing the coupling between stimulation and sensing vectors, as shown in FIG. 23. This may help to prevent the amplifier from saturating. The coding properties of LFPs can be used to further suppress feed-through contamination. This method exploits the potential separation between the LFP biomarker and the finite band excited by neurostimulation. With this approach, if the stimulation frequency is selected to be outside the sensitive band of the biomarker, then the spectral processing characteristics of the sensing device can be used to reject stimulation artifacts. In some cases, the sharp attenuation of out of band signals with the heterodyning spectral processor can reject stimulation coupling adequately to extract biomarker fluctuations indicating seizure activity. In some cases, the stimulation frequency and LFP biomarker may be separated in the frequency domain.

FIG. 26 is a block diagram of another example superheterodyning, chopper-stabilized instrumentation amplifier 500 that may be useful within a frequency-selective signal monitor. In the example of FIG. 26, instrumentation amplifier 500 is arranged to implement a nested chopper architecture. The tunable heterodyning amplifier circuit extracts signal power within the physiologically relevant band. The dual-nested chopper architecture uses two different chopper frequencies fclk/m and fclk to improve the power-bandwidth tradeoff while eliminating offsets and low frequency noise. An outer chopper uses the fclk/m frequency while an inner chopper used the fclk frequency (and fclk + delta frequency for heterodyning). The value of m may be greater than 1. Accordingly, the outer chopper frequency fclk/m may be slower than the inner chopper frequency fclk.

Several chopper modulation techniques may be used to achieve microvolt signal resolution with the spectral analysis strategy described in this disclosure. The total signal chain with modulation is detailed in FIG. 26. The 'core' chopper modulation, with two clocks at fclk separated by $\delta$, provides the mechanism for heterodyning and thereby selecting the band of interest. Although this does achieve the necessary frequency heterodyning, two practical issues may remain.

The first issue is that the residual offsets in the core chopper can be on the order of several microvolts. The problem with this residual offset is that it is superimposed on the signal of interest, which may cause significant signal perturbations in the output signal as the phase of the biomarker beats against the $\delta$ clock. To address this issue, a 'nested' chopper switch set may be implemented before the first chopper amplifier, and after the programmable gain amplifier (PGA), with the fclk/m clock, as shown in FIG. 26. The optional PGA may be provided to increase the gain of the amplified signal.

The small residual offsets are then up-modulated and filtered out using the BW/2 selection filter. As an illustration, the nested chopper may run nominally at Fclk/64, 128 Hz, to minimize residual charge injection offset, but fast enough to minimize perturbations to low-frequency dynamics. Note that since the PGA is also embedded in the loop, its residual 1/f noise and offset is also suppressed at the lower rate. The use of passive lowpass filter architecture in the BW/2-selection block may minimize additional contributions of offset to the signal chain after the nested chopper.

The second issue is that residual offsets in the output multiplier blocks create an intermodulation product that also creates significant distortion when trying to resolve microvolt signals. The use of an additional, low-frequency chopper prior to multiplication may be used to correct that issue. For example, a chopper at a frequency of fclk/2m may be used to address the intermodulation product. This chopper frequency may be less than the fclk/m and fclk frequencies of the outer and inner choppers, respectively. Notably, with the additional chopper, because the multiplier squares the signal, a subsequent explicit down-modulation block may not be required. A low pass filter may be provided to set the power bandwidth to produce the EEG bandpower output.

Hence, in accordance with this disclosure, a physiological signal monitoring device may have a nested chopper architecture. The nested chopper architecture may include an outer chopper circuit comprising a modulator and a demodulator. Between the modulator and demodulator of the outer chopper circuit, the nested chopper architecture may include an inner chopper circuit comprising a modulator, amplifier, and a demodulator. The outer chopper circuit may modulate and demodulate at a first frequency and the inner chopper circuit may modulate at a second frequency and demodulate at a third frequency. The first frequency may be less than the second frequency. The third frequency may differ from the second frequency by an offset. The offset may correspond to a frequency within a selected frequency band. In this way, the baseband for the heterodyning inner chopper is effectively shifted to an intermediate frequency.

Such a device may comprise, in an example embodiment, a physiological sensing element that receives a physiological signal, and a first modulator that modulates the signal at a first frequency to produce a first modulated signal, and a second modulator that modulates the first modulated signal at a second frequency different from the first frequency to produce a second modulated signal, an amplifier that amplifies the second modulated signal, and a first demodulator that demodulates the amplified signal at a third frequency different from the second frequency. The third frequency may be selected such that the demodulator substantially centers the selected frequency band of the signal at the first frequency. The device may also comprise a second demodulator that demodulates the demodulated signal at the first frequency such that the selected frequency band is substantially centered at the baseband. The second modulator and the first demodulator may form an inner chopper circuit surrounding the amplifier. In addition, the first modulator and second demodulator may form an outer chopper circuit thereby providing a nested chopper architecture. In some embodiments, a second amplifier may be placed between the first and second demodulators such that the second amplifier is placed inside of the outer chopper circuit but outside of the inner chopper circuit.

Superheterodyne instrumentation amplifier 500 contains several components that correspond in structure and operation to various components shown in the instrumentation amplifier of FIG. 9. Such corresponding components have been referenced by the same numerals. Similar to the instrumentation amplifier in FIG. 9, superheterodyne instrumentation amplifier 500 includes a first set of inner chopper modulators 120, 124 in an in-phase channel surrounding adder 121 and amplifier 122 as well as a second set of inner chopper modulators 128, 132 in a quadrature channel surrounding adder 129 and amplifier 130. Like the instrumentation amplifier in FIG. 9, modulators 120 and 128 are driven at a chopping frequency ($f_c$) and demodulators 124 and 132 are driven at a clock of frequency equal to the chopping frequency plus or minus an offset ($f_c \pm \delta$). The demodulating signal for demodulator 132 in the quadrature signal path may be shifted by 90 degrees in relation to the demodulating signal for demodulator 124 in the in-phase signal path. The heterodyning frequency offsets 123, 131 ($\delta$) operate in a substantially similar fashion to the amplifier illustrated in FIG. 9. In addition, the lowpass filters 125, 133, 137, squaring units 126, 134, and summing unit 136 all operate in a similar fashion to what has been already described with respect to instrumentation amplifier in FIG. 9. In some embodiments, up-modulators 120 and 128, down-modulators 124 and 132, and amplifiers 122 and 130 may form a heterodyning circuit configured to convert a selected frequency band of the physiological signal to a baseband according to this disclosure. In other embodiments, only the in-phase modulators 120, 124 and amplifier 122 may form the heterodyning circuit. In some cases, modulators 510 and 520 may be implemented by and correspond to front-end chopper 442 shown in FIG. 19.

Superheterodyne instrumentation amplifier 500 also includes outer chopper modulators 502 and 508 in the in-phase channel and outer chopper modulators 512 and 518 in the quadrature channel. Outer chopper modulators 502 and 512 may modulate the physiological input signal ($V_{in}$) at an intermediate frequency (e.g., $f_c/m$). Then, inner chopping modulators 120 and 128 may modulate the respective signals at a chopping frequency. The net modulation frequency may then be described as the chopping frequency plus or minus the intermediate frequency (e.g., $f_c \pm f_c/m$). The twice up-modulated signals are then fed through amplifiers 122, 130, which may add noise 121, 129 to the signals. Inner chopping demodulators 124 and 132 demodulate the amplified signals at a frequency equal to the chopping frequency plus or minus an offset ($f_c \pm \delta$) and upmodulate the baseband noise components to higher frequencies. The frequency driving the demodulators may be selected such that the demodulator substantially centers a selected frequency band of the signal at the intermediate frequency.

The signals are then fed through programmable gain amplifiers (PGAs) 506, 516, which provide the ability to set the gain and/or dynamic range of the frequency channels. These settings may be programmable and based upon the physical condition or therapy being measured. The PGAs may also add additional noise 504, 514 to the signals. After a second amplification of the signals, outer chopping demodulators 508, 518 may demodulate the signals back to baseband. A selected frequency, which was centered at the intermediate frequency, may now be centered at DC in the baseband. Low pass filters 125, 133 filter out noise components that have been upmodulated as well as higher-order signal harmonics. Additional modulators 510, 520 modulate the baseband signal to a second intermediate frequency (e.g., $f_c/2m$) in order to reduce intermodulation noise. The signals are then fed through squaring units 126, 128 and added together with adder 136 to form a band power measurement. Low pass filter 137 filters the signal to extract the low-frequency fluctuations in the band power.

In some embodiments, the front-end modulators may be implemented as a single modulator. For example, modulators 502 and 120 may be implemented as a single modulator and modulators 512 and 128 may be implemented a single modulator with a composite frequency chosen to be $(f_c + f_c/m)$.

A sensing device that contains a heterodyning chopper amplifiers designed in accordance with this disclosure may provide an independent adjustment of $\delta$ and Q over a wide spectrum of biomarkers with parameters well within process tolerances. These parameters may be able to be adjusted over a broad range through microprocessor control. After the bandwidth of the signal is reduced to the order of 1Hz, the microprocessor may provide digitization and algorithmic processing functionality. With low data-rates, microprocessor overhead may be minimal and algorithm blocks, such as the median filtering and loop recording blocks, can be run with less power.

The use of feedback within the heterodyning chopper and programmable gain amplifier makes it very linear prior to the power extraction stage. This means that attenuation may not be required. In addition, a sensing system designed in accordance with this disclosure may improve overall system power efficiency by two orders of magnitude through elimination of fast digital processing.

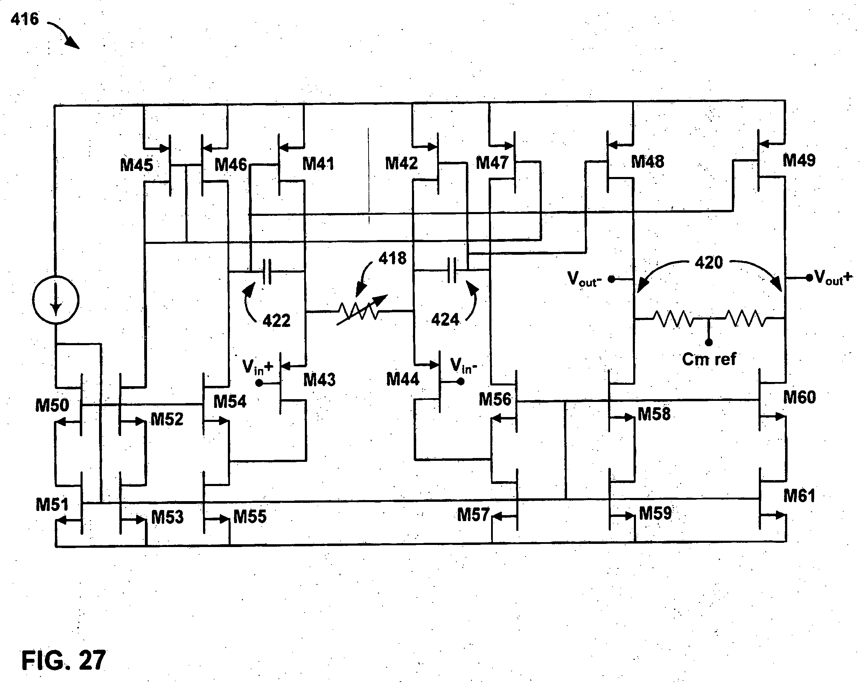

FIG. 27 is a circuit diagram illustrating a programmable differential gain amplifier 416 suitable for use within the superheterodyne instrumentation amplifier of FIG. 26. For example, programmable differential gain amplifier 416 may correspond to PGAs 506 and 516 in instrumentation amplifier 500 of FIG. 26. In other examples, programmable differential gain amplifier 416 may be used in sensing device 302. In this case, gain amplifier 416 may be coupled between the output of one of the chopper amplifiers 322 and the analog-to-digital converter (ADC) 316 of sensing device 302 illustrated in FIG. 16. In addition, multiple gain amplifiers similar to gain amplifier 416 may be placed in parallel between each of the chopper amplifiers 322 and the analog-to-digital converter 316. As another example, gain amplifier 416 may be used in a frequency selective monitoring circuit as shown in FIGS. 3 and 6A. In such an example, gain amplifier 416 may be coupled between the output of the instrumentation amplifier (32, 72) and the input of the signal analysis unit (33, 73). It should be understood that these configurations are merely exemplary, and that other configurations are possible.

Gain amplifier 416 may further amplify the physiological signal to minimize the dynamic range requirements of analog-to-digital converter 316 in microprocessor block 306. Since the gain required from this block is dependent on the specific patient, electrode location and intended control algorithm, the amplifier may be configured from a signal fed back by the algorithm running in microprocessor block 306. In an example embodiment, gain amplifier 416 may have a programmable gain that takes on different values (e.g., x5, x10, x20, x40) with a high degree of stability (e.g., +/- 5%). Gain amplifier 416 may provide high linearity and a high input impedance to avoid loading the chopper amplifier. The transistors in gain amplifier 416 may be field effect transistors (FETs), and more particularly complementary metal-oxide semiconductor (CMOS) transistors.

The current through the front-end FETs in amplifier 416 may be held constant by a minor servo loop. The servo loop forces the differential voltage at the inputs to fall predominantly across source resistor 418, minimizing distortion from a variable gate-source voltage. Source resistor 418 may be programmable at several different levels of resistance. For example, source resistor 418 may be programmable from one to eight megaohms using switches shunting one or more CrSi resistors. By mirroring the top-side servo currents to output resistor tap 420, a gain can be set using the ratios of the resistors that is stable across process corners and temperature. In addition, by supplying a reference to the midpoint of the resistor string 420, we can also set an arbitrary bias point on the amplifier's output depending on the requirements of the next stage.

The various transistors in the example gain amplifier of FIG. 27 may be sized for appropriate operation and biasing per general design techniques. As an example, transistors M41, M42, M48, M49, M59 and M61 may have sizing ratios of 4x25/25; transistors M43 and M44 may have sizing ratios of 200/4; transistor M45 may have a sizing ratio of 25/25; transistors M46 and M47 may have sizing ratios of 2x25/25; transistor M52 may have a sizing ratio of 25/2; transistor M53 may have a sizing ratio of 25/25; transistors M54 and M56 may have sizing ratios of 2x25/2; transistors M55 and M57 may have sizing ratios of 6x25/25; and transistors M58 and M60 may have sizing rations of 4x25/2. In one example, capacitors 422 and 424 may each have a value of 4 picofarads, adjustable resistor 418 may have a range of 1-8 mega-ohms, and resistors 420 may each have a value of 20 mega-ohms. The transistors in the gain amplifier may be field effect transistors (FETs), and more particularly complementary metal-oxide semiconductor (CMOS) transistors.

In some embodiments, a frequency selection monitor based on a heterodyning chopper amplifier circuit may be implemented within an implantable system that provides deep brain stimulation (DBS). Deep Brain Stimulation (DBS) may refer to the extracellular electrical stimulation of brain tissue via the delivery of relatively high frequency current pulses, and can be an effective therapy for a number of pathologies of the human nervous system. A DBS system may include an implantable pulse generator (IPG) that is placed into the pectoral region of the chest of a patient. The IPG may contain the energy for stimulation within its battery, as well as the circuitry to provide stimulation pulses. The IPG may interface to neural tissue through a series of electrodes placed in a specific physiological target in the brain. Stimulation pulses from the IPG may be localized to the vicinity of the electrodes thereby providing targeted modulation of the firing pattern in a specific neural circuit. DBS may be used for the treatment of movement disorders such as Parkinson's Disease, essential tremor, dystonia. In addition DBS may used as therapy for epileptic seizure, bipolar disorders, chronic obesity, and obsessive-compulsive disorders. Similar modulation circuits may also used for the treatment of incontinence, by stimulating the sacral nerve, and chronic pain, through stimulation of the spinal chord.

Traditional DBS systems are commonly referred to as "open-loop" systems, meaning that the device has no sensing capability and adjustments require clinician intervention. A frequency selective monitor in accordance with this disclosure may assist in measuring neurological activity to help provide "closed-loop" therapy based on relevant neurophysiological biomarkers. In addition, a DBS system that incorporates a frequency selection monitor as described in this disclosure may assist in the practical measurement of chronic neurological information and in the implementation of algorithms for closed-loop titration of therapy.

Some systems for monitoring neuronal activity may include EEG monitoring using scalp electrodes and single neuron spike detection. However, there may be limitations to both these methods. For example, scalp electrodes may be prone to movement artifacts, which can greatly increase the difficulty of algorithm development. In addition, scalp electrodes may not be able to capture frequencies greater than approximately 50 Hz, which prevents exploration of promising biomarkers that have higher frequency content. For example, high gamma band power fluctuations in the motor cortex may signal motion intent of a patient. These signals may be commonly filtered out in EEG recordings derived from scalp electrodes. Also, the use of scalp electrodes may not be well suited for chronic studies. Neuron spike detection may also be susceptible to chronic recording issues like tissue encapsulation and micromotion.

Thus, it may be desirable to sense neuronal activity by recording and analyzing local field potentials (LFPs) using frequency-selective monitoring in accordance with techniques described in this disclosure. Because LFPs represent the ensemble activity of thousands to millions of cells in an in vivo neural population, their recording may avoid chronic recording issues. LFPs may be obtained with leads having sensing electrodes located on or in the brain. This may be well-suited for devices providing DBS, which already requires access to the brain. Low-frequency power fluctuations of discrete frequency bands in LFPs provide useful biomarkers for discriminating between brain states. Relevant biomarkers span a broad frequency spectrum, from approximately 1 Hz oscillations in deep sleep to greater than 500 Hz "fast ripples" in the hippocampus, and have widely varying bandwidths. In many cases, pathological states can be differentiated by such biomarkers. A system designed in accordance with this disclosure may be designed to sense such biomarkers. This may allow researchers to develop and test novel algorithms, including closed-loop therapy with the goal of improving therapy outcomes.

The primary role of the brain can broadly be considered in terms of its functional capacity as an information processor. Information about the current state of the 'system', as well as the world in which it is acting, is provided to the central nervous system through various afferent sensory signals, where it is then transformed, or 'processed', in some way. The transformed information effects action through efferent pathways connected to musculature, hormone regulating organs and other bio-physical and bio-chemical mechanisms. The input/output transformation can be viewed as an information transformation with the mutual information providing a measure of the capacity of that system.

Pathological dysfunction of brain systems can take a number of forms, and in accordance with the information processing framework, can be viewed as an information processing failure. Information might be corrupted due to noise or the intermittent loss of signal, or it can be lost entirely due to a transmission failure or lesion of central elements as occurs with infarction due to stroke. The information transfer functions can be corrupted due to many factors including the loss of individual neurons throughout the brain or the failure of various biochemical reactions affecting cellular processes.

A particular form of information processing failure is increasingly being investigated as a causal agent in numerous brain pathologies including epilepsy, Parkinson's disease, bipolar disorders and obsessive compulsive disorders to name a few. This failure occurs when the normally uncorrelated firing of individual neurons throughout a region of brain tissue devolves into a coherently organized synchronous oscillation. In this state, the normal, transiently correlated behavior of individual elements throughout the network is forced into a phase-locked firing pattern that significantly reduces the mutual information between afferent/efferent signals and completely disrupts the information processing capacity of the system as a whole.

An interesting property of this disease model is that correlated firing makes it feasible to design sensing systems to detect and monitor the presence of an information processing pathology. A 'biomarker,' or clinical signature, of this type of pathology is represented as electrical oscillation that appears within a discrete frequency band in a specific anatomical location. Using spectral analysis, the coding of the network close to the sensing electrodes can be deciphered and deductions can be made with respect to the state of the neural circuit. Unlike the spike recordings often discussed for motor prosthesis systems, these ensemble cell firings result in diffuse field potentials that are amenable to chronic measurement from electrodes already approved for DBS therapy. As such, it may be desirable to map the field fluctuations to a specific disease state, and to devise a stimulation strategy that can provide therapeutic benefits when the pathological state is detected.

As an example, epilepsy is characterized by the abnormal emergence of highly coherent, periodic synchronous firing of large populations of neurons. If the phase of individual neurons firing in a population is taken into account, the total phase coherence across the population can be loosely considered as a probability measure over phase. In the case of oscillatory dysfunctions, as phase coherence increases the entropy measure over the phase distribution decreases, negatively impacting the information capacity of the system as a whole. In a seizure, this phase-locked behavior becomes extreme, yielding a nearly total information processing failure and a strong increase of energy diffused across the alpha (8-12 Hz) and beta (12-40 Hz) spectral bands

Another example is Parkinson's disease. The functional mechanisms of Parkinson disease are presently unknown; however, recent research has demonstrated a strong correlation between patient symptoms and highly coherent Beta band (15-30hz) oscillations in spike firing intervals within certain motor-control populations of neurons. The result of this synchronized firing could be a reduction in the uncorrelated (high information capacity) state space or, alternatively, increased power in a correlated noise source. In either case, the information processing capacity of the system may be degraded.

A difficulty in deciphering neural dynamics is the barrier to extracting information from the brain circuit. Scientific tools that monitor neural dynamics are needed to uncover the basic principles of function, the therapeutic affects of stimulation, and to provide the observability needed for adaptive neuromodulation. Systems for accomplishing these tasks are becoming more practical, as we learn enough about brain coding to architect devices for practical sensing and stimulation. These devices, per the next section, improve the link between silicon- and carbon-based electrical systems.

Adding sensing technology to a stimulator could provide several benefits. The scientific benefit is driven by the need for better understanding of basic network dynamics, information flow, and mechanisms of action for DBS therapies. From a clinical standpoint, there is interest in using sensing of neurological activity to help provide "closed-loop" therapy based on therapeutically relevant biomarkers. The goals of closed-loop therapy, also known as adaptive modulation, are to improve therapeutic outcomes and potentially increase device longevity by entering low-energy states when stimulation is not required. The addition of sensing can also provide quantitative diagnostics to aid in therapy titration in "open loop" use.

A saline tank model was developed for evaluating the closed-loop neurostimulator prototype. The concept is to adjust the information flow in a neural circuit, essentially dynamic entropy control, based on a measured biomarker. For the adaptive controller, we programmed the algorithm to initiate stimulation upon detection of a burst of LFP energy in the 'β band' (15-40 Hz). The β band is often an indicator of a pathological information pattern flowing through the neural circuit. A recorded signal from a human subject was fed into a saline tank. This signal was then extracted by the input electrodes placed across the appropriate sensing vector representing a cortical input, while the stimulation electrodes were placed within 1cm of the sensing electrode using a return provided with an indifferent far-field electrode. The saline conductivity and signal drive strength was adjusted to mimic the electrical properties and signal levels of brain tissue, respectively.

After amplification and bandpower extraction with the sensing IC, the microprocessor sampled the signal at 5 Hz and ran an algorithm comparing the mean energy in the last two seconds to the median energy of the last thirty minutes. When the ratio exceeded a present threshold and time duration, indicative of a true pathological event, a detection flag was passed to the neurostimulator stimulation controller over the I2C bus. This initiated stimulation at 140 Hz. Stimulation proceeded over the duration of the elevated β-band energy. The frequency separation between stimulation and LFP band energy allowed the system to maintain sensitivity to the biomarker, even in the presence of stimulation from an electrode 1cm away.

This model illustrates that the research tool can address the major challenges of implementing an adaptive neuromodulation system. The system may be designed around the electrical biomarker of LFP band fluctuations. In some embodiments, the processing partition can extract the signal with a total current draw of under 15uA/channel (sense, control), which is practical for implementing within a battery-powered implantable neuromodulation system.

Neuromodulation may be defined as the actuation of the nervous system with electrical stimulation. A neuromodulator may translate energy from a battery into information embedded within the nervous system. This information may provide therapeutic benefit to patients by modulating the pathological oscillations within a diseased neural circuit. One specific method of neuromodulation is deep brain stimulation (DBS). DBS is an approved therapy for the treatment of movement disorders such as Parkinson's, essential tremor and dystonia. DBS systems commonly operate in an "open-loop" mode, meaning the device has no inherent sensing capability and adjustments require external intervention through a telemetry system. A DBS system that provides closed-loop therapy may improve therapeutic outcomes with active titration of stimulation, and increase device longevity by entering low energy stimulation states when therapy is not required. Thus, it may be desirable to integrate a system for measuring neurological activity within a DBS system in order to provide "closed-loop" therapy based on therapeutically relevant biomarkers such as bioelectrical or activity sensing. A closed-loop system may provide chronic measurement of neurological information and may assist in the creation algorithms for closed-loop titration of therapy actuation.

A closed loop neuromodulation architecture may be modeled within the context of classical state equations:

$$\dot{\mathbf{x}}(t) = \mathbf{A}(t)\mathbf{x}(t) + \mathbf{B}(t)u(t)$$
$$\mathbf{y}(t) = \mathbf{C}(t)\mathbf{x}(t) + \mathbf{D}(t)u(t) \qquad\qquad (12)$$

where vector x(t) is the neural circuit's 'state,' u(t) is the input to the neural circuit, which can include sensory input, drugs or electrical stimulation, and y(t) is the output of interest such as tremor or another representative biomarker. The neural circuit dynamics and therapeutic transfer functions are then represented by the four transfer function matrices: A(t), representing neural circuit dynamics, B(t), defining the effect of stimulation on the neural state, C(t), representing how the neural state is mapped to observable therapeutic biomarkers, and D(t), representing the feed-forward path from stimulation to biomarker. Stimulation may also almost impact A(t) as well. The therapeutically-relevant variable y(t), denoted as the biomarker, may be controlled through modulation of the stimulation parameter u(t). This may be done by creating a net feedback path to the stimulation of the network. The relevant state equations including the net feedback path are shown below:

$$\dot{\mathbf{x}}(t) = \mathbf{A}(t)\mathbf{x}(t) + \mathbf{B}(t)\mathbf{K}(y,t)y(t) + \mathbf{B}_s(t)u_s(t)$$
$$\mathbf{y}(t) = \mathbf{C}(t)\mathbf{x}(t) + \mathbf{D}(t)\mathbf{K}(y,t)y(t) \qquad\qquad (13)$$

where K(y,t) is the control matrix. Note that a separate $u_s$(t) has been partitioned to represent sources like sensory input which are not part of the feedback controller.

In some embodiments, the biomarker y(t) may be closely correlated to the therapeutic outcome of interest. In further embodiments, a control algorithm may be created to implement K(y,t), which is flexible, time dependent and potentially non-linear. Additional embodiments may minimize the feedforward corruption of the biomarker through stimulation coupling represented by D(t).

A typical DBS stimulation system may require roughly $250\mu$W of power to be delivered to the tissue to provide therapeutic benefit. Thus, the power of the feedback controller, in some embodiments of this disclosure, may be limited to approximately $25\mu$W to avoid undermining device longevity.

Chronic closed-loop neuromodulation may be achieved by using local field potentials (LFPs). Because LFPs represent the ensemble activity of thousands to millions of cells in an in vivo neural population, their recording can often avoid chronic recording issues like tissue encapsulation and micromotion encountered in single-unit recording. In addition, the large geometry of stimulation electrodes, on the order of a few $mm^2$, takes a spatial average of neuronal activity that is by default representative of the LFP activity. In addition the modeling of the disease states as synchronously coherent oscillations may result in biomarkers which are often encoded robustly as field potential spectral fluctuations.

Low frequency power fluctuations of LFPs within discrete frequency bands can provide useful biomarkers for discriminating brain states. In many cases, pathological states can be differentiated by such biomarkers. LFP biomarkers are ubiquitous and span a broad frequency spectrum, from approximately 1 Hz oscillations in deep sleep to greater than 500 Hz "fast ripples" in the hippocampus, and show wide bandwidth variations. The high gamma band power fluctuations within the premotor cortex, which signal motion intent, constitutes an example of field potential coding. The ability of a patient to modulate this band may be used as a control input for a prosthetic actuator for spinal chord injuries. In addition, high gamma band power fluctuations may be useful for modulating stimulation parameters of movement disorders patients. Other examples of high-frequency activity include fast ripples at approximately 200 Hz to 500 Hz, and gamma frequency processing that is indicative of processing of smells in the olfactory bulb. The bandpower coding of LFPs can be used as a sensing paradigm to detect the activity of targeted neural circuits. In addition, LFPs may offer certain practical advantages over spike-based systems, such as providing contextual information and better chronic recording capability.

Sensing systems designed in accordance with this disclosure may provide for the neural coding of field potentials. Such a system may partition the signal chain to play to the relative strengths of analog and digital processing in order to minimize power while maintaining acceptable flexibility and robustness. Referring to the feedback state equations shown above in Equation (13), the signal chain may be partitioned to extract the low-frequency bandpower in a physiological band as the therapeutic signal y(t) using analog preprocessing, such as the preprocessing provided by analog sensing unit 308 in sensing system 300 of FIG. 16. The analog spectral processing may decrease the bandwidth and dynamic range requirements prior to transitioning to digital processing. The control kernel represented by K(y,t) may be implemented in software using a microprocessor, such as microprocessor block 306 in FIG. 16. The processor may provide the mechanism for flexible algorithmic control, and also have ability to run at reduced bandwidth so that the net system power requirements are reduced to a practical level of tens of microwatts. Another advantage of this partition is that adjustments to the control kernel can be made through telemetry download, based on observations and learning made during research.

The partitioning of the signal chain between analog and digital blocks is a balance between power and algorithmic flexibility. The analog block may include a flexible analog processor to extract the core biomarker information, LFP bandpower fluctuations, and thereby maximize information content prior to digitization. The digital block may include flexible algorithms that are implemented in a microprocessor. In some cases, the algorithms may be implemented with low overhead and can achieve a duty cycle of approximately 1%.

Micropower spectral analysis techniques may be useful for many applications including prosthetic applications beyond neuromodulation. In particular, such techniques may be useful with respect to cochlea implants in order to extract the Fourier transforms from a signal and map the extracted information to titrating stimulation in the cochlea An advantage of the heterodyning chopper is that gain-bandwidth requirement of the signal chain may be set by the passband width as opposed to the center frequency.

Various techniques described in this disclosure may be implemented in hardware, software, firmware or any combination thereof For example, various aspects of the techniques may be implemented within or in conjunction with one or more microprocessors, digital signal processors (DSPs), application specific integrated circuits (ASICs), field programmable logic arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry.

When implemented in software, the functionality ascribed to the systems and devices described in this disclosure may be embodied as instructions on a computer-readable medium such as random access memory (RAM), read-only. memory (ROM), non-volatile random access memory (NVRAM), electrically erasable programmable read-only memory (EEPROM), FLASH memory, magnetic media, optical media, or the like. The instructions may be executed to cause a processor to perform or support one or more aspects of the functionality described in this disclosure.

Although the invention is described in the context of EEG signals, various embodiments of the invention may be applied to monitor a variety of a variety of physiological signals, such as EEG, ECoG, ECG, EMG, pressure, temperature, impedance, motion, and other types of signals. Additional embodiments of this invention may be applied to monitor average spike firings of single brain cells by measuring single cell action, potentials and binning the number of spikes over a period of time. Measuring an EMG signal according to the techniques described herein may assist in determining how hard a muscle is firing. In addition, frequency selective monitoring as described in this disclosure may also be used to support any of a variety of therapeutic and/or diagnostic applications.

## Claims

1. A physiological signal monitoring device comprising:

   means for receiving a physiological signal (7, 17);
   means for converting, with a chopper-stabilized heterodyning circuit, a selected frequency band of the physiological signal to a baseband (72, 72A, 72B, 210, 72C, 72D, 322A, 322B, 322C, 322D, 384A, 384B, 384C, 384D, 500); and
   means for analyzing a characteristic of the signal in the selected frequency band (73, 73A)

   wherein the means for converting, with the chopper-stabilized heterdyning circuit, the selected frequency band of the physiological signal to the baseband comprises:

   means for modulating the signal at a first frequency (82, 95, 120, 128, 200, 212A, 212B, 224, 250);
   means for amplifying the modulated signal (86, 97, 122, 130, 200, 250); and **characterised by**
   means for demodulating the amplified signal at a second frequency different from the first frequency (188, 99, 124, 132, 200, 250), wherein the second frequency differs from the first frequency by an offset that is approximately equal to a center frequency of the selected frequency band.

2. The device of claim 1, wherein the second frequency is selected such that the means for demodulating substantially centers the selected frequency band of the signal at the baseband.

3. The device of claim 1, wherein the characteristic of the signal is a power fluctuation of the signal in the selected frequency band, the device further comprising means for generating a signal triggering at least one of control of therapy to the patient or recording of diagnostic information when the power fluctuation exceeds a threshold (88, 99, 124, 132, 200, 250).

4. The device of claim 1, wherein the modulated signal is a second modulated signal, wherein the means for modulating is a first means for modulating, wherein the means for demodulating is a first means for demodulating, and wherein the means for converting, with the chopper-stabilized heterodyning circuit, the selected frequency band of the physiological signal to the baseband further comprises:

> second means for modulating the signal at a third frequency different than the first frequency to produce the first modulated signal (502, 512);
> wherein the first means for modulating modulates the first modulated signal at the first frequency to produce the second modulated signal (120, 128),
> wherein the means for amplifying amplifies the second modulated signal to produce the amplified signal (122, 130),
> wherein the first means for demodulating demodulates the amplified signal at the second frequency to produce a demodulated signal, wherein the second frequency is selected such that the first means for demodulating substantially centers the selected frequency band of the signal at the third frequency in the demodulated signal (124, 132), and
> wherein the means for converting, with the chopper-stabilized heterdyning circuit, the selected frequency band of the physiological signal to the baseband further comprises:

>> second means for demodulating the demodulated signal at the third frequency such that the selected frequency band is substantially centered at the baseband (508, 518).

5. The device of claim 1, wherein:

> the means for receiving comprises a physiological sensing element (7, 17),
> the means for converting comprises the chopper-stabilized heterodyning circuit (72, 72A, 72B, 210, 72C, 72D, 322A, 322B, 322C, 322D, 384A, 384B, 384C, 384D, 500), and
> the means for analyzing comprises a signal analysis unit (73, 73A).

6. The device of claim 5, wherein the signal analysis unit comprises a lowpass filter that filters the converted signal to extract the selected frequency band of the signal at the baseband (74, 74A, 74B, 348).

7. The device of claim 5, wherein the selected frequency band comprises a first selected frequency band and the characteristic comprises a first power, wherein the chopper-stabilized heterodyning circuit is further configured to convert a second selected frequency band of the signal to the baseband (72, 72A, 72B, 210, 72C, 72D, 322A, 322B, 322C, 322D, 384A, 384B, 384C, 384D, 500), and wherein the signal analysis unit analyzes a second power of the signal in the second selected frequency band, and calculates a power ratio between the first power and the second power (73, 73A); and preferably wherein the signal analysis unit generates a signal triggering at least one of control of therapy to the patient or recording of diagnostic information based on the power ratio (73, 73A).

8. A method for monitoring a physiological signal, the method comprising:

> receiving a physiological signal (7, 17, 160);
> converting, with a chopper-stabilized heterodyning circuit, a selected frequency band of the physiological signal to a baseband (72, 72A, 72B, 210, 72C, 72D, 322A, 322B, 322C, 322D, 384A, 384B, 384C, 384D, 500, 162, 164, 165); and
> analyzing a characteristic of the signal in the selected frequency band (73, 73A, 168, 170, 172), wherein converting, with the chopper-stabilized heterodyning circuit, the selected frequency band of the physiological signal to the baseband comprises:

>> modulating the signal at a first frequency (82, 95, 120, 128, 200, 212A, 212B, 224, 250, 162);
>> amplifying the modulated signal (86, 97, 122,130,200, 250, 164); and **characterised by**
>> demodulating the amplified signal at a second frequency different from the first frequency (88, 99, 124, 132, 200, 250, 165), wherein the second frequency differs from the first frequency by an offset that is approximately equal to a center frequency of the selected frequency band.

9. The method of claim 8, wherein demodulating the amplified signal comprises demodulating, with a demodulator, the amplified signal at a second frequency, wherein the second frequency is selected such that the demodulator substantially centers the selected frequency band of the signal at the baseband.

10. The method of claim 8, wherein the characteristic of the signal is a power fluctuation of the signal in the selected frequency band, the method further comprising generating a signal triggering at least one of control of therapy to the patient or recording of diagnostic information when the power fluctuation exceeds a threshold (88, 99, 124, 132, 200, 250, 176).

11. The method of claim 8, wherein the selected frequency band comprises a first selected frequency band and the characteristic comprises a first power, the method further comprising:

converting, with the chopper-stabilized heterodyning circuit, a second selected frequency band of the signal to the baseband (72, 72A, 72B, 210, 72C, 72D, 322A, 322B, 322C, 322D, 384A, 384B, 384C, 384D, 500, 162, 164, 165);
analyzing a second power of the signal in the second selected frequency band (73, 73A); and
calculating a power ratio between the first power and the second power; and preferably further comprising generating a signal triggering at least one of control of therapy to the patient or recording of diagnostic information based on the power ratio (73, 73A).

12. The method of claim 8, wherein the modulated signal is a second modulated signal, and wherein converting, with the chopper-stabilized heterodyning circuit, the selected frequency band of the physiological signal to the baseband further comprises:

modulating the signal at a third frequency different than the first frequency to produce a first modulated signal (502, 512),
wherein modulating the signal at the first frequency comprises modulating the first modulated signal at the first frequency to produce the second modulated signal (120, 128),
wherein amplifying the modulated signal comprises amplifying the second modulated signal to produce the amplified signal (122, 130);
wherein demodulating the amplified signal at the second frequency comprises demodulating the amplified signal at the second frequency, wherein the second frequency is selected such that the demodulator substantially centers the selected frequency band of the signal at the third frequency in the demodulated signal (124, 132), and
wherein converting, with the chopper-stabilized heterodyning circuit, the selected frequency band of the physiological signal to the baseband further comprises:

demodulating the demodulated signal at the third frequency such that the selected frequency band is substantially centered at the baseband (508, 518).

13. A medical device comprising:

a physiological signal monitoring device according to any of claims 1 to 7, wherein the means for receiving comprises a physiological sensing element that receives the physiological signal (7, 17), wherein the means for converting comprises the chopper-stabilized heterodyning circuit configured to covert a selected frequency band of the physiological signal to the baseband (72, 72A, 72B, 210, 72C, 72D, 322A, 322B, 322C, 322D, 384A, 384B, 384C, 384D, 500), and wherein the means for analyzing comprises a signal analysis unit that analyzes the characteristic of the signal in the selected frequency band, and generates a trigger signal triggering control of therapy to the patient based on the analyzed characteristic (73, 73A); and
a therapy delivery module that controls the therapy in response to the trigger signal (12, 28).

**Patentansprüche**

1. Überwachungsvorrichtung für physiologische Signale, die Folgendes enthält:

ein Mittel (7, 17) zum Empfangen eines physiologischen Signals;
ein Mittel (72, 72A, 72B, 210, 72C, 72D, 322A, 322B, 322C, 322D, 384A, 384B, 384C, 384D, 500) zum Umsetzen eines ausgewählten Frequenzbands des physiologischen Signals in ein Basisband mit einer zerhackerstabilisierten Überlagerungsschaltung; und
ein Mittel (73, 73A) zum Analysieren einer Kennlinie des Signals in dem ausgewählten Frequenzband,

wobei das Mittel zum Umsetzen des ausgewählten Frequenzbands des physiologischen Signals in das Basisband mit der zerhackerstabilisierten Überlagerungsschaltung Folgendes enthält:

ein Mittel (82, 95, 120, 128, 200, 212A, 212B, 224, 250) zum Modulieren des Signals bei einer ersten Frequenz;
ein Mittel (86, 97, 122, 130, 200, 250) zum Verstärken des modulierten Signals; und **gekennzeichnet durch**
ein Mittel (188, 99, 124, 132, 200, 250) zum Demodulieren des verstärkten Signals bei einer zweiten Frequenz, die von der ersten Frequenz verschieden ist, wobei die zweite Frequenz von der ersten Frequenz um einen Versatz abweicht, der näherungsweise gleich einer Mittenfrequenz des ausgewählten Frequenzbands ist.

2. Vorrichtung nach Anspruch 1, wobei die zweite Frequenz derart ausgewählt ist, dass das Mittel zum Demodulieren das ausgewählte Frequenzband des Signals auf das Basisband im Wesentlichen zentriert.

3. Vorrichtung nach Anspruch 1, wobei die Kennlinie des Signals eine Leistungsschwankung des Signals in dem ausgewählten Frequenzband ist, wobei die Vorrichtung ferner Mittel (88, 99, 124, 132, 200, 250) zum Erzeugen eines Signals, das die Steuerung einer Therapie an den Patienten und/oder das Aufnehmen von diagnostischen Informationen auslöst, wenn die Leistungsschwankung einen Schwellenwert überschreitet, enthält.

4. Vorrichtung nach Anspruch 1, wobei das modulierte Signal ein zweites moduliertes Signal ist, wobei das Mittel zum Modulieren ein erstes Mittel zum Modulieren ist, wobei das Mittel zum Demodulieren ein erstes Mittel zum Demodulieren ist und wobei das Mittel zum Umsetzen des ausgewählten Frequenzbands des physiologischen Signals in das Basisband mit der zerhackerstabilisierten Überlagerungsschaltung ferner Folgendes enthält:

ein zweites Mittel (502, 512) zum Modulieren des Signals auf eine dritte Frequenz, die von der ersten Frequenz verschieden ist, um das erste modulierte Signal zu erzeugen;
wobei das erste Mittel (120, 128) zum Modulieren das erste modulierte Signal auf die erste Frequenz moduliert, um das zweite modulierte Signal zu erzeugen,
wobei das Mittel (122, 130) zum Verstärken das zweite modulierte Signal verstärkt, um das verstärkte Signal zu erzeugen,
wobei das erste Mittel (124, 132) zum Demodulieren das verstärkte Signal auf der zweiten Frequenz demoduliert, um ein demoduliertes Signal zu erzeugen, wobei die zweite Frequenz derart ausgewählt ist, dass das erste Mittel zum Demodulieren im Wesentlichen das ausgewählte Frequenzband des Signals auf die dritte Frequenz in dem demodulierten Signal zentriert, und

wobei das Mittel zum Umsetzen des ausgewählten Frequenzbands des physiologischen Signals in das Basisband mit der zerhackerstabilisierten Überlagerungsschaltung ferner Folgendes enthält:

ein zweites Mittel (508, 518) zum Demodulieren des demodulierten Signals bei der dritten Frequenz, derart, dass das ausgewählte Frequenzband auf das Basisband im Wesentlichen zentriert wird.

5. Vorrichtung nach Anspruch 1, wobei:

das Mittel zum Empfangen ein physiologisches Erfassungselement (7, 17) enthält,
das Mittel zum Umsetzen die zerhackerstabilisierte Überlagerungsschaltung (72, 72A, 72B, 210, 72C, 72D, 322A, 322B, 322C, 322D, 384A, 384B, 384C, 384D, 500) enthält, und
das Mittel zum Analysieren eine Signalanalyseeinheit (73, 73A) enthält.

6. Vorrichtung nach Anspruch 5, wobei die Signalanalyseeinheit ein Tiefpassfilter (74, 74A, 74B, 348) enthält, das das umgesetzte Signal filtert, um das ausgewählte Frequenzband des Signals auf dem Basisband zu extrahieren.

7. Vorrichtung nach Anspruch 5, wobei das ausgewählte Frequenzband ein erstes ausgewähltes Frequenzband enthält und die Kennlinie eine erste Leistung enthält, wobei die zerhackerstabilisierte Überlagerungsschaltung (72, 72A, 72B, 210, 72C, 72D, 322A, 322B, 322C, 322D, 384A, 384B, 384C, 384D, 500) ferner konfiguriert ist, ein zweites ausgewähltes Frequenzband des Signals in das Basisband umzusetzen und wobei die Signalanalyseeinheit (73, 73A) eine zweite Leistung des Signals in dem zweiten ausgewählten Frequenzband analysiert und ein Leistungsverhältnis zwischen der ersten Leistung und der zweiten Leistung berechnet und wobei die Signalanalyseeinheit (73, 73A) vorzugsweise auf der Basis des Leistungsverhältnisses ein Signal erzeugt, das die Steuerung einer Therapie an den Patienten und/oder das Aufnehmen von diagnostischen Informationen auslöst.

8. Verfahren zum Überwachen eines physiologischen Signals, wobei das Verfahren Folgendes umfasst:

Empfangen eines physiologischen Signals (7, 17, 160);

Umsetzen eines ausgewählten Frequenzbands des physiologischen Signals in ein Basisband mit einer zerhackerstabilisierten Überlagerungsschaltung (72, 72A, 72B, 210, 72C, 72D, 322A, 322B, 322C, 322D, 384A, 384B, 384C, 384D, 500, 162, 164, 165); und

Analysieren einer Kennlinie des Signals in dem ausgewählten Frequenzband (73, 73A, 168, 70, 172), wobei das Umsetzen des ausgewählten Frequenzbands des physiologischen Signals in das Basisband mit einer zerhackerstabilisierten Überlagerungsschaltung Folgendes umfasst:

Modulieren des Signals bei einer ersten Frequenz (82, 95, 120, 128, 200, 212A, 212B, 224, 250, 162);

Verstärken des modulierten Signals (86, 97, 122, 130, 200, 250, 164); und **gekennzeichnet durch** Demodulieren des verstärkten Signals bei einer zweiten Frequenz, die von der ersten Frequenz verschieden ist (88, 99, 124, 132, 200, 250, 165), wobei die zweite Frequenz von der ersten Frequenz um einen Versatz abweicht, der näherungsweise gleich einer Mittenfrequenz des ausgewählten Frequenzbands ist.

9. Verfahren nach Anspruch 8, wobei das Demodulieren des verstärkten Signals das Demodulieren des verstärkten Signals mit einem Demodulator bei einer zweiten Frequenz umfasst, wobei die zweite Frequenz derart ausgewählt ist, dass der Demodulator im Wesentlichen das ausgewählte Frequenzband des Signals auf das Basisband zentriert.

10. Verfahren nach Anspruch 8, wobei die Kennlinie des Signals eine Leistungsschwankung des Signals in dem ausgewählten Frequenzband ist, wobei das Verfahren ferner das Erzeugen eines Signals, das die Steuerung einer Therapie an den Patienten und/oder das Aufnehmen von diagnostischen Informationen auslöst, wenn die Leistungsschwankung einen Schwellenwert überschreitet (88, 99, 1124, 132, 200, 250, 176), umfasst.

11. Verfahren nach Anspruch 8, wobei das ausgewählte Frequenzband ein erstes ausgewähltes Frequenzband umfasst und die Kennlinie eine erste Leistung umfasst, wobei das Verfahren ferner Folgendes umfasst:

Umsetzen eines zweiten ausgewählten Frequenzbands des Signals in das Basisband mit einer zerhackerstabilisierten Überlagerungsschaltung (72, 72A, 72B, 210, 72C, 72D, 322A, 322B, 322C, 322D, 384A, 384B, 384C, 384D, 500, 162, 164, 165);

Analysieren einer zweiten Leistung des Signals in dem zweiten ausgewählten Frequenzband (73, 73A); und Berechnen eines Leistungsverhältnisses zwischen der ersten Leistung und der zweiten Leistung; und

wobei das Verfahren vorzugsweise ferner das Erzeugen eines Signals, das auf der Basis des Leistungsverhältnisses die Steuerung einer Therapie an den Patienten und/oder das Aufnehmen diagnostischer Informationen auslöst, umfasst (73, 73A).

12. Verfahren nach Anspruch 8, wobei das modulierte Signal ein zweites moduliertes Signal ist und wobei das Umsetzen des ausgewählten Frequenzbands des physiologischen Signals in das Basisband mit der zerhackerstabilisierten Überlagerungsschaltung ferner Folgendes umfasst:

Modulieren des Signals bei einer dritten Frequenz, die von der ersten Frequenz verschieden ist, um ein erstes moduliertes Signal zu erzeugen (502, 512),

wobei das Modulieren des Signals bei der ersten Frequenz das Modulieren des ersten modulierten Signals bei der ersten Frequenz umfasst, um das zweite modulierte Signal zu erzeugen (120, 128),

wobei das Verstärken des modulierten Signals das Verstärken des zweiten modulierten Signals umfasst, um das verstärkte Signal zu erzeugen (122, 130);

wobei das Demodulieren des verstärkten Signals bei der zweiten Frequenz das Demodulieren des verstärkten Signals bei der zweiten Frequenz umfasst, wobei die zweite Frequenz derart ausgewählt ist, dass der Demodulator das ausgewählte Frequenzband des Signals auf die dritte Frequenz in dem demodulierten Signal im Wesentlichen zentriert (124, 132), und

wobei das Umsetzen des ausgewählten Frequenzbands des physiologischen Signals in das Basisband mit der zerhackerstabilisierten Überlagerungsschaltung ferner Folgendes umfasst:

Demodulieren des demodulierten Signals bei der dritten Frequenz, derart, dass das ausgewählte Frequenzband auf das Basisband im Wesentlichen zentriert wird (508, 518).

13. Medizinische Vorrichtung, die Folgendes enthält:

eine Überwachungsvorrichtung für ein physiologisches Signal nach einem der Ansprüche 1 bis 7, wobei das Mittel zum Empfangen ein physiologisches Erfassungselement (7, 17) enthält, das das physiologische Signal empfängt, wobei das Mittel zum Umsetzen die zerhackerstabilisierte Überlagerungsschaltung (62, 72A, 72B, 210, 72C, 72D, 322A, 322B, 322C, 322D, 384A, 384B, 384C, 384D, 500) enthält, die konfiguriert ist, ein ausgewähltes Frequenzband des physiologischen Signals in das Basisband umzusetzen, und wobei das Mittel zum Analysieren eine Signalanalyseeinheit (73, 73A) enthält, die die Kennlinie des Signals in dem ausgewählten Frequenzband analysiert und ein Auslösersignal erzeugt, das auf der Basis der analysierten Kennlinie die Steuerung einer Therapie an den Patienten auslöst; und

ein Therapieverabreichungsmodul (12, 28), das als Antwort auf das Auslösersignal die Therapie steuert.

**Revendications**

1. Dispositif de surveillance de signal physiologique compourtant :

   des moyens pour recevoir un signal physiologique (7, 17) ;
   des moyens pour convertir, à l'aide d'un circuit d'hétérodynage stabilisé avec un amplificateur à hacheur, une bande de fréquences choisie du signal physiologique en une bande de base (72, 72A, 72B, 210, 72C, 72D, 322A,322B, 322C, 322D, 384A, 384B, 384C, 384D, 500) ; et
   des moyens pour analyser une caractéristique du signal dans la bande de fréquences choisie (73, 73A) ;

   dans lequel les moyens pour convertir, à l'aide du circuit d'hétérodynage stabilisé avec un amplificateur à hacheur, la bande de fréquences choisie du signal physiologique en bande de base comporte :

   des moyens pour moduler le signal à une première fréquence (82,95, 120, 128, 200, 212A, 212B, 224, 150) ;
   des moyens pour amplifier le signal modulé (86, 97, 122, 130, 200, 250) ; et **caractérisé par**
   des moyens pour démoduler le signal amplifié à une seconde fréquence différente de la première fréquence (188, 99, 124, 132, 200, 250), dans lequel la seconde fréquence diffère de la première fréquence d'un décalage qui est approximativement égal à une fréquence centrale de la bande de fréquences choisie.

2. Dispositif selon la revendication 1, dans lequel la seconde fréquence est choisie de sorte que les moyens de démodulation centrent en grande partie la bande de fréquences choisie du signal à la bande de base.

3. Dispositif selon la revendication 1, dans lequel la caractéristique du signal est une fluctuation de puissance du signal dans la bande de fréquences choisie, le dispositif comportant en outre des moyens pour générer un signal déclenchant au moins l'une d'une commande de thérapie au patient ou de l'enregistrement d'informations de diagnostic lorsque la fluctuation de puissance dépasse un seuil (88, 99, 124, 132, 200, 250).

4. Dispositif selon la revendication 1, dans lequel le signal modulé est un second signal modulé, dans lequel les moyens de démodulation sont des premiers moyens pour moduler, dans lequel les moyens de démodulation sans des premiers moyens pour démoduler, et dans lequel les moyens de conversion, à l'aide du circuit d'hétérodynage stabilisé avec un amplificateur à hacheur, de la bande de fréquences choisie du signal physiologique en bande de base comporte en outre :

   des seconds moyens pour moduler le signal à une troisième fréquence différente de la première fréquence afin de produire le premier signal modulé (502, 512) ;
   dans lequel les premiers moyens de démodulation modulent le premier signal modulé à la première fréquence afin de produire le second signal modulé (120, 128),
   dans lequel les moyens d'amplification amplifient le second signal modulé afin de produire le signal amplifié (122, 130),
   dans lequel les premiers moyens de démodulation démodulent le signal amplifié à la seconde fréquence afin de produire un signal démodulé, dans lequel la seconde fréquence est choisie de sorte que les premiers moyens de démodulation centrent en grande partie la bande de fréquences choisie du signal à la troisième fréquence dans le signal démodulé (124, 132), et

   dans lequel les moyens de conversion, à l'aide du circuit d'hétérodynage stabilisé avec un amplificateur à hacheur, de la bande de fréquences choisie du signal physiologique en bande de base comportent en outre :

des seconds moyens pour démoduler le signal démodulé à la troisième fréquence de sorte que la bande de fréquences choisie est en grande partie centrée sur la bande de base (508, 518).

5. Dispositif selon la revendication 1, dans lequel :

les moyens de réception comportent un élément de détection physiologique (7, 17),
les moyens de conversion comportent le circuit d'hétérodynage stabilisé avec un amplificateur à hacheur (72, 72A, 72B, 210, 72C, 72D, 322A, 322B, 322C, 322D, 384A, 384B, 384C, 384D, 500), et
les moyens d'analyse comportent une unité d'analyse de signal (73, 73A).

6. Dispositif selon la revendication 5, dans lequel l'unité d'analyse de signal comporte un filtre passe-bas qui filtre le signal converti afin d'extraire la bande de fréquences choisie du signal à la bande de base (74, 74A, 74B, 348).

7. Dispositif selon la revendication 5, dans lequel la bande de fréquences choisie comporte une première bande de fréquences choisie et la caractéristique comporte une première puissance, dans lequel le circuit d'hétérodynage stabilisé avec un amplificateur à hacheur est en outre configuré pour convertir une seconde bande de fréquences choisie du signal à la bande de base (72, 72A, 72B, 210, 72C, 72D, 322A,322B, 322C, 322D, 384A, 384B, 384C, 384D, 500), et dans lequel l'unité d'analyse de signal analyse une seconde puissance du signal dans la seconde bande de fréquences choisie, et calcule un rapport de puissance entre la première puissance et la seconde puissance (73, 73A) ; et de manière préférée dans lequel l'unité d'analyse de signal génère un signal déclenchant au moins l'une d'une commande de thérapie au patient ou de l'enregistrement d'informations de diagnostic sur la base du rapport de puissance (73, 73A).

8. Procédé pour surveiller un signal physiologique, le procédé comportant :

de recevoir un signal physiologique (7, 17, 160) ;
de convertir, à l'aide d'un circuit d'hétérodynage stabilisé avec un amplificateur à hacheur, une bande de fréquences choisie du signal physiologique en une bande de base (72, 72A, 72B, 210, 72C, 72D, 322A,322B, 322C, 322D, 384A, 384B, 384C, 384D, 500) ; et
d'analyser une caractéristique du signal dans la bande de fréquences choisie (73, 73A) ; dans lequel la conversion, à l'aide du circuit d'hétérodynage stabilisé avec un amplificateur à hacheur, de la bande de fréquences choisie du signal physiologique en bande de base comporte :

de moduler le signal à une première fréquence (82,95, 120, 128, 200, 212A, 212B, 224, 150) ;
d'amplifier le signal modulé (86, 97, 122, 130, 200, 250) ; et **caractérisé par**
la démodulation du signal amplifié à une seconde fréquence différente de la première fréquence (88, 99, 124, 132, 200, 250), dans lequel la seconde fréquence diffère de la première fréquence d'un décalage qui est approximativement égal à une fréquence centrale de la bande de fréquences choisie.

9. Procédé selon la revendication 8, dans lequel la démodulation du signal amplifié comporte de démoduler, à l'aide du démodulateur, le signal amplifié à une seconde fréquence, dans lequel la seconde fréquence est choisie de sorte que le démodulateur centre en grande partie à bande de fréquences choisies du signal à la bande de base.

10. Procédé selon la revendication 8, dans lequel la caractéristique du signal est une fluctuation de puissance du signal dans la bande de fréquences choisie, le procédé comportant en outre de générer un signal déclenchant au moins l'une d'une commande de thérapie au patient ou de l'enregistrement d'informations de diagnostic lorsque la fluctuation de puissance dépasse un seuil (88, 99, 124, 132, 200, 250, 176).

11. Procédé selon la revendication 8, dans lequel la bande de fréquences choisie comporte une première bande de fréquences choisie et la caractéristique comporte une première puissance, le procédé comportant en outre les étapes consistant à :

convertir, à l'aide du circuit d'hétérodynage stabilisé avec un amplificateur à hacheur, une bande de fréquences choisie du signal en bande de base (72, 72A, 72B, 210, 72C, 72D, 322A, 322B, 322C, 322D, 384A, 384B, 384C, 384D, 500, 162, 164, 165);
analyser une seconde puissance du signal dans la seconde bande de fréquences choisie (73, 73A) ; et
calculer un rapport de puissance entre la première puissance et la seconde puissance ; et
de manière préférée comportant en outre de générer un signal déclenchant au moins l'une d'une commande de thérapie au patient ou de l'enregistrement d'informations de diagnostic sur la base du rapport de puissance (73, 73A).

**12.** Procédé selon la revendication 8, dans lequel le signal modulé est un second signal modulé, et dans lequel la conversion, à l'aide du circuit d'hétérodynage stabilisé avec un amplificateur à hacheur, de la bande de fréquences choisie du signal physiologique en bande de base comporte en outre les étapes consistant à :

moduler le signal à une troisième fréquence différente de la première fréquence pour produire un premier signal modulé (502, 512),
dans lequel la modulation du signal à la première fréquence comporte de moduler le premier signal modulé à la première fréquence afin de produire le second signal modulé (120, 128),
dans lequel l'amplification du signal modulé comporte d'amplifier le second signal modulé afin de produire le signal amplifié (122, 130) ;
dans lequel la démodulation du signal amplifié à la seconde fréquence comporte de démoduler le signal amplifié à la seconde fréquence, dans lequel la seconde fréquence est choisie de sorte que le démodulateur centre en grande partie la bande de fréquences choisie du signal à la troisième fréquence dans le signal démodulé (124, 132), et

dans lequel la conversion, à l'aide du circuit d'hétérodynage stabilisé avec un amplificateur à hacheur, de la bande de fréquences choisie du signal physiologique en bande de base comporte en outre :

de démoduler le signal démodulé à la troisième fréquence de sorte que la bande de fréquences choisie est en grande partie centrée sur la bande de base (508, 518).

**13.** Dispositif médical comportant :

un dispositif de surveillance de signal physiologique selon l'une quelconque des revendications 1 à 7, dans lequel les moyens de réception comportent un élément de détection physiologique qui reçoit le signal physiologique (7, 17), dans lequel les moyens de conversion comportent le circuit d'hétérodynage stabilisé avec un amplificateur à hacheur configuré pour convertir une bande de fréquences choisie du signal physiologique en bande de base (72, 72A, 72B, 210, 72C, 72D, 322A, 322B, 322C, 322D, 384A, 384B, 384C, 384D, 500), et dans lequel les moyens d'analyse comportent une unité d'analyse de signal qui analyse la caractéristique du signal dans la bande de fréquences choisie, et génère un signal de déclenchement déclenchant une commande de thérapie au patient sur la base de la caractéristique analysée (73, 73A) ; et
un module d'administration de thérapie qui commande la thérapie en réponse au signal de déclenchement (12, 28).

**FIG. 1**

SENSOR
14

FREQUENCY
SELECTIVE
MONITOR
16

TELEMETRY
MODULE
18

POWER
SOURCE
15

SENSING
ELEMENTS
17

MEDICAL DEVICE
20

MEMORY
26

PROCESSOR
22

TELEMETRY
MODULE
24

THERAPY
DELIVERY
MODULE
28

POWER
SOURCE
21

THERAPY DELIVERY
ELEMENTS
29

FIG. 2

FIG. 3

EP 2 200 692 B1

FIG. 4

FIG. 5A

FIG. 5B

FIG. 5C

FIG. 5D

FIG. 6A

EP 2 200 692 B1

$V_{out}(\delta 1)$

$V_{out}(\delta 2)$

SIGNAL ANALYSIS UNIT
73A

PASSIVE LOWPASS FILTER 74A

PASSIVE LOWPASS FILTER 74B

POWER MEASUREMENT MODULE 76A

POWER MEASUREMENT MODULE 76B

LOWPASS FILTER 77A

LOWPASS FILTER 77B

COMPARATOR 80

TRIGGER SIGNAL

FIG. 6B

FIG. 7

FIG. 8A

FIG. 8B

FIG. 8C

FIG. 8D

FIG. 9

```
                                                                    140
┌─────────────────────────────────────────────────┐
│   RECEIVE WIDE BAND PHYSIOLOGICAL SIGNAL FROM     │
│      ELECTRODES ASSOCIATED WITH PATIENT           │
└─────────────────────────────────────────────────┘
                         │
                         ▼                          142
┌─────────────────────────────────────────────────┐
│          UPMODULATE BASEBAND SIGNAL               │
│             TO CARRIER FREQUENCY                  │
└─────────────────────────────────────────────────┘
                         │
                         ▼                          143
┌─────────────────────────────────────────────────┐
│      LOW-BAND NOISE ADDED TO MODULATED SIGNAL     │
└─────────────────────────────────────────────────┘
                         │
                         ▼                          144
┌─────────────────────────────────────────────────┐
│          AMPLIFY NOISY MODULATED SIGNAL           │
└─────────────────────────────────────────────────┘
                         │
                         ▼                          145
┌─────────────────────────────────────────────────┐
│         DOWN-MODULATE AMPLIFIED SIGNAL            │
│       BY CARRIER FREQUENCY TO BASEBAND            │
└─────────────────────────────────────────────────┘
                         │
                         ▼                          146
┌─────────────────────────────────────────────────┐
│          LOWPASS FILTER BASEBAND SIGNAL           │
│             TO REMOVE EXCESS NOISE                │
└─────────────────────────────────────────────────┘
                         │
                         ▼                          148
┌─────────────────────────────────────────────────┐
│     TUNE POWERED BANDPASS FILTER TO DESIRED       │
│     FREQUENCY BAND OF PHYSIOLOGICAL SIGNAL        │
└─────────────────────────────────────────────────┘
                         │
                         ▼                          150
┌─────────────────────────────────────────────────┐
│   BANDPASS FILTER BASEBAND SIGNAL TO EXTRACT      │
│     SIGNAL WITHIN DESIRED FREQUENCY BAND          │
└─────────────────────────────────────────────────┘
                         │
                         ▼                          152
┌─────────────────────────────────────────────────┐
│            MEASURE POWER OF SIGNAL                │
│        WITHIN DESIRED FREQUENCY BAND              │
└─────────────────────────────────────────────────┘
                         │
                         ▼                          154
┌─────────────────────────────────────────────────┐
│          COMPARE CURRENT POWER WITH               │
│     BASELINE POWER THRESHOLD OF PATIENT           │
└─────────────────────────────────────────────────┘
                         │
   155                   ▼
        ◇ CURRENT >         NO    ┌──────────────────┐  156
        ◇ THRESHOLD? ◇ ─────────▶ │ DO NOT GENERATE   │
                                   │  TRIGGER SIGNAL  │
                         │         └──────────────────┘
                        YES              158
                         ▼
┌───────────────────────────────────┐
│     GENERATE TRIGGER SIGNAL        │
└───────────────────────────────────┘
```

**FIG. 10**

160 RECEIVE WIDE BAND PHYSIOLOGICAL SIGNAL FROM
ELECTRODES ASSOCIATED WITH PATIENT

162 UPMODULATE SIGNAL AT DESIRED FREQUENCY
BAND TO CARRIER FREQUENCY

163 LOW-BAND NOISE ADDED TO MODULATED SIGNAL

164 AMPLIFY NOISY MODULATED SIGNAL

165 DOWN-MODULATE AMPLIFIED SIGNAL AT CARRIER
FREQUENCY +/- OFFSET TO BASEBAND

166 LOWPASS FILTER BASEBAND SIGNAL
TO REMOVE EXCESS NOISE

168 LOWPASS FILTER BASEBAND SIGNAL TO EXTRACT
SIGNAL WITHIN DESIRED FREQUENCY BAND

170 MEASURE POWER OF SIGNAL
WITHIN DESIRED FREQUENCY BAND

172 COMPARE CURRENT POWER WITH
BASELINE POWER THRESHOLD OF PATIENT

173 CURRENT >
THRESHOLD?

NO → 174 DO NOT GENERATE
TRIGGER SIGNAL

YES

176 GENERATE TRIGGER SIGNAL

FIG. 11

**FIG. 12**

FIG. 13

EP 2 200 692 B1

FIG. 14

FIG. 15

EP 2 200 692 B1

**FIG. 16**

FIG. 17  TRANSCONDUCTOR  DEMODULATOR  INTEGRATOR

FIG. 18

**FIG. 19**

FIG. 20

**FIG. 21**

400

402

PREPROCESS PHYSIOLOGICAL SIGNALS TO GENERATE
ONE OR MORE BANDPOWER SIGNALS

404

CONVERT ANALOG BANDPOWER SIGNAL INTO A DIGITAL
SIGNAL

406

CALCULATE A ROLLING MEAN FOR THE DIGITIZED
BANDPOWER SIGNAL TO GENERATE A FOREGROUND
SIGNAL

408

DOWNSAMPLE THE DIGITIZED BANDPOWER SIGNAL

410

APPLY A THREE-STAGE MEDIAN FILTER TO THE
DOWNSAMPLED SIGNAL OVER A BACKGROUND TIME
WINDOW TO GENERATE A BACKGROUND SIGNAL

412

COMPARE THE FOREGROUND SIGNAL TO THE
BACKGROUND SIGNAL TO NORMALIZE THE SIGNAL

FIG. 22

Current
Dipole
(Sense)

Current
Dipole
(Stim)

A

D

B

C

**FIG. 23**

FIG. 24

FIG. 25

IG. 26

EP 2 200 692 B1

**FIG. 27**

**EP 2 200 692 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006126186 A
- WO 9710747 A
- US 7385443 B, Timothy J. Denison